# EUROPEAN PATENT SPECIFICATION

(11) **EP 2 753 645 B1**
(45) Date of publication and mention of the grant of the patent: **21.02.2018**
(21) Application number: 12761785.0
(22) Date of filing: 05.09.2012
(51) Int. Cl.: C07K 16/22, C07K 16/46, A61P 29/00

(54) **MODIFIED ANTIBODIES AND METHOD FOR THE PRODUCTION OF SAME**
MODIFIZIERTE ANTIKÖRPER UND VERFAHREN ZU DEREN HERSTELLUNG
ANTICORPS MODIFIES ET PROCEDES POUR LEUR PRODUCTION

(30) Priority: 06.09.2011 US 201161531439 P
(43) Date of publication of application: 16.07.2014
(73) Proprietor: Nexvet Australia Pty Ltd, Melbourne VIC 3000 (AU)
(72) Inventor: GEARING, David, Melbourne, Victoria 3000 (AU)
(74) Representative: Mannion, Sally Kim
(86) International application number: PCT/GB2012/052174
(87) International publication number: WO 2013/034900

(56) References cited:
- WO-A2-2005/061540
- WO-A2-2006/131951
- WO-A2-2010/027488
- WO-A2-2012/024650
- PELAT THIBAUT ET AL: "Non-human primate immune libraries combined with germline humanization: an (almost) new, and powerful approach for the isolation of therapeutic antibodies", MABS,, vol. 1, no. 4, 1 July 2009 (2009-07-01), pages 377-381, XP009135983,
- Sonia Covaceuszach ET AL: "Single Cycle Structure-Based Humanization of an Anti-Nerve Growth Factor Therapeutic Antibody", PLoS ONE, vol. 7, no. 3, 5 March 2012 (2012-03-05), page e32212, XP055369726, DOI: 10.1371/journal.pone.0032212
- David Gareth Williams ET AL: "Humanising Antibodies by CDR Grafting", Antibody Engineering, 1 January 2010 (2010-01-01), pages 319-399, XP055369742, Retrieved from the Internet: URL:https://ebookcentral.proquest.com/lib/ epo-ebooks/home.action?ebraryDocId=null [retrieved on 2017-05-04]
- LAZAR ET AL: "A molecular immunology approach to antibody humanization and functional optimization", MOLECULAR IMMUNOLOGY, PERGAMON, GB, vol. 44, no. 8, 1 December 2006 (2006-12-01), pages 1986-1998, XP005792736, ISSN: 0161-5890, DOI: 10.1016/J.MOLIMM.2006.09.029

## Description

### Field of the Disclosure

The present disclosure relates to methods for producing non-immunogenic binding agents, in particular immunoglobulins and fragments thereof, which can be used in therapeutic methods. In particular, the methods allow a donor immunoglobulin to be modified such that it can be administered to a target species with minimal risk of neutralising antibodies being raised there against. The disclosure further extends to immunoglobulins produced by said methods and to their use in therapy.

### Background to the Disclosure

Advances in recombinant DNA technology have resulted in a variety of proteins being developed for pharmaceutical applications. This has resulted in a number of protein-based drugs, which may be more generally referred to as biologics, being the subject of clinical trials or market approval. Due to their inherent properties and structures, protein-based drugs are significantly larger and more complex than more traditional small molecule organic and inorganic based molecules. In particular, the folded tertiary structure of the protein is essential to its biological function.

Antibodies are one family of proteins that are being widely developed and used in therapeutic applications in man. The popularity of antibodies in therapeutic applications has been due to their versatility in being able to specifically target virtually any desired target molecule. The majority of protein based therapeutic products are monoclonal antibodies. However one significant drawback associated with the use of monoclonal antibody-based therapies is the production of neutralising antibodies against the therapeutic monoclonal antibody when administered to a subject. These neutralising antibodies result from the immune system of the subject recognising as foreign sequences of amino acids that are present in the administered monoclonal antibody. As a result, an immune response is mounted against the administered therapeutic antibody. The production of neutralising antibodies by the subject can significantly impair the ability to continue treating the subject with the therapeutic monoclonal antibody. Typically, once neutralising antibodies have been produced in the subject, the use of the therapeutic antibody needs to be increased as the neutralising antibodies effectively reduce or negate the therapeutic effect of the administered monoclonal antibody. This can limit the use of the therapeutic antibody to an initial treatment only, with the result that repeat or long term dosing of the therapeutic antibody is not an option. In short, the production of neutralising antibodies against a therapeutic antibody can significantly limit the therapeutic use of that antibody and this, in turn can significantly limit, or completely prevent, the use of the antibody in treatment of chronic or recurring diseases.

To attempt to reduce the likelihood of neutralising antibodies being produced against a therapeutic antibody, a number of approaches have been developed which are designed to reduce the immunogenicity of the antibody by modifying its structure. One such approach is the production of a chimeric antibody whereby the heavy and light chain constant domains of the antibody are derived from an antibody obtained from the same species as the subject to whom the antibody is to be administered. As most therapeutic antibodies have been developed for use in humans, such chimeric antibodies typically comprise human derived heavy and light chain constant domains conjoined to heavy and light chain variable regions derived from a non-human antibody, most typically of mouse or rat origin.

Further approaches to reduce the immunogenicity of therapeutic antibodies employ techniques referred to as humanisation. The term humanisation reflects the fact that the modified antibodies are being made more like antibodies which would be produced by a human, in order to limit the possibility of an immune response resulting. Humanisation techniques extend to a number of different approaches to making an antibody more human-like.

One commonly used humanisation technique is that of CDR grafting whereby the complementarity determining regions (CDRs) derived from a donor antibody, such as a murine derived antibody, are combined with framework regions derived from an antibody of human origin to form heavy and light chain variable domains which are then combined with human antibody derived heavy and light chain constant domains. The resulting antibody therefore contains only a limited number of non-human derived amino acids, this serving to limit the presence of epitopes which will be viewed by the human immune system as foreign.

One significant drawback associated with humanisation is that it often results in a significant reduction in the binding affinity of the resulting humanised antibody over that exhibited by the non-humanised donor antibody. Just as is the case with the production of neutralising antibodies against a therapeutic monoclonal antibody, this can result in the therapeutic use of that antibody being significantly compromised. In particular, the reduction in binding affinity results in the need to administer a greater dose of the therapeutic antibody and it may further require the dosage frequency to be increased too. Both of these factors result in an increase in the cost of therapy and an increase in the inconvenience to the patient. Further, the administration of larger quantities of the antibody to a subject results in an increased risk that neutralising antibodies will be raised against the therapeutic antibody.

In cases where the humanised antibodies bind to the desired epitope with lower affinity than the initial donor antibody, this can be due to the incompatible structural alignment of framework region sequences with the epitope binding CDR sequences. Through an iterative process of back-mutating human residues with the amino acids at the same position in the donor antibody, the affinity of the humanised antibody can often be restored. Although the back mutation process can result in further non-human amino acid residues being reintroduced into the humanised antibody, the resulting antibody is still termed humanised despite the presence of significant donor amino acids in the final sequence.

By direct analogy, the speciesisation of antibodies for use in species other than humans is similarly compromised by the requirement to make framework changes that maintain the binding specificity of the modified antibody, while reducing the immunogenicity of the resultant antibody in the target species of choice. From a commercial perspective, the use of antibodies in species other than humans is desirable because of the range of common disease processes that could usefully be treated, in particular, in high-value species such as companion animals (cats and dogs) and endurance animals (horses and camels), or to improve meat quality in food producing animals, such as cows, sheep, pigs and chickens. Accordingly, methods that enable simpler conversion of antibodies for use in these species would be highly desirable.

There is therefore a need for improved methods of modifying a donor antibody in order that it can be administered to a target subject without the resulting antibody exhibiting a loss in binding affinity to the target antigen, while minimizing changes from those of the recipient antibody sequences in order to reduce the likelihood of neutralising antibodies being raised there against. It follows that a method that converts a donor antibody sequence to a target species sequence with a minimum number of changes to achieve a target species framework structure with minimum impact on CDR structure would be highly desirable.

Pelat & Thullier (2009) mAbs 1(4):377-381 describe non-human primate immune libraries combined with germline humanization.
WO2006/131951 describes molecules that are able to inhibit the binding between NGF and the TrkA receptor as analgesics with prolonged effect.
WO2005/061540 describes a method for the humanization of antibodies and humanized antibodies thereby obtained.
WO2010/027488 describes heterochimeric antibodies.
Lazar et al. (2007) Molecular Immunology 44:1986-1998 describes a molecular immunology approach to antibody humanization and functional optimization. Williams et al. (2010) Chapter 21 in Antibody Engineering Vol. 1 (Edited by Kontermann & Dübel) describe humanising antibodies by CDR grafting. Covaceuszach et al. (2012) PLos ONE 7(3):e32212:1-12 describe a single cycle structure-based humanization of an anti-nerve growth factor therapeutic antibody.

### Summary of the invention

The invention is defined by the claims. Those aspects/ instances of the present disclosure which constitute the invention are defined by the claims.

### Summary of the disclosure

The present disclosure describes methods to modify a donor antibody for use in a target species so that the resultant antibody does not contain any amino acid at any position within the framework regions which would be foreign at that position in that species. The modified antibody will therefore retain the specificity and affinity of the donor antibody, but at the same time will be modified so that no potentially foreign epitopes will be created. The modified antibody will therefore not be seen as foreign in the target species and hence will not induce an immune response which could lead to a neutralisation of its efficacy, especially following long term administration. The method whereby this can be achieved overcomes all the disadvantages inherent in earlier methods and yet is characterised by a remarkable simplicity and elegance.

Following extensive efforts, the present inventor has surprisingly developed a method for altering a donor antibody or an antigen binding fragment derived therefrom such that it is completely or significantly non-immunogenic when administered to a target species, said target species being a different species to that from which the donor antibody was derived. Typically, neutralising antibodies are not raised against the resulting antibody following administration to a subject. Furthermore, the alteration of the antibody to make it non-immunogenic would not result in a reduction in binding specificity or affinity to the intended target.

According to a first aspect of the present disclosure there is provided method of producing a non-immunogenic immunoglobulin for administration to a target species, the method comprising the steps of:
- identifying a donor immunoglobulin from a species other than the target species, wherein the donor immunoglobulin has binding specificity to a target epitope present in the target species,
- determining an amino acid sequence of framework regions of heavy and/or light chain variable domains of the donor immunoglobulin,
- comparing each amino acid residue of the amino acid sequence of the framework regions of the heavy and/or light chain variable domains of the donor immunoglobulin with an amino acid residue present at a corresponding position in an amino acid sequence of framework regions of one or more immunoglobulins derived from the target species to identify one or more amino acid residues within the amino acid sequence of the framework regions of the heavy and/or light chain variable domains of the donor immunoglobulin that is not present at the corresponding position in the amino acid sequence of the framework regions of at least one of the one or more immunoglobulins derived from the target species, and
- substituting the one or more identified amino acid residues present in the amino acid sequence of the framework regions of the heavy and/or light chain variable domains of the donor immunoglobulin, but not present at the corresponding position in the amino acid sequence of the framework regions of at least one of the one or more immunoglobulins derived from the target species, with an amino acid residue which is present at the corresponding position in the amino acid sequence of the framework regions of at least one of the one or more immunoglobulins derived from the target species.

The method leaves unaltered (i.e. unsubstituted) any amino acid which is present at a specific framework (FW) region position in the donor immunoglobulin which is also present in at least one of the corresponding framework region positions in immunoglobulins derived from the target species. In certain instances, the amino acid sequences of the framework regions of the immunoglobulins of the target species comprise a pool of residues which can be compared to the amino acid residue present at the corresponding position in the donor immunoglobulin. Typically such a pool comprises positional specific amino acid residues derived from a plurality of target species immunoglobulins. Typically the pool comprises framework region sequence data derived from as many immunoglobulins of a target species as possible, and, if viable, all framework region sequences of all immunoglobulins known for a particular species which are present in published databases.

The amino acid sequences of the framework regions of immunoglobulins derived from different species can be derived from a number of publically available databases which will be well known to the person skilled in the art. For example, the databases held by the National Center for Biotechnology Information (NCBI) contain immunoglobulin sequence information for antibodies derived from a wide variety of species. Further databases may include any database which comprises publicly available germline and expressed cDNA sequences and may include journal publications or databases such as, but not limited to, the Kabat database of immunoglobulin sequences (URL: www.kabatdatabase.com) and V BASE, the human antibody database (http://vbase.mrc-cpe.cam.ac.uk/). Procedures to prepare a table of possible target amino acids is routine to the skilled person.

Although the comparison may be between the donor sequence and a single member of the target sequence, it will be obvious that comparison with a pool of target sequences is preferred because this will expand the number of natural options at each Kabat position in the target species. Not only will this increase the chance of a "match" between the donor and the target, but it will also expand the options for replacement where a match does not exist.

As herein defined, a non-immunogenic immunoglobulin is an immunoglobulin which does not have an immune response raised there against when it is administered to a target species. In particular, a humoral (antibody mediated) response is not mediated against the antibody, particularly against epitopes comprising amino acid residues derived from the framework (FW) regions.

Where donor and target amino acids differ at any Kabat number, the amino acid must be chosen from one known to be natural at that position in the target. This will lead to a number of possible sequences, any of which may lead to a preferred or at least suitable sequence of the disclosure. Where substitution of an amino acid residue present in a donor immunoglobulin framework region is required, typically this is undertaken using the principle of conservative substitution. Typically, conservative substitution requires replacement of the amino acid with a homologous amino acid residue, that is a residue which shares similar characteristics or properties. Such a replacement may be known as homologous substitution.

In determining whether a substituted amino acid can be replaced with a conserved amino acid, an assessment may typically be made of factors such as, but not limited to, (a) the structure of the polypeptide backbone in the area of the substitution, for example, a sheet or helical conformation, (b) the charge or hydrophobicity of the molecule at the target site, and/or (c) the bulk of the side chain(s). If a residue can be substituted with a residue which has common characteristics, such as a similar side chain or similar charge or hydrophobicity, then such a residue is preferred as a substitute.

When considering whether a donor immunoglobulin derived residue which is not present in the pool of target species immunoglobulin residues present at a corresponding position can be conservatively substituted, it may be preferable to assess whether a homologous amino acid is available in the pool of corresponding residues derived from the target species for that specific position based on the amino acids being grouped together according to similarities in the properties of their side chains (A. L. Lehninger, in Biochemistry, 2nd Ed., 73-75, Worth Publishers, New York (1975)). For example, the following groups can be determined: (1) non-polar: Ala (A), Val (V), Leu (L), Ile (I), Pro (P), Phe (F), Trp (W), Met (M); (2) uncharged polar: Gly (G), Ser (S), Thr (T), Cys (C), Tyr (Y), Asn (N), Gln (Q); (3) acidic: Asp (D), Glu (E); and (4) basic: Lys (K), Arg (R), His (H). Hence the substitution of an amino acid residue with another present in the same group is preferred.

Alternatively, the amino acids may be grouped as follows: (1) aromatic: Phe (F), Trp (W), Tyr (Y); (2) apolar: Leu (L), Val (V), Ile (I), Ala (A), Met (M); (3) aliphatic: Ala (A), Val (V), Leu (L), Ile (I); (4) acidic: Asp (D), Glu (E); (5) basic: His (H), Lys (K), Arg (R); and (6) polar: Gln (Q), Asn (N), Ser (S), Thr (T), Tyr (Y). Again, the substitution of an amino acid residue with another present in the same group is preferred.

Alternatively, amino acid residues may be divided into groups based on common side-chain properties: (1) hydrophobic: Met (M), Ala (A), Val (V), Leu (L), Ile (I); (2) neutral hydrophilic: Cys (C), Ser (S), Thr (T), Asn (N), Gin (Q); (3) acidic: Asp (D), Glu (E); (4) basic: His (H), Lys (K), Arg R); (5) residues that influence chain orientation: Gly (G), Pro (P); and (6) aromatic: Trp (W), Tyr (Y), Phe (F). Again, the substitution of an amino acid residue with another present in the same group would be preferred.

Accordingly, conservative substitutions will entail exchanging (substituting) a member of one of these classes for another member of that same class. In certain instances, the amino acid residue which is introduced into the donor immunoglobulin framework region sequence is the consensus amino acid defined at that specific position from the pool of immunoglobulins derived from the target species. The consensus amino acid is the amino acid which is most commonly found at that position in immunoglobulins which comprise the collection of target species immunoglobulins which contribute to the pool.

Typically the substitution of the framework region residues does not result in a reduction in the binding of the immunoglobulin to its intended ligand. In particular, there is no reduction in the binding affinity or specificity.

In certain instances the method further comprises the step of replacing at least one and preferably all of the constant domains of the heavy and/or light chain of the donor immunoglobulin with equivalent heavy and/or light chains derived from an immunoglobulin derived from the target species. In certain instances, the target species derived constant domains are of the antibody subtype Immunoglobulin G (IgG).

In certain instances, the pool of donor immunoglobulin amino acid sequences used to assess the amino acid residues of the framework regions may be restricted to sub-types of immunoglobulins derived from the target species, e.g. from kappa or lambda light chains.

Without wishing to be bound by theory, the method results in only a minimum number of essential changes (amino acid substitutions) being made to the donor immunoglobulin framework region sequences, whilst ensuring that all of the amino acids in the resulting framework region sequence align with those of the target species. This consequently minimises structural changes resulting from the modification of the antibody from a donor immunoglobulin to an antibody which will be completely or substantially non-immunogenic when administered to a target subject. Due to the method involving the substitution of the fewest possible residues, the method may be referred to as Parsimonious Essential Translation (PET). By extension, the changes made to a donor immunoglobulin to allow it to be non-immunogenic when administered to a target animal species, may be referred to as PETisation and the resulting antibody may be referred to as being PETized. This process may, for example, result in the speciesisation of an existing human, mouse or rat antibody such that it may be administered to another target species, in particular an animal species, such as human, dog, cat, or horse without neutralising antibodies being raised there against.

In certain instances, the target species is a mammalian target species. In one instance, the target mammalian species is an animal, in particular a companion animal such as, but not limited to, a dog, cat or horse or a livestock animal. In further instances, the mammalian target species is a human.

In certain instances, the target epitope is nerve growth factor (NGF) or tumour necrosis factor (TNF).

In various further instances there is provided a composition containing an immunoglobulin provided by the method of a foregoing aspect of the disclosure, or an antigen binding fragment thereof. The composition may further comprises at least one pharmaceutically acceptable diluent or carrier.A yet further aspect provides use of an immunoglobulin produced by the method of a foregoing aspect of the disclosure, or an antigen binding fragment thereof, in the preparation of a medicament for the treatment or prevention of disease. In various further aspects, the present disclosure extends to the use of the foregoing immunoglobulins,or an antigen binding fragment thereof, in therapeutic and diagnostic methods. The disclosure further extends to an immunoglobulin produced according to any one of claims 1 to 15,or an antigen binding fragment thereof, for use in the treatment or prevention of disease.

A yet further aspect of the disclosure relates to the administration of an immunoglobulin produced in accordance with any of the methods defined herein, or an antigen binding fragment thereof, to a subject, in particular a mammalian subject, for the treatment or prevention of disease.

### De-immunisation

In various further aspects, the disclosure extends to the modification of a therapeutic immunoglobulin in order to render it non-immunogenic when administered to a specific species. Said modification may be applied to a chimeric antibody, an antibody produced by a CDR grafting technique or a humanised antibody.

In a further aspect, the disclosure therefore provides a method for modifying a therapeutic immunoglobulin to make it non-immunogenic, the method comprising the steps of:
- providing a donor therapeutic immunoglobulin,
- determining an amino acid sequence of at least one framework region of variable domains of light and/or heavy chains of the donor immunoglobulin
- obtaining a pool of amino acid sequences relating to at least one framework region of variable domains of light and/or heavy chains of immunoglobulins derived from a target species to which the immunoglobulin is to be administered,
- comparing amino acid residues of the amino acid sequence of the at least one framework region from the light and/or heavy chains of the donor immunoglobulin to amino acid residues having the same Kabat numbering in the pool of amino acid sequences, and
- substituting any amino acid residue of the amino acid sequence of the at least one framework region from the light and/or heavy chains of the donor immunoglobulin with an amino acid residue having the same Kabat numbering in the pool of amino acid sequences where the amino acid residue present in the amino acid sequence of the at least one framework region from the light and/or heavy chains of the donor immunoglobulin differs from the amino acid residues having the same Kabat numbering in the pool of amino acid sequences.

Typically re-humanising therapeutic immunoglobulins using this methodology results in an immunoglobulin that is less immunogenic than the unaltered donor therapeutic antibody. Furthermore, the modified antibody retains its binding affinity and specificity. Hence, the resulting modified antibody is more therapeutically useful.

In certain instances, the resulting PETised antibody, or binding fragment thereof, binds to the desired target epitope with a binding affinity K_{D} of 1x10⁻⁸ or less.

A yet further aspect of the disclosure extends to the provision of at least one framework region for use in an immunoglobulin heavy and/or light chain variable domain. The method of this aspect of the disclosure may have particular utility in a process such as humanisation of an antibody, or in an equivalent process used to modify an antibody to de-immunise it prior to its administration to a species other than a human. The framework regions provided by this aspect of the disclosure may be introduced into an antibody which is either about to undergo humanisation or a similar speciesisation process, or can be retrospectively introduced into an antibody which has previously undergone speciesisation. Specifically, the modified framework regions may be introduced into an antibody which has undergone, or which is about to undergo, modification by virtue of a process such as CDR grafting, or which is a chimeric antibody wherein the Fab region of the antibody is derived from a first species and the Fc region of the antibody is derived from a second species.

Accordingly, this further aspect provides a method of modifying an amino acid sequence of at least one framework region of a donor immunoglobulin heavy and/or light chain variable domain, said method comprising the steps of:
- determining the amino acid sequence of the at least one framework region sequence of the donor immunoglobulin;
- on a residue by residue basis, comparing specific amino acid residues at each position of the at least one framework region of the donor immunoglobulin to a database comprising a pool of amino acid residues found at a corresponding amino acid position in framework region sequences found in antibodies derived from a species to whom the modified source immunoglobulin is to be administered;
- substituting any amino acid residues which are present at a specific position in the at least one framework region of the donor immunoglobulin, but not in the pool of amino acid residues found at the corresponding amino acid position, with an amino acid residue which is present in the pool of amino acid residues found at the corresponding amino acid position; and
- leaving unaltered any amino acid residues which are present at a specific position in the at least one framework region of the donor immunoglobulin and also present in the pool of amino acid residues found at the corresponding amino acid position.

Typically, any replaced amino acid residues are substituted with an amino acid residue which is the most homologous to the amino acid residue being replaced. As defined hereinbefore, homologous groups of amino acid residues are known. If a homologous amino acid residue is not present in the pool, then the amino acid may be substituted with the amino acid residue which occurs most frequently at that specific position, the so called consensus amino acid residue.

In certain instances, the method extends to a method for producing a modified antibody which comprises the steps of expressing the modified framework region sequence along with complementarity determining regions (CDRs) and heavy and/or light chain constant domains such that a heterotetrameric antibody is produced comprising said modified framework region sequences.

Certain further aspects of the present disclosure extend to providing an oligonucleotide which expresses the amino acid sequence of the modified framework region sequence and to the expression of same in a host cell.

A yet further aspect of the present disclosure relates to a method for producing a therapeutic antibody with non-immunogenic framework region sequences, said method comprising the steps of:
- identifying framework region amino acid residues which are to be substituted by comparing amino acid sequences of framework regions to pools of corresponding amino acid residues present at corresponding amino acid positions in a plurality of immunoglobulins derived from a species to which the therapeutic antibody is to be administered to identify one or more amino acid residues which differ at a specific position; and
- substituting the one or more identified amino acid residues with an amino acid residue present in the pool of corresponding amino acid residues.

In certain instances the identification of whether a framework region amino acid residue is present in the corresponding positional pool of amino acid residues from the species to which the antibody will be administered is achieved by performing an alignment of the sequence and the pool residues. Essentially, the residues which are substituted do not reduce the binding activity of the resulting modified antibody. That is, the amino acid which is substituted may be substituted for a different amino acid without significantly affecting the binding characteristics of the antibody. This is primarily achieved by substituting the amino acid with a homologous amino acid, that is, an amino acid with similar or related characteristics, such as size, polarity/charge or hydrophobicity of the molecule at the target site, or the bulk of the side chain. Alternatively, the residue may be substituted with the consensus residue which occurs in the target species at a corresponding position.

The amino acid residues which are substituted (or substitutable) are present at positions which may be known as variant tolerant positions. That is, the substitution of that residue for another residue does not alter the binding specificity of the complementarity determining regions which are interposed between the framework regions. Such a substitution may be deemed necessary due to the fact that a residue present at a specific position of a framework region in one species may be absent at a corresponding position in the framework region sequences of a second species. Hence, the amino acid may cause an immunogenic response to be raised there against by virtue of forming an epitope which is viewed as foreign by the immune system of a species where that amino acid residue is not normally present at that position of a framework region sequence. Using the methodology of substituting that outlier residue with a homologous residue or consensus residue which is present in the target species, the potentially foreign epitope can be altered to form an epitope which will not be recognised as foreign. Removal of all such epitopes from the framework region of an antibody can therefore prevent a humoral response being raised against that portion of the antibody when administered to a subject which is of a different species to the species from which the antibody was initially derived.

### Ubiquitous species-specific framework region production

The inventor has further defined a series of framework regions (FR) which can be combined with complementarity determining regions (CDRs) to form non-immunogenic PETised heavy and light chain variable domains. Each of the heavy and light chain domains has 4 framework regions, designated FR1, FR2, FR3 and FR4. This methodology can be applied to de-immunise any antibody (immunoglobulin) in order that it can be administered to a desired species. However, for the purposes of exemplification only, the undernoted examples will illustrate the production and use of such framework regions in human, canine, feline and equine-based antibodies.

### Antibody structure

An antibody molecule may comprise a heavy chain variable domain comprising CDR1, CDR2 and CDR3 regions and associated interposed framework regions. The heavy chain variable domain (VH) CDRs are known as VHCDRs, with these CDRs being found at the following positions according to the Kabat numbering system: VHCDR1 - Kabat residues 31-35, VHCDR2 - Kabat residues 50-65 and VHCDR3 - Kabat residues 95-102 (Kabat EA et al. (1991) Sequences of proteins of immunological interest, 5th edition. Bethesda: US Department of Health and Human Services).

Furthermore, an antibody further comprises a light chain variable domain comprising CDR1, CDR2 and CDR3 regions and associated interposed framework regions. The light chain variable domain (VL) CDRs are known as VLCDRs, with these CDRs being found at the following amino acid residue positions according to the Kabat numbering system: VLCDR1 - Kabat residues 24-34, VLCDR2 - Kabat residues 50-56 and VLCDR3 - Kabat residues 89-97.

A light or heavy chain variable domain comprises four framework regions, FR1, FR2, FR3 and FR4, interposed with CDRs in the following arrangement: FR1-CDR1-FR2-CDR2-FR3-CDR3-FR4.

A yet further aspect of the present disclosure provides an immunoglobulin for administration to a target species, comprising:
- light and heavy chain constant domains derived from an immunoglobulin of the target species,
- complementary determining regions (CDRs) derived from a donor immunoglobulin wherein the donor immunoglobulin specifically binds to a ligand which is present in the target species, and
- framework regions derived from the donor immunoglobulin wherein any amino acid residues not also present at a corresponding position in any immunoglobulin from the target species are substituted with an amino acid which is found in the corresponding position in the target species.

Typically no amino acid substitutions are made to the CDR regions of the antibody. Furthermore, in certain instances the framework regions comprise no more than 7 consecutive amino acid residues being substituted with residues from the target species. Furthermore, in certain instances the framework regions comprise no more than 5 consecutive amino acid residues being substituted with residues from the target species. Furthermore, in certain instances the framework regions comprise no more than 3 consecutive amino acid residues being substituted with residues from the target species.

Furthermore, in certain instances no more than 10 amino acid residues of the donor immunoglobulin framework region (heavy chain FR1, FR2, FR3 and FR4 and light chain FR1, FR2, FR3 and FR4) are substituted with residues from the target species. Furthermore, in certain instances no more than 7 amino acid residues of the donor immunoglobulin framework region (heavy chain FR1, FR2, FR3 and FR4 and light chain FR1, FR2, FR3 and FR4) are substituted with residues from the target species. Furthermore, in certain instances no more than 5 amino acid residues of the donor immunoglobulin framework region (heavy chain FR1, FR2, FR3 and FR4 and light chain FR1, FR2, FR3 and FR4) are substituted with residues from the target species.

In certain instances the target species is a mammalian species. In particular the target species may be a human, canine, feline or equine.

Accordingly, in instances where the immunoglobulin is to be administered to a canine as the target species, the immunoglobulin comprises constant domain regions derived from a canine derived antibody or antibodies, and the residues substituted into the donor immunoglobulin framework regions are derived from corresponding canine framework regions amino acid residues.

In instances where the immunoglobulin is to be administered to a feline as the target species, the immunoglobulin comprises constant domain regions derived from a feline derived antibody or antibodies, and the residues substituted into the donor immunoglobulin framework regions are derived from corresponding feline framework regions amino acid residues.

In instances where the immunoglobulin is to be administered to an equine as the target species, the immunoglobulin comprises constant domain regions derived from an equine derived antibody or antibodies, and the residues substituted into the donor immunoglobulin framework regions are derived from corresponding equine framework regions amino acid residues.

In instances where the immunoglobulin is to be administered to a human as the target species, the immunoglobulin comprises constant domain regions derived from a human derived antibody or antibodies, and the residues substituted into the donor immunoglobulin framework regions are derived from corresponding human framework regions amino acid residues.

In various further instances there is provided a composition containing an immunoglobulin of the foregoing aspect of the disclosure, or an antigen binding fragment thereof. The composition may further comprises at least one pharmaceutically acceptable diluent or carrier.A yet further aspect provides use of an immunoglobulin of the foregoing aspect of the disclosure, or an antigen binding fragment thereof, in the preparation of a medicament for the treatment or prevention of disease. In various further aspects, the present disclosure extends to the use of the foregoing immunoglobulins,or an antigen binding fragment thereof, in therapeutic and diagnostic methods. The disclosure further extends to an immunoglobulin of the foregoing aspect of the disclosure,or an antigen binding fragment thereof, for use in the treatment or prevention of disease.

A yet further aspect of the disclosure relates to the administration of an immunoglobulin of the foregoing aspect of the disclosure, or an antigen binding fragment thereof, to a subject, in particular a mammalian subject, for the treatment or prevention of disease.

A yet further aspect of the present disclosure provides a method of producing an immunoglobulin comprising the steps of:
- introducing nucleotides encoding heavy and light chains into a cell, wherein the nucleotides encode light and chain variable domains which comprise framework regions produced according to the methods defined herein, and
- expressing the nucleotides in a cell to produce the immunoglobulin.

A yet further aspect of the disclosure provides a method of producing an immunoglobulin which is substantially non-immunogenic when administered to a target species, the method comprising the steps of:
(1) comparing sequences of donor immunoglobulin heavy and/or light chain framework regions to a collection of sequences of target species immunoglobulin heavy and/or light chain framework regions;
(2) substituting any amino acid residues which are present at a specific position in the donor immunoglobulin framework region sequences, but not present at a corresponding position in the collection of target species immunoglobulin framework region sequences, with an amino acid residue which is present at a corresponding position in the collection of target species immunoglobulin framework region sequences;
(3) synthesizing a DNA segment encoding heavy and/or light chain variable regions, comprising CDRs from the donor immunoglobulin variable regions and the substituted framework regions, together with species-appropriate heavy and/or light chain constant domains;
(4) introducing the DNA segment into a cell; and
(5) expressing the DNA segment in the cell to produce the immunoglobulin.

In certain instances, the method further comprises the step of sequencing the donor immunoglobulin light chain and heavy chain variable region. In certain instances, the method further comprises the step of purifying the immunoglobulin.

In certain instances, the method further comprises the step of formulating the immunoglobulin in a pharmaceutically acceptable carrier or diluent.

A further aspect of the present disclosure provides a neutralising antibody or an antigen binding fragment thereof which is capable of specifically binding to human nerve growth factor (NGF) wherein the antibody or antigen binding fragment comprises a light chain variable region comprising, consisting of or consisting essentially of the amino acid sequence of SEQ ID NO:13 or an amino acid sequence which has an identity of at least 85%, 90%, 95% or 99% thereto and/or a heavy chain variable region comprising, consisting of or consisting essentially of the amino acid sequence of SEQ ID NO:14 or an amino acid sequence which has an identity of at least 85%, 90%, 95% or 99%thereto. In certain instances said identity is over a length of at least about 15 amino acids, preferably about 20 amino acids, more preferably about 25 amino acids.

The antibody may be prepared using a method of the disclosure.

In certain instances, the light chain comprises, consists of or consists essentially of the amino acid sequence of SEQ ID NO:25, or an amino acid sequence which has an identity of at least 85%, 90%, 95% or 99% thereto.In certain instances said identity is over a length of at least about 15 amino acids, preferably about 20 amino acids, more preferably about 25 amino acids.

In certain instances, the heavy chain comprises, consists of or consists essentially of the amino acid sequence of SEQ ID NO:24, or an amino acid sequence which has a sequence identity of at least 85%, 90%, 95% or 99% thereto. In certain instances said identity is over a length of at least about 15 amino acids, preferably about 20 amino acids, more preferably about 25 amino acids.

The inventor has further defined a series of framework regions (FR) which can be combined with complementarity determining regions (CDRs) to form humanised heavy and light chain variable domains. Each of the human heavy and light chain domains has 4 framework regions, designated FR1, FR2, FR3 and FR4.

Accordingly, also provided is a neutralising antibody or an antigen binding fragment thereof which is capable of specifically binding to human nerve growth factor (NGF) wherein the antibody or antigen binding fragment comprises a light chain variable region comprising at least one of:
an FR1 framework region consisting of or comprising the amino acid sequence of SEQ ID NO:60,
an FR2 framework region consisting of or comprising the amino acid sequence of SEQ ID NO:61,
an FR3 framework region consisting of or comprising the amino acid sequence of SEQ ID NO:62, and
an FR4 framework region consisting of or comprising the amino acid
sequence of SEQ ID NO:63,
and/or a heavy chain variable region comprising at least one of:
an FR1 framework region consisting of or comprising the amino acid sequence of SEQ ID NO:64,
an FR2 framework region consisting of or comprising the amino acid sequence of SEQ ID NO:65,
an FR3 framework region consisting of or comprising the amino acid sequence of SEQ ID NO:66, and
an FR4 framework region consisting of or comprising the amino acid sequence of SEQ ID NO:67.

In certain instances, the light chain comprises all of light chain FR1, FR2, FR3 and FR4 and/or the heavy chain comprises all of heavy chain FR1, FR2, FR3 and FR4.

In certain instances, the antibody or binding fragment of the above aspects of the disclosure specifically binds to human NGF with a binding affinity having an equilibrium dissociation constant (K_{D}) of 1x10⁻⁸ or less.

In certain instances, the antibody or binding fragment of the above aspects of the disclosure inhibits the ability of human NGF to bind to the p75 or the TrkA human NGF receptors.

Preferably, the antibody or binding fragment of the above aspects of the disclosure is not immunogenic in humans.

Typically the antibody of the above aspects of the disclosure comprises light chain and/or heavy chain constant domains derived from an immunoglobulin derived from a human.

In certain instances, the binding fragment of the above aspects of the disclosure is selected from the group consisting of a single chain Fv (scFv) antibody fragment, a Fab antibody fragment, a Fab' antibody fragment and a F(ab')₂ antibody fragment.

In various further aspects, the present disclosure extends to an isolated nucleic acid which encodes the antibody or antigen binding fragments of the disclosure.

Accordingly, a yet further aspect of the disclosure provides an isolated nucleic acid that encodes an antibody or antigen binding fragment according to any of the foregoing aspects of the disclosure.

In certain instances, the polynucleotide encodes the light chain variable domain of an anti-NGF antibody or antigen binding fragment having the amino acid sequence of SEQ ID NO:13, or an amino acid sequence which has an identity of at least 85%, 90%, 95% or 99% thereto.

Also provided is an isolated nucleic acid that encodes the light chain of an anti-NGF antibody or antigen binding fragment having the amino acid sequence of SEQ ID NO:25, or an amino acid sequence which has an identity of at least 85%, 90%, 95% or 99% thereto.

In certain instances, the polynucleotide encodes the heavy chain variable domain of an anti-NGF antibody or antigen binding fragment having the amino acid sequence of SEQ ID NO:14, or an amino acid sequence which has an identity of at least 85%, 90%, 95% or 99% thereto.

Also provided is an isolated nucleic acid that encodes the heavy chain of an anti-NGF antibody or antigen binding fragment having the amino acid sequence of SEQ ID NO:24, or an amino acid sequence which has an identity of at least 85%, 90%, 95% or 99% thereto.

In certain instances, the isolated nucleic acid further comprises a nucleic acid encoding one or more regulatory sequences operably linked thereto.

In a further aspect there is provided an expression vector comprising a polynucleotide encoding a heavy and/or light chain variable domain or a heavy and/or light chain of the disclosure. In certain instances the expression vector further comprises one or more regulatory sequences. In certain instances the vector is a plasmid or a retroviral vector.

A yet further aspect provides a host cell incorporating the expression vector of the foregoing aspect of the disclosure. A further aspect of the disclosure provides a host cell which produces the antibody of any of the foregoing aspects of the disclosure.

A yet further aspect of the disclosure provides a method for producing a humanised NGF neutralising antibody, the method comprising the step of culturing the host cell of the foregoing aspect of the disclosure to allow the cell to express the humanised NGF neutralising antibody.

A yet further aspect of the present disclosure provides a method of producing an NGF neutralising antibody according to the disclosure comprising the steps of expressing one or more of the polynucleotides / nucleic acids or vectors of the foregoing aspects of the disclosure which express the light and/or heavy chains of the antibodies of the disclosure in a suitable host cell, recovering the expressed polypeptides, which may be expressed together in a host cell, or separately in different host cells, and isolating antibodies.

In certain instances, there is provided an antibody or binding fragment of the disclosure and at least one pharmaceutically acceptable diluent or carrier.

A yet further aspect of the present disclosure provides use of the antibody or binding fragment, nucleic acid, pharmaceutical composition or expression vector of the above aspects of the disclosure in the preparation of a medicament for the treatment or prevention of disease, such as arthritis, or for the treatment, prevention of amelioration of pain, such as pain associated with disease (e.g. neuropathic pain, post-operative pain, chronic pain, oncologic pain, etc). In various further aspects, the present disclosure extends to the use of the antibody or binding fragment of the above aspects of the disclosure in therapeutic and diagnostic methods.

A yet further aspect of the disclosure relates to the administration of the antibody or binding fragment, nucleic acid, pharmaceutical composition or expression vector of the above aspects of the disclosure to a subject, in particular a mammalian subject, for the treatment or prevention of disease (e.g. arthritis) or pain.

In certain instances, the disease is a condition caused by, associated with or resulting in increased sensitivity to nerve growth factor (NGF). In certain instances, the disease relates to a tumour induced to proliferate by NGF (e,g, an osteosarcoma).

In certain instances, the foregoing methods of the disclosure further comprise the step of co-administering at least one further agent which may enhance and/or complement the effectiveness of the anti-NGF antibody of the disclosure. For example, the antibody or antigen binding fragment thereof may be co-administered along with at least one analgesic, NSAID, opioid, corticosteroid, steroid, hyaluronan or hyaluronic acid.

In a yet further aspect there is provided a cell line, or a derivative or progeny cell thereof, that produces anti-human NGF neutralising monoclonal antibodies, or fragments thereof according to the disclosure.

A yet further aspect of the present disclosure provides a kit for the treatment of pain in humans, or for the treatment of a condition associated with pain, or for the treatment, amelioration or inhibition of pain associated with osteoarthritis, rheumatoid arthritis and inflammation, comprising an anti- NGF antibody according to any of the foregoing aspects of the disclosure and instructions for use of the same.

A yet further aspect of the present disclosure provides a diagnostic kit for the detection of an anti-human NGF monoclonal antibody in fluids in vitro, ex vivo and in vivo, for use in determining the concentration of said antibody. The kit may comprise any of the antibodies of the disclosure or a binding fragment thereof. The kit may comprise instructions for use of same.

A further aspect of the present disclosure provides a neutralising antibody or an antigen binding fragment thereof which is capable of specifically binding to caninetumour necrosis factor (TNF) wherein the antibody or antigen binding fragment comprises a light chain variable region comprising, consisting of or consisting essentially of the amino acid sequence of SEQ ID NO:71 or an amino acid sequence which has an identity of at least 85%, 90%, 95% or 99% thereto and/or a heavy chain variable region comprising, consisting of or consisting essentially of the amino acid sequence of SEQ ID NO:16 or an amino acid sequence which has an identity of at least 85%, 90%, 95% or 99%thereto. In certain instances said identity is over a length of at least about 15 amino acids, preferably about 20 amino acids, more preferably about 25 amino acids.

The antibody may be prepared using a method of the disclosure.Typically the antibody of the above aspects of the disclosure comprises light chain and/or heavy chain constant domains derived from an immunoglobulin derived from a canine. In certain instances, the heavy chain comprises, consists of or consists essentially of the amino acid sequence of SEQ ID NO:18, 19, 20 or 21, or an amino acid sequence which has a sequence identity of at least 85% , 90%, 95% or 99% thereto.In certain instances said identity is over a length of at least about 15 amino acids, preferably about 20 amino acids, more preferably about 25 amino acids.

In certain instances, the antibody or binding fragment of the above aspects of the disclosure specifically binds to canine TNF with a binding affinity having an equilibrium dissociation constant (K_{D}) of 1x10⁻⁸ or less. Preferably, the antibody or binding fragment of the above aspects of the disclosure is not immunogenic in canines.

In certain instances, the binding fragment of the above aspects of the disclosure is selected from the group consisting of a single chain Fv (scFv) antibody fragment, a Fab antibody fragment, a Fab' antibody fragment and a F(ab')₂ antibody fragment.

In various further aspects, the present disclosure extends to an isolated nucleic acid which encodes the antibody or antigen binding fragments of the disclosure.

Accordingly, a yet further aspect of the disclosure provides an isolated nucleic acid that encodes an antibody or antigen binding fragment according to any of the foregoing aspects of the disclosure.

In certain instances, the polynucleotide encodes the light chain variable domain of an anti-TNF antibody or antigen binding fragment having the amino acid sequence of SEQ ID NO:71, or an amino acid sequence which has an identity of at least 85%, 90%, 95% or 99% thereto.

In certain instances, the isolated nucleic acid further comprises a nucleic acid encoding one or more regulatory sequences operably linked thereto. In a further aspect there is provided an expression vector comprising a polynucleotide encoding a heavy and/or light chain variable domain or a heavy and/or light chain of the disclosure. In certain instances the expression vector further comprises one or more regulatory sequences. In certain instances the vector is a plasmid or a retroviral vector.A yet further aspect provides a host cell incorporating the expression vector of the foregoing aspect of the disclosure. A further aspect of the disclosure provides a host cell which produces the antibody of any of the foregoing aspects of the disclosure.

A yet further aspect of the disclosure provides a method for producing a caninised TNF neutralising antibody, the method comprising the step of culturing the host cell of the foregoing aspect of the disclosure to allow the cell to express the caninised TNF neutralising antibody.

A yet further aspect of the present disclosure provides a method of producing an TNFneutralising antibody according to the disclosure comprising the steps of expressing one or more of the polynucleotides / nucleic acids or vectors of the foregoing aspects of the disclosure which express the light and/or heavy chains of the antibodies of the disclosure in a suitable host cell, recovering the expressed polypeptides, which may be expressed together in a host cell, or separately in different host cells, and isolating antibodies.

In certain instances, there is provided an antibody or binding fragment of the disclosure and at least one pharmaceutically acceptable diluent or carrier.

A yet further aspect of the present disclosure provides use of the antibody or binding fragment, nucleic acid, pharmaceutical composition or expression vector of the above aspects of the disclosure in the preparation of a medicament for the treatment or prevention of disease, in particular any condition caused by, associated with or resulting in increased expression of canine TNF or increased sensitivity to TNF in a canine. In various further aspects, the present disclosure extends to the use of the antibody or binding fragment of the above aspects of the disclosure in therapeutic and diagnostic methods.

A yet further aspect of the disclosure relates to the administration of the antibody or binding fragment, nucleic acid, pharmaceutical composition or expression vector of the above aspects of the disclosure to a canine, for the treatment or prevention of disease.

In a yet further aspect there is provided a cell line, or a derivative or progeny cell thereof, that produces anti-canine TNF neutralising monoclonal antibodies, or fragments thereof according to the disclosure.

A further aspect of the present disclosure provides a neutralising antibody or an antigen binding fragment thereof which is capable of specifically binding to canine nerve growth factor (NGF) wherein the antibody or antigen binding fragment comprises a light chain variable region comprising, consisting of or consisting essentially of the amino acid sequence of SEQ ID NO:1 or an amino acid sequence which has an identity of at least 85%, 90%, 95% or 99% thereto and/or a heavy chain variable region comprising, consisting of or consisting essentially of the amino acid sequence of SEQ ID NO:69 or an amino acid sequence which has an identity of at least 85%, 90%, 95% or 99%thereto. In certain instances said identity is over a length of at least about 15 amino acids, preferably about 20 amino acids, more preferably about 25 amino acids.

The antibody may be prepared using a method of the disclosure.

In certain instances, the light chain comprises, consists of or consists essentially of the amino acid sequence of SEQ ID NO:7, or an amino acid sequence which has an identity of at least 85%, 90%, 95% or 99% thereto. In certain instances said identity is over a length of at least about 15 amino acids, preferably about 20 amino acids, more preferably about 25 amino acids.

In certain instances, the heavy chain comprises, consists of or consists essentially of the amino acid sequence of SEQ ID NO:70, or an amino acid sequence which has a sequence identity of at least 85%, 90%, 95% or 99% thereto. In certain instances said identity is over a length of at least about 15 amino acids, preferably about 20 amino acids, more preferably about 25 amino acids.

In certain instances, the antibody or binding fragment of the above aspects of the disclosure specifically binds to canine NGF with a binding affinity having an equilibrium dissociation constant (K_{D}) of 1x10⁻⁸ or less. In certain instances, the antibody or binding fragment of the above aspects of the disclosure inhibits the ability of canine NGF to bind to the p75 or the TrkA canine NGF receptors. Preferably, the antibody or binding fragment of the above aspects of the disclosure is not immunogenic in canines.

Typically the antibody of the above aspects of the disclosure comprises light chain and/or heavy chain constant domains derived from an immunoglobulin derived from a canine.

In certain instances, the binding fragment of the above aspects of the disclosure is selected from the group consisting of a single chain Fv (scFv) antibody fragment, a Fab antibody fragment, a Fab' antibody fragment and a F(ab')₂ antibody fragment.

In various further aspects, the present disclosure extends to an isolated nucleic acid which encodes the antibody or antigen binding fragments of the disclosure.

Accordingly, a yet further aspect of the disclosure provides an isolated nucleic acid that encodes an antibody or antigen binding fragment according to any of the foregoing aspects of the disclosure.

In certain instances, the polynucleotide encodes the light chain variable domain of an anti-NGF antibody or antigen binding fragment having the amino acid sequence of SEQ ID NO:1, or an amino acid sequence which has an identity of at least 85%, 90%, 95% or 99% thereto.

Also provided is an isolated nucleic acid that encodes the light chain of an anti-NGF antibody or antigen binding fragment having the amino acid sequence of SEQ ID NO:7, or an amino acid sequence which has an identity of at least 85%, 90%, 95% or 99% thereto.

In certain instances, the polynucleotide encodes the heavy chain variable domain of an anti-NGF antibody or antigen binding fragment having the amino acid sequence of SEQ ID NO:69, or an amino acid sequence which has an identity of at least 85%, 90%, 95% or 99% thereto.

Also provided is an isolated nucleic acid that encodes the heavy chain of an anti-NGF antibody or antigen binding fragment having the amino acid sequence of SEQ ID NO:70, or an amino acid sequence which has an identity of at least 85%, 90%, 95% or 99% thereto.

In certain instances, the isolated nucleic acid further comprises a nucleic acid encoding one or more regulatory sequences operably linked thereto.

In a further aspect there is provided an expression vector comprising a polynucleotide encoding a heavy and/or light chain variable domain or a heavy and/or light chain of the disclosure. In certain instances the expression vector further comprises one or more regulatory sequences. In certain instances the vector is a plasmid or a retroviral vector.

A yet further aspect provides a host cell incorporating the expression vector of the foregoing aspect of the disclosure. A further aspect of the disclosure provides a host cell which produces the antibody of any of the foregoing aspects of the disclosure.

A yet further aspect of the disclosure provides a method for producing a caninised NGF neutralising antibody, the method comprising the step of culturing the host cell of the foregoing aspect of the disclosure to allow the cell to express the caninised NGF neutralising antibody.

A yet further aspect of the present disclosure provides a method of producing an NGF neutralising antibody according to the disclosure comprising the steps of expressing one or more of the polynucleotides / nucleic acids or vectors of the foregoing aspects of the disclosure which express the light and/or heavy chains of the antibodies of the disclosure in a suitable host cell, recovering the expressed polypeptides, which may be expressed together in a host cell, or separately in different host cells, and isolating antibodies.

In certain instances, there is provided an antibody or binding fragment of the disclosure and at least one pharmaceutically acceptable diluent or carrier.

A yet further aspect of the present disclosure provides use of the antibody or binding fragment, nucleic acid, pharmaceutical composition or expression vector of the above aspects of the disclosure in the preparation of a medicament for the treatment or prevention of disease, such as arthritis, or for the treatment, prevention of amelioration of pain, such as pain associated with disease (e.g. neuropathic pain, post-operative pain, chronic pain, oncologic pain, etc) in a canine. In various further aspects, the present disclosure extends to the use of the antibody or binding fragment of the above aspects of the disclosure in therapeutic and diagnostic methods.

A yet further aspect of the disclosure relates to the administration of the antibody or binding fragment, nucleic acid, pharmaceutical composition or expression vector of the above aspects of the disclosure to a canine for the treatment or prevention of disease (e.g. arthritis) or pain.

In certain instances, the disease is a condition caused by, associated with or resulting in increased sensitivity to nerve growth factor (NGF). In certain instances, the disease relates to a tumour induced to proliferate by NGF (e,g, an osteosarcoma).

In certain instances, the foregoing methods of the disclosure further comprise the step of co-administering at least one further agent which may enhance and/or complement the effectiveness of the anti-NGF antibody of the disclosure. For example, the antibody or antigen binding fragment thereof may be co-administered along with at least one analgesic, NSAID, opioid, corticosteroid, steroid, hyaluronan or hyaluronic acid.

In a yet further aspect there is provided a cell line, or a derivative or progeny cell thereof, that produces anti-canine NGF neutralising monoclonal antibodies, or fragments thereof according to the disclosure.

A yet further aspect of the present disclosure provides a kit for the treatment of pain in canines, or for the treatment of a condition associated with pain, or for the treatment, amelioration or inhibition of pain associated with osteoarthritis, rheumatoid arthritis and inflammation, comprising an anti- NGF antibody according to any of the foregoing aspects of the disclosure and instructions for use of the same.

A yet further aspect of the present disclosure provides a diagnostic kit for the detection of an anti-caninie NGF monoclonal antibody in fluids in vitro, ex vivo and in vivo, for use in determining the concentration of said antibody. The kit may comprise any of the antibodies of the disclosure or a binding fragment thereof. The kit may comprise instructions for use of same.

### Brief Description of the Figures

Figure 1 shows an alignment of framework region sequences FR1 to FR4 of the light chain of the rat αD11 antibody with canine, feline and equine species specific versions (SEQ ID NO:28 to 43).
Figure 2 shows an alignment of framework region sequences FR1 to FR4 of the heavy chain of the rat αD11 antibody with canine, feline and equine species specific versions (SEQ ID NO:44 to 59).
Figure 3 shows amino acid sequences of the heavy (SEQ ID NO:2) and light (SEQ ID NO:1) chain variable domains of a canine version of αD11 anti-NGF MAb and of the complete light (SEQ ID NO:7) and heavy chain (SEQ ID NO:8).
Figure 4 shows amino acid sequences of the heavy (SEQ ID NO:4) and light (SEQ ID NO:3) chain variable domains of a feline version of αD11 anti-NGF MAb and of the complete light (SEQ ID NO:9) and heavy (SEQ ID NO:10) chains.
Figure 5 shows amino acid sequences of the heavy (SEQ ID NO:6) and light (SEQ ID NO:5) chain variable domains of an equine version of αD11 anti-NGF MAb and of the complete light (SEQ ID NO:11) and heavy (SEQ ID NO:12) chains.
Figure 6 shows a gel with the expression of canine, feline and equine speciesised versions of the αD11 MAb.
Figure 7A is a graph showing that expressed and purified canine MAbs to canine NGF are biologically active. Figure 7B is a graph showing that expressed and purified feline MAbs to feline NGF are biologically active, while Figure 7C is a graph showing that expressed and purified equine MAbs to equine NGF are biologically active. Figure 7D is a graph comparing the ability of canine, feline and equine MAbs to neutralise NGF biological activity.
Figure 8A is an amino acid alignment showing a comparison of light chain framework region modifications between a known humanised version of the rat alpha D11 antibody (Pavone et al, WO 06/131951) and a novel humanised variant of αD11 (New Hu - SEQ ID NO:60-63).Figure 8B is a comparison of heavy chain framework region modifications between the known humanised version of the rat alpha D11 antibody (Pavone et al, WO 06/131951) and the novel humanised variant of αD11 (New Hu - SEQ ID NO:64-67).

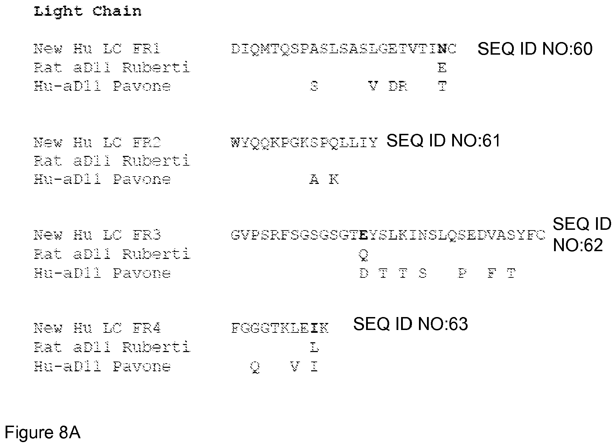

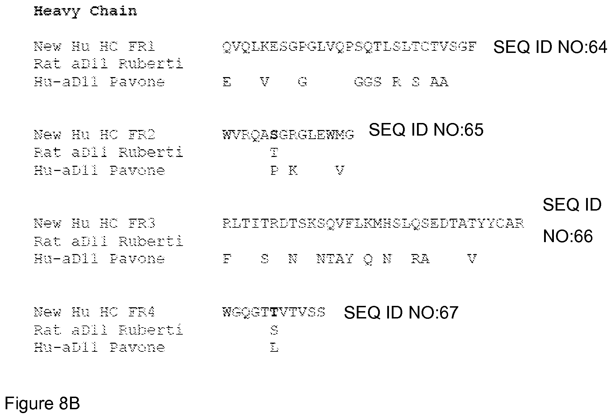

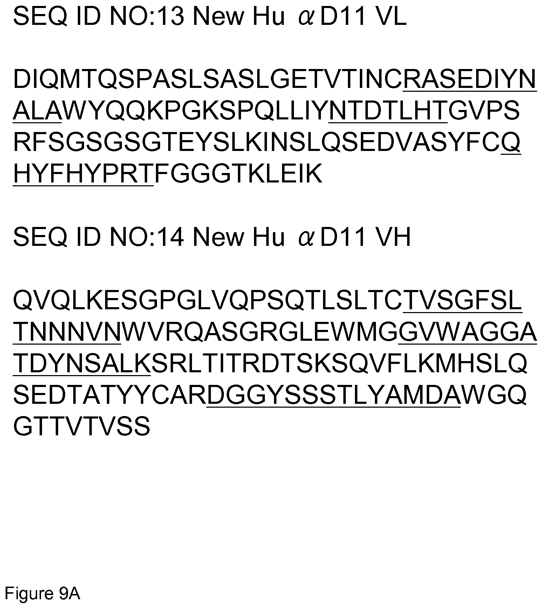

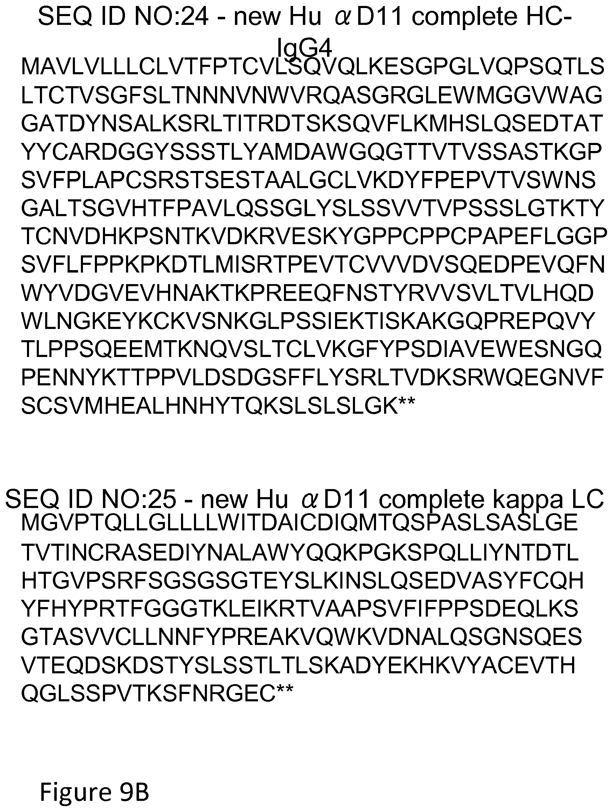

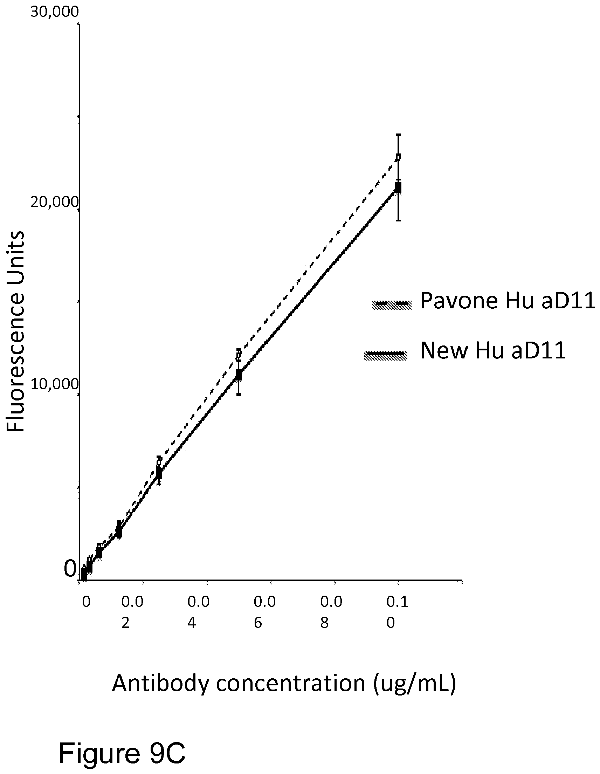

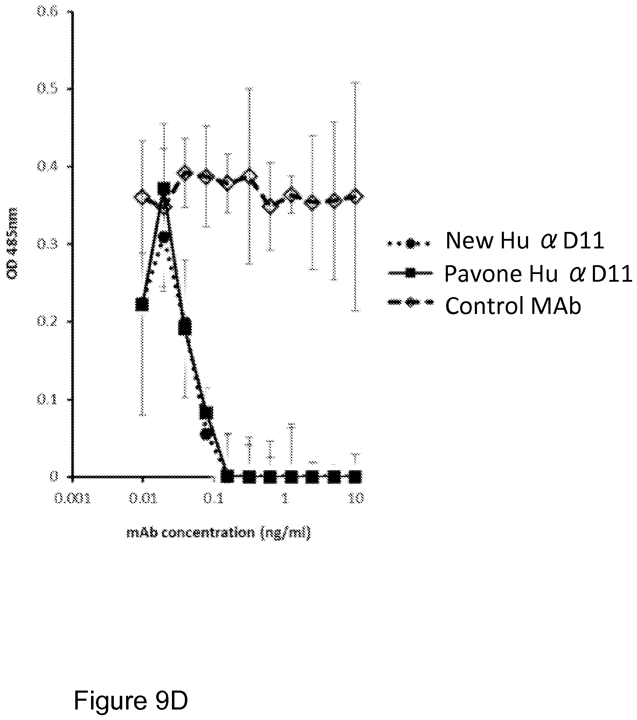

Figure 9A shows the light chain (SEQ ID NO:13) and heavy chain (SEQ ID NO:14) variable domain amino acid sequences of the novel humanised alpha D11 antibody from Figure 8 - CDRs are underlined. Figure 9B shows complete heavy and light chains (SEQ ID NO:24 and 25) designed using the light chain and heavy chain variable domains shown in Figure 9A. Figure 9C shows an ELISA assay comparing an antibody made from the sequences in Figure 9B with an antibody designed by CDR grafting, as previously described by Pavone and colleagues. Figure 9D shows inhibition of NGF proliferation of TF-1 cells by the two humanised variants of alpha D11 monoclonal antibodies used in Figure 9C.
Figure 10 shows light chain (SEQ ID NO:15) and heavy chain (SEQ ID NO:16) variable domain amino acid sequences of a caninised anti-TNF antibody based on the human MAb D2E7 (Salfield et al., US Patent No. 6,090,382).
Figure 11 shows the kappa light chain amino acid sequence (SEQ ID NO:17) of a caninised anti-TNF antibody.
Figure 12 shows the heavy chain amino acid sequences of 4 subtypes (heavy chain type A, B, C and D) of a caninised anti-TNF antibody.
Figure 13 shows the complete light chain (SEQ ID NO:22) and heavy chain (SEQ ID NO:23) amino acid sequences of a chimeric human-canine anti-TNF antibody.
Figure 14A shows the results of co-expressed caninised (Ca) and chimeric (Ch) anti-TNF antibodies purified using Protein A and analysed by SDS-PAGE, while Figure 14B shows the results of an ELISA showing binding of expressed recombinant proteins to canine TNF-alpha. Results with various dilutions of antibodies from 5µg/ml to 0.05 µg/ml are shown.
Figure 15 shows inhibition of canine TNF bioactivity using 293-HEK cells transfected with the NF-kB-EGFP reporter construct pTRH1. These cells respond to canine TNF by fluorescence. Both the caninised (Figure 15A) and chimeric (Figure 15B) MAbs inhibited TNF-induced fluorescence equally well, as quantified in Figure 15C.
Figure 16 shows a comparison to a further caninised MAb based on anti-TNF MAb clone 148 expressed in CHO cells and purified using Protein A chromatography (Panel A, left lane). The MAb was tested for binding to human TNF (Panel B) and canine TNF (Panel C) in comparison to the caninised (Ca) and chimeric (Ch) D2E7 based MAbs from Figure 14 (background negative control binding is shown by the arrows).
Figure 17 shows the heavy chain (SEQ ID NO:26 - ca148-HCB) and light chain (SEQ ID NO:27 - ca148-kLC)of caninised MAb 148.
Figure 18 shows that anti-canine NGF monoclonal antibodies prepared by a method of the present invention reduce inflammatory pain in dogs.

### Detailed description of the Disclosure

The present disclosure extends to novel methods for designing and making a variant of a donor antibody which has reduced immunogenicity when administered to a species which is different to that from which the antibody is derived, while at the same time maintaining (i.e. not decreasing) the binding affinity, avidity or specificity of the antibody for the target ligand. In particular the sequence of the framework regions of the antibody or an antibody binding fragment are assessed and modified to remove residues which are not found at a corresponding position in a pool of antibodies derived from the species to which the antibody is to be administered.

In particular, the methods of the disclosure modify the framework of a donor (parental) antibody so that it is optimally compatible with the intended recipient where the intended recipient is a species other than the donor.

Hitherto in the art, the most commonly used approach to de-immunising an antibody has been to select a compatible framework from germline sequence of a recipient species and to graft the CDR regions into this framework. The problem with this approach is that the selected framework is rarely a perfect match for the CDRs and, as a result, binding affinity to the intended target epitope is reduced. Affinity maturation is then required which results in the introduction of amino acids that are sometimes foreign to the recipient at the positions at which they are introduced. The solution is the procedure of this disclosure where modification of the donor framework is only undertaken where an amino acid is foreign to the recipient at a specific position. The replacement is chosen from the list of options assembled as part of this disclosure. As a result, which is the crux of the disclosure, there is essentially no structural modification of the donor framework and hence essentially no distortion of the conformation of the CDRs, but at the same time there are no "non-self" amino acids within the framework and the resultant immunoglobulin lacks framework epitopes that are foreign in the target species. The methodology can be used to modify an immunoglobulin for administration to any desired target species.

In the most expansive study of immunoglobulin sequence variety to date, Glanville and colleagues (2009) studied the amino acid composition of nearly 100,000 heavy and light chain cDNAs amplified from naive IgM of 654 human donors. 95% of sequences differed from germline by as many as 30 mutations each. CDRs 1 and 2 which mutate only via somatic mutation were demonstrated to be unaltered from germline DNA only 17% of the time and 78% of CDR 1 and 2 sequences had between 1 and 6 amino acid differences. This study did not describe the mutation of non-CDR framework residues, described in the Tables shown herein. Given that somatic hypermutation is via an error prone DNA replication mechanism, the mutation rate per se is unlikely to be different between that observed for CDR 1 or 2 and the rest of the framework sequences and, furthermore, profound secondary selection following somatic hypermutation in the antigen-experienced repertoire during maturation of the immune response would permit further amino acid diversity in framework regions. For these reasons, the diversity observed in the collections of human, canine, equine and feline IgG sequences described herein are likely to be representative of circulating IgG sequences. Surprisingly, there is considerable overlap between the amino acids encoded at homologous "Kabat" framework positions of immunoglobulins of each species post-somatic hypermutation and, consequently, this reduces the changes necessary to convert from one species to another according to the method of the present disclosure.

As an example of this disclosure and following extensive experimentation, the inventor has taken the D2E7 anti-human TNF antibody and the αD11 rat anti-mouse NGF antibody, which were not known to bind to canine TNF alpha or canine NGF, and has surprisingly used these as a basis to produce non-immunogenic antibodies suitable for use in canines. The resulting non-immunogenic antibodies, which are not produced using standard CDR grafting techniques, are shown to exhibit high affinity binding to canine TNF and NGF respectively. Furthermore, the antibodies have been designed so that the framework and constant regions incorporate only residues present in canine IgG molecules so that when administered to a canine, xenoantibodies are unlikely to be produced there against. Accordingly, the caninised antibodies of the disclosure are suitable for long-term administration for the treatment of diseases in canines.Similarly, the felinised, humanised and equinised NGF antibodies of the disclosure are suitable for long-term administration for the treatment of diseases in felines, humans and equines respectively.

The process of generating the heavy and light chain variable domains for the antibodies of the disclosure which has been employed by the inventor results in the replacement of specific donor amino acid residues known to be foreign to the target (e.g. canines) at that position with a target (e.g. canine) residue which, based on the inventor's analysis, will retain the conformation of the CDR regions and therefore maintain binding specificity and avidity, while reducing the presence of immunogenic epitopes which may result in neutralising antibodies being generated against the antibody if it were to be administered to the target (e.g. canines) in an unaltered form. Specifically, the method of preparing antibodies of the disclosure

(known as PETisation) comprises assessing the sequence of the framework regions of a donor (e.g. human) antibody for suitability for administering to a target (e.g. canine) by comparing the sequence of the framework regions of the donor antibody with the sequence of an antibody or a pool of antibodies derived from the target (e.g. canine). Although the comparison may be between the donor sequence and a single member of the target sequence, it will be obvious that comparison with a pool of target sequences is preferred because this will expand the number of natural options at each Kabat position in the target species. Not only will this increase the chance of a "match" between the donor and the target, but it will also expand the options for replacement where a match does not exist. As a result, a replacement with characteristics as close as possible to the donor will be able to be chosen. Where the donor sequence and the canine sequence differ at any Kabat number or corresponding position, the donor sequence is modified to substitute the amino acid residue in question with an amino acid residue which is known to be natural at that position in the target (e.g. canines).

Where substitution of an amino acid residue present in a donor immunoglobulin framework region is required, typically this is undertaken using the principle of conservative substitution wherein an amino acid residue is replaced with an amino acid residue which is natural at that Kabat position in the target (e.g. a canine) and is as closely related as possible in size, charge and hydrophobicity to the amino acid being substituted in the donor sequence. The intention is to choose a replacement which would cause no, or at least only minimum, perturbation or disruption to the three-dimensional structure of the donor antibody. In certain situations, there will be no clear option and each choice will have benefits and downsides. A final decision may require three-dimensional modelling or even expression of various alternative sequences. However, generally, a clear preference will be available. As a result of this procedure, a change in the donor sequence is only made when that residue would be foreign in the target and the replacement amino acid is as closely related as possible to that which it replaces. Thus, the creation of foreign epitopes is avoided, but the overall three-dimensional structure is preserved and as a result, affinity and specificity are also preserved. Exemplary examples of this aspect of the disclosure include:
- light chain sequences SEQ ID NO: 1, 3, 5, 7, 9, 11, 13, 15, 17, 25, 27 and 71.
- heavy chain sequences SEQ ID NO: 2, 4, 6, 8, 10, 12, 14, 16, 18, 19, 20, 21, 24, 26, 68, 69 and 70.

### Antibody production

The antibodies and binding members of the disclosure may be produced wholly or partly by chemical synthesis. For example, the antibodies and binding members of the disclosure can be prepared by techniques which are well known to the person skilled in the art, such as standard liquid peptide synthesis, or by solid-phase peptide synthesis methods. Alternatively, the antibodies and binding members may be prepared in solution using liquid phase peptide synthesis techniques, or further by a combination of solid-phase, liquid phase and solution chemistry.

The present disclosure further extends to the production of the antibodies or binding members of the disclosure by expression of a nucleic acid which encodes at least one amino acid which comprises an antibody of the disclosure in a suitable expression system, such that a desired peptide or polypeptide can be encoded. For example, a first nucleic acid encoding the amino acid light chain and a second nucleic acid encoding an amino acid heavy chain can be expressed to provide an antibody of the present disclosure.

Accordingly, in certain further aspects of the disclosure, there is provided nucleic acids encoding amino acid sequences which form the antibodies or binding members of the present disclosure.

Typically, nucleic acids encoding the amino acid sequences which form antibodies or binding members of the present disclosure can be provided in an isolated or purified form, or provided in a form which is substantially free of material which can be naturally associated with it, with the exception of one or more regulatory sequences. Nucleic acids which expresses an antibody or binding member of the disclosure may be wholly or partially synthetic and may include, but are not limited to, DNA, cDNA and RNA.

Nucleic acid sequences encoding the antibodies or binding members of the disclosure can be readily prepared by the skilled person using techniques which are well known to those skilled in the art, such as those described in Sambrook et al. "Molecular Cloning", A laboratory manual, Cold Spring Harbor Laboratory Press, Volumes 1-3, 2001 (ISBN-0879695773), and Ausubel et al. Short Protocols in Molecular Biology. John Wiley and Sons, 4th Edition, 1999 (ISBN - 0471250929). Said techniques include (i) the use of the polymerase chain reaction (PCR) to amplify samples of nucleic acid, (ii) chemical synthesis, or (iii) preparation of cDNA sequences. DNA encoding antibodies or binding members of the disclosure may be generated and used in any suitable way known to those skilled in the art, including taking encoding DNA, identifying suitable restriction enzyme recognition sites either side of the portion to be expressed, and cutting out said portion from the DNA. The excised portion may then be operably linked to a suitable promoter and expressed in a suitable expression system, such as a commercially available expression system. Alternatively, the relevant portions of DNA can be amplified by using suitable PCR primers. Modifications to the DNA sequences can be made by using site directed mutagenesis.

Nucleic acid sequences encoding the antibodies or binding members of the disclosure may be provided as constructs in the form of a plasmid, vector, transcription or expression cassette which comprises at least one nucleic acid as described above. The construct may be comprised within a recombinant host cell which comprises one or more constructs as above. Expression may conveniently be achieved by culturing, under appropriate conditions, recombinant host cells containing suitable nucleic acid sequences. Following expression, the antibody or antibody fragments may be isolated and/or purified using any suitable technique, then used as appropriate.

Systems for cloning and expression of a polypeptide in a variety of different host cells are well known. Suitable host cells include bacteria, mammalian cells, yeast, insect and baculovirus systems. Mammalian cell lines available in the art for expression of a heterologous polypeptide include Chinese hamster ovary (CHO) cells, HeLa cells, baby hamster kidney cells and NS0 mouse myeloma cells. A common, preferred bacterial host is E. coli. The expression of antibodies and antibody fragments in prokaryotic cells such as E. coli is well established in the art. Expression in eukaryotic cells in culture is also available to those skilled in the art as an option for production of a binding member.

General techniques for the production of antibodies are well known to the person skilled in the field, with such methods being discussed in, for example, Kohler and Milstein (1975) Nature 256: 495-497; US Patent No. 4,376,110; Harlow and Lane, Antibodies: a Laboratory Manual, (1988) Cold Spring Harbor. Techniques for the preparation of recombinant antibody molecules are described in the above references and also in, for example, European Patent Number 0,368,684.

In certain instances of the disclosure, recombinant nucleic acids comprising an insert coding for a heavy chain variable domain and/or for a light chain variable domain of antibodies or binding members are employed. By definition, such nucleic acids comprise single stranded nucleic acids, double stranded nucleic acids consisting of said coding nucleic acids and of complementary nucleic acids thereto, or these complementary (single stranded) nucleic acids themselves.

Furthermore, nucleic acids encoding a heavy chain variable domain and/or a light chain variable domain of antibodies can be enzymatically or chemically synthesised nucleic acids having the authentic sequence coding for a naturally-occurring heavy chain variable domain and/or for the light chain variable domain, or a mutant thereof.

An antibody of the disclosure may be produced by recombinant means, not only directly, but also as a fusion polypeptide with a heterologous polypeptide, which is preferably a signal sequence or other polypeptide having a specific cleavage site at the N-terminus of the mature protein or polypeptide. The selected heterologous signal sequence preferably is one that is recognized and processed (i.e., cleaved by a signal peptidase) by the host cell. For prokaryotic host cells that do not recognize and process a native antibody signal sequence, the signal sequence is substituted by a prokaryotic signal sequence selected, for example, from the group of the alkaline phosphatase, penicillinase, lpp and heat-stable enterotoxin II leaders.

The term "isolated", when used in reference to the antibodies of the disclosure, or to binding members derived therefrom, or polypeptides which encode the same, refers to the state in which said antibodies, binding members or nucleic acids (polynucleotides) are provided in an isolated and/or purified form, that is they have been separated, isolated or purified from their natural environment, and are provided in a substantially pure or homogeneous form, or, in the case of nucleic acid, free or substantially free of nucleic acid or genes of origin other than the sequence encoding a polypeptide with the required function. Accordingly, such isolated antibodies, binding members and isolated nucleic acids will be free or substantially free of material with which they are naturally associated, such as other polypeptides or nucleic acids with which they are found in their natural environment, or the environment in which they are prepared (e.g. cell culture) when such preparation is by recombinant DNA technology practised in vitro or in vivo.

Antibodies, binding members and nucleic acids may be formulated with diluents or adjuvants and still, for practical purposes, be considered as being provided in an isolated form. For example the antibodies and binding members can be mixed with gelatin or other carriers if used to coat microtitre plates for use in immunoassays, or will be mixed with pharmaceutically acceptable carriers or diluents when used in diagnosis or therapy. The antibodies or binding members may be glycosylated, either naturally or by systems of heterologous eukaryotic cells (e.g. CHO or NSO cells), or they may be (for example, if produced by expression in a prokaryotic cell) unglycosylated.

Heterogeneous preparations comprising antibodies of the disclosure also form part of the disclosure. For example, such preparations may be mixtures of antibodies with full-length heavy chains and heavy chains lacking the C-terminal lysine, with various degrees of glycosylation and/or with derivatized amino acids, such as cyclization of an N-terminal glutamic acid to form a pyroglutamic acid residue.

### Definitions

Unless otherwise defined, all technical and scientific terms used herein have the meaning commonly understood by a person who is skilled in the art in the field of the present disclosure. The meaning and scope of the terms should be clear, however, in the event of any ambiguity, definitions provided herein take precedent over any dictionary or extrinsic definition.

Throughout the specification, unless the context demands otherwise, the terms "comprise" or "include", or variations such as "comprises" or "comprising", "includes" or "including" will be understood to imply the inclusion of a stated integer or group of integers, but not the exclusion of any other integer or group of integers.

As used herein, terms such as "a", "an" and "the" include singular and plural referents unless the context clearly demands otherwise. Thus, for example, reference to "an active agent" or "a pharmacologically active agent" includes a single active agent as well as two or more different active agents in combination, while references to "a carrier" includes mixtures of two or more carriers as well as a single carrier, and the like. Further, unless otherwise required by context, singular terms shall include pluralities and plural terms shall include the singular.

The term "corresponding amino acid" means an amino acid residue which is found at an identical position (i.e., they lie across from each other) when two or more amino acid sequences are aligned to allow for maximum sequence identity between the sequences. Amino acid residues at corresponding positions have the same Kabat numbering. In particular, amino acid sequences of framework regions of different antibodies may be aligned or the amino acid sequence of a framework region sequence of one antibody may be compared to a pool of positional specific framework region amino acid residues derived from a plurality of immunoglobulins of a particular species. Methods for aligning and numbering antibody sequences are well known to the person skilled in the art and are disclosed in Kabat et al. (Kabat,E.A., Wu,T.T., Perry,H., Gottesman,K. and Foeller,C. (1991) Sequences of Proteins of Immunological Interest, Fifth Edition. NIH Publication No. 91-3242).

The term "complementarity determining region (CDR)", as used herein, refers to amino acid sequences which together define the binding affinity and specificity of the natural Fv region of a native immunoglobulin binding site as delineated by Kabat et al. (Kabat,E.A., Wu,T.T., Perry,H., Gottesman,K. and Foeller,C. (1991) Sequences of Proteins of Immunological Interest, Fifth Edition. NIH Publication No. 91-3242). The term "framework region (FR)", as used herein, refers to amino acid sequences interposed between CDRs. These portions of the antibody serve to hold the CDRs in appropriate orientation (allows for CDRs to bind antigen).

The term "constant region (CR)" as used herein, refers to the portion of the antibody molecule which confers effector functions. In the present disclosure, constant regions typically mean constant regions of the target species, that is, that the constant regions of the subject speciesised antibodies are derived from immunoglobulins of the target species. For example, in canine antibodies the heavy chain constant region can be selected from any of four isotypes A, B, C or D.

The term "chimeric antibody" as used herein refers to an antibody containing sequences derived from two different antibodies, which typically are of different species. Most typically, chimeric antibodies comprise variable domains derived from a donor species which bind specifically to a target epitope and constant domains derived from antibodies obtained from the target species to whom the antibody is to be administered.

The term "immunogenicity" as used herein refers to a measure of the ability of a targeting protein or therapeutic moiety to elicit an immune response (humoral or cellular) when administered to a recipient. The present disclosure is concerned with the immunogenicity of the subject speciesised antibodies. Preferably the antibodies of the present disclosure have no immunogenicity, that is, that no neutralising antibodies will be raised against them when administered to a target species.

The term "consists essentially of' or "consisting essentially of" as used herein means that a polypeptide may have additional features or elements beyond those described provided that such additional features or elements do not materially affect the ability of the antibody or antibody fragment to have binding specificity to the desired target. That is, the antibody or antibody fragments comprising the polypeptides may have additional features or elements that do not interfere with the ability of the antibody or antibody fragments to bind to the desired target and antagonise its functional activity. Such modifications may be introduced into the amino acid sequence in order to reduce the immunogenicity of the antibody. For example, a polypeptide consisting essentially of a specified sequence may contain one, two, three, four, five or more additional, deleted or substituted amino acids at either end or at both ends of the sequence provided that these amino acids do not interfere with, inhibit, block or interrupt the role of the antibody or fragment in binding to the desired target and sequestering its biological function. Similarly, a polypeptide molecule which contributes to the antagonistic antibodies of the disclosure may be chemically modified with one or more functional groups provided that such functional groups do not interfere with the ability of the antibody or antibody fragment to bind to the desired target and antagonise its function.

The present disclosure will now be described with reference to the following examples which are provided for the purpose of illustration and are not intended to be construed as being limiting on the present disclosure.

### EXAMPLES

### Example 1 - Conversion of murine antibodies to canine, feline, equine and human antibodies

Immunoglobulin gamma (IgG) variable domain heavy (VH) and light (VL) chain protein sequences of human, feline, canine and equine origin, derived from publicly available expressed cDNA sequence databases and publications, were aligned in groups according to species using the program ClustalW using the BLOSUM Cost Matrix and Gap open cost of 10 and Gap extend cost of 0.1. Poor quality sequences of low homology were deleted from the alignment to avoid spurious gaps in framework regions. Framework and CDR regions were identified according to the Kabat nomenclature and the amino acid residues at each Kabat framework region position were identified and tabulated according to light and heavy chain (Tables 1-8). Although the light chain table is constructed from a collection of kappa light chains, similar tables can be constructed from lambda light chains for use in converting lambda light chains from one species to another according to the methods described in this patent.

The conservation of sequence between the four species' IgG sequences at light chain framework positions Q6, C23, W35, P44, Y83, C85 and G98, and heavy chain framework positions G8, C22, W36, R38, D86, Y90, C92 and G106 suggest that there is minimal contamination of the pooled amino acid data set by simple nucleotide sequence errors in the starting data set. The composition of the pool of residues present at each framework region in Tables 1-8 is determined by the available data for each species. The determination of additional amino acid sequences for immunoglobulins derived from any of the analysed species could further diversify the composition of choices at any of the positions in the Tables. This is particularly true of feline derived variable light chains for which only a single example is currently available in the literature and for which the inventor has generated a new set by degenerate oligonucleotide primed polymerase chain reaction amplification of feline spleen tissue mRNA IgG light chain sequences, as shown in Tables 1-8.. Nevertheless, as will be demonstrated below, the current tables can be used along with the methodology disclosed herein to produce antibodies which are suitable for administration to a number of different species.

The method according to the disclosure uses the information provided in Tables 1 to 8 to compare amino acid residues which are present at each position of the framework regions of the light and/or heavy chains of a donor immunoglobulin with a residue present in the pool of immunoglobulins derived from the target species. Should the amino acid residue present at a specific position of the donor framework region not be an amino acid which is present in that position in the pool of immunoglobulins derived from the target species, then the residue is substituted with an amino acid present in the pool as relevant to that position. When determining which amino acid should replace the substituted residue in the donor sequence, it is preferable to substitute the donor residue with the residue from the pool which is the nearest homologous residue. That is, the substitution is preferably a conservative substitution. If no homologous residue is available, then the consensus pool amino acid (i.e. the amino acid which is most commonly found at that position) of the target species may preferably be chosen for substitution.

In determining whether a substituted amino acid can be replaced with a conserved amino acid, an assessment may typically made of factors such as, but not limited to, (a) the structure of the polypeptide backbone in the area of the substitution, for example, as a sheet or helical conformation, (b) the charge or hydrophobicity of the molecule at the target site, and/or (c) the bulk of the side chain.

When considering whether a donor amino acid not present in the target pool can be conservatively substituted, it may be preferable to assess whether a homologous amino acid is present based on the amino acids being grouped together according to similarities in the properties of their side chains (A. L. Lehninger, in Biochemistry, 2nd Ed., 73-75, Worth Publishers, New York (1975)). For example, the following groups can be determined: (1) non-polar: Ala (A), Val (V), Leu (L), lie (I), Pro (P), Phe (F), Trp (W), Met (M); (2) uncharged polar: Gly (G), Ser (S), Thr (T), Cys (C), Tyr (Y), Asn (N), Gln (Q); (3) acidic: Asp (D), Glu (E); and (4) basic: Lys (K), Arg (R), His(H).

Alternatively, amino acid residues may be divided into groups based on common side-chain properties: (1) hydrophobic: Met, Ala, Val, Leu, Ile; (2) neutral hydrophilic: Cys, Ser, Thr, Asn, Gln; (3) acidic: Asp, Glu; (4) basic: His, Lys, Arg; (5) residues that influence chain orientation: Gly, Pro; and (6) aromatic: Trp, Tyr, Phe.

Conservative substitutions will entail exchanging (substituting) a member of one of these classes for another member of that same class.

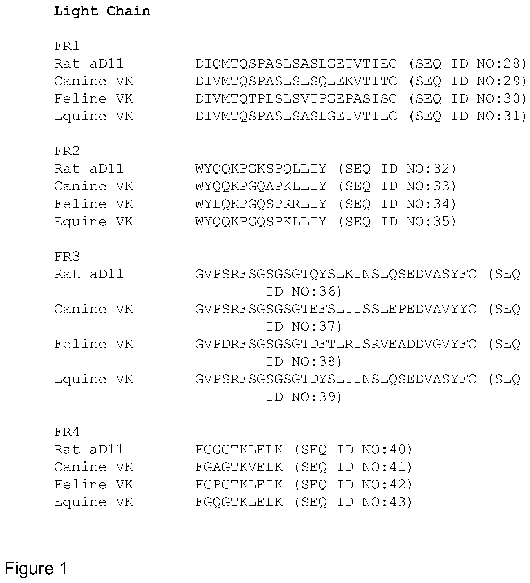

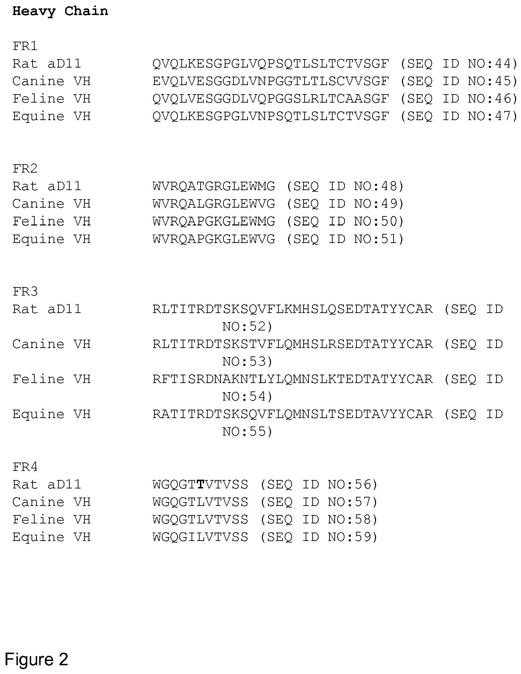

By way of example, donor immunoglobulin light and heavy chain variable domain sequences determined from a rat anti-mouse NGF monoclonal antibody (αD11) were aligned with these tables and PETised canine, equine and feline variants were designed and constructed for expression. The framework region residues selected for each species are shown in Figure 1 and the differences from the donor rat sequence are shown in Figure 2. As can be seen from Figures 1 and 2, the changes necessary to make canine, feline or equine versions of αD11 are different in number and type for each species.

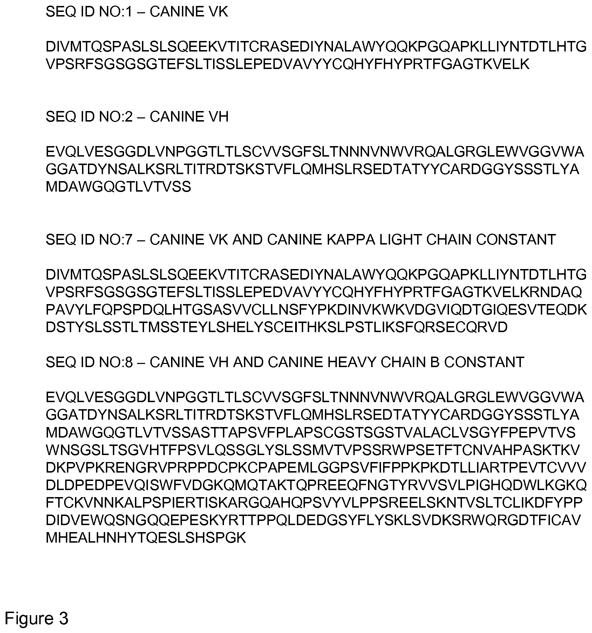

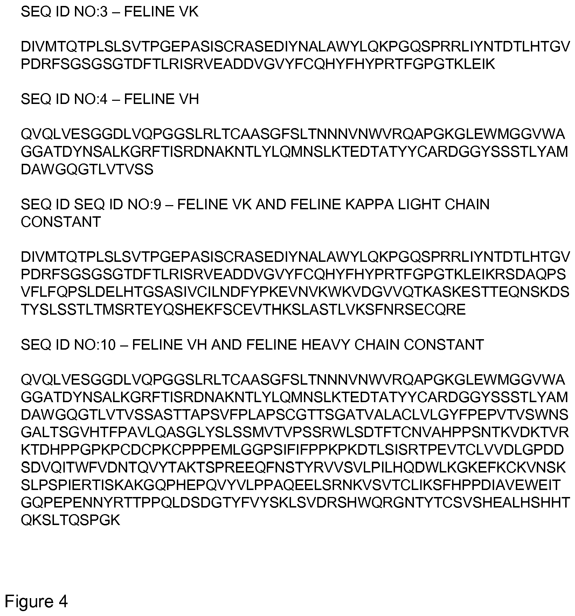

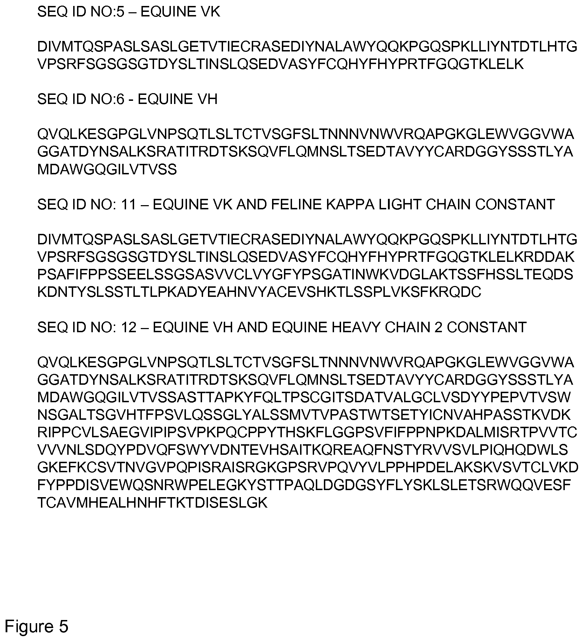

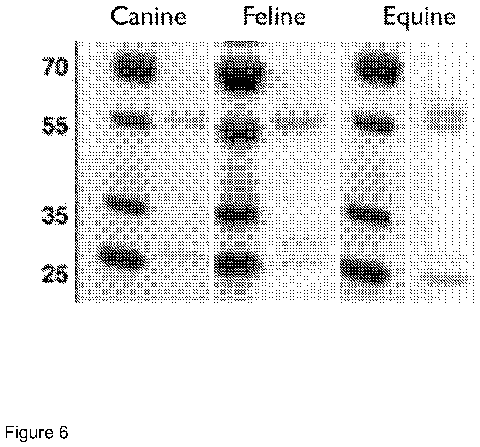

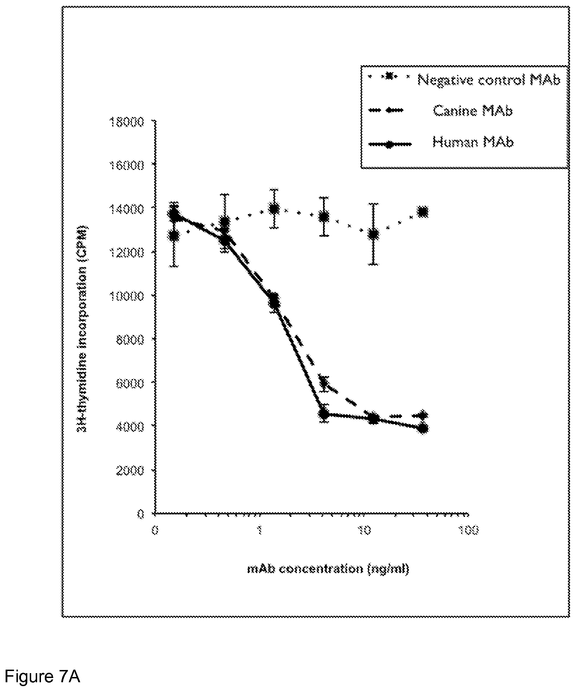

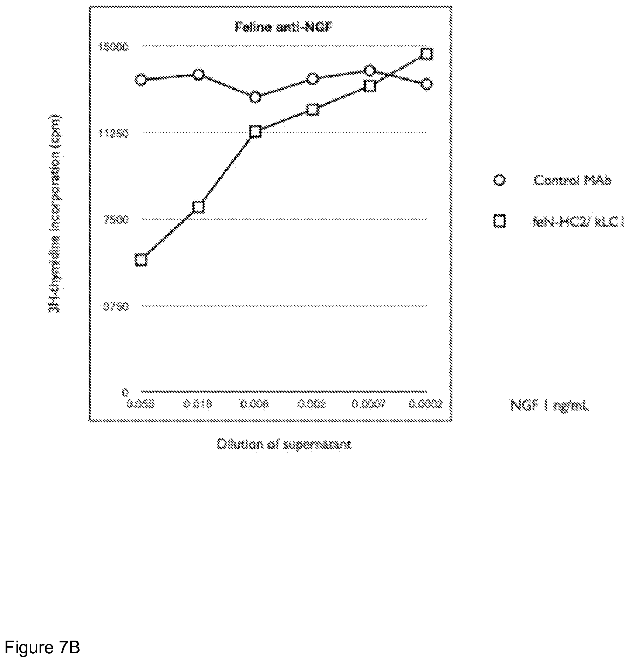

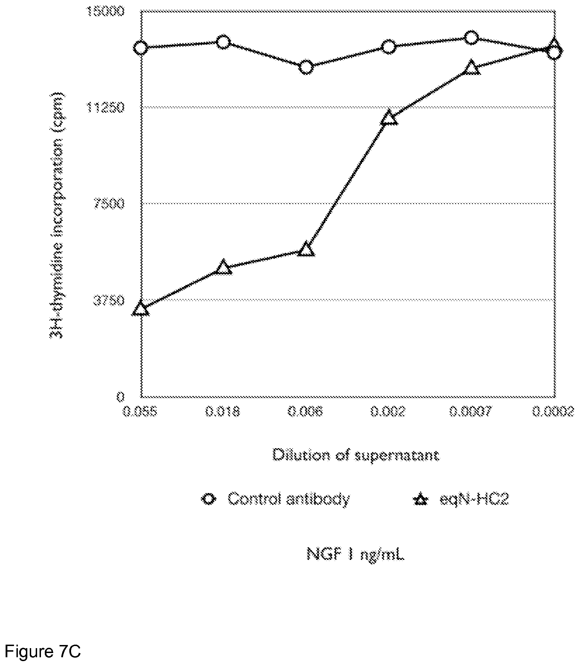

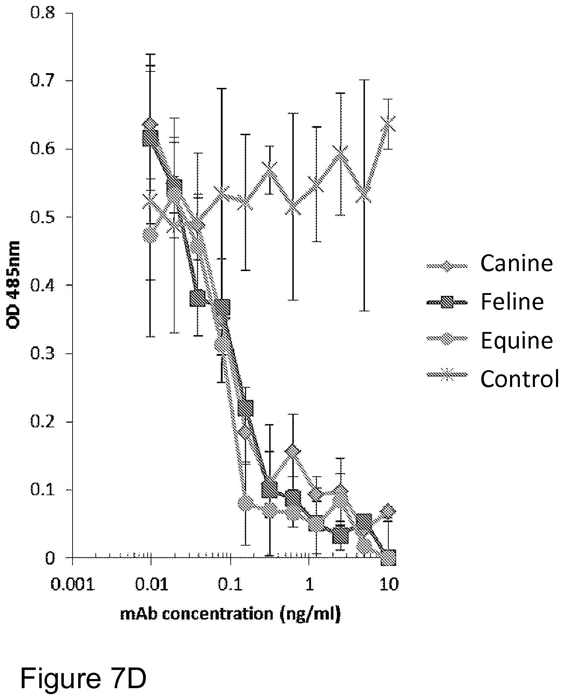

The variable domains for the canine, feline and equine versions of αD11 were expressed as full antibodies by C-terminal fusion to constant domains of each species as shown in Figure 3, 4 and 5 (variable domains SEQ ID NO 1-6, full antibodies SEQ ID NO 7-12) and co-expression of appropriate heavy and light chain pairs in expression vectors transfected into CHO cells. The supernatants containing the expressed heavy and light chain pairs as whole IgG were analyzed by SDS-PAGE and tested for their ability to inhibit proliferation of TF-1 cells by nerve growth factor in culture. Heavy and light chains were observed in Coomassie Blue stained SDS-PAGE gels (Figure 6) and the supernatants were able to inhibit the proliferation of TF-1 cells induced by NGF (Figure 7). By way of comparison, a purified sample of the caninised variant of αD11 was as effective at inhibiting NGF activity as a humanised version (Pavone et al. WO 2006/131951) of αD11 (Figure 5a) illustrating that the PETising technique described herein does not lead to a reduction in the efficacy of the antibody structure per se, by comparison with the standard method of CDR grafting used to humanize the antibody by Pavone et al. The similar bioactivity of canine, feline and equine versions of αD11 is further illustrated in Figure 7D.

**Table 1 - Light chain variable domain FR1 residues**

| **Light chain FR1 position** | **Kabat light chain numbering position** | **Canine VK** | **Feline VK** | **Equine VK** | **Human VK** |
|---|---|---|---|---|---|
| **1** | **1** | DEA | DEN | DEGKV | DEAKY |
| **2** | **2** | ILV | VIPT | VIFNST | IVK |
| **3** | **3** | V | VEM | VMAGI | VQREAL |
| **4** | **4** | ML | MLI | MLVQ | MLC |
| **5** | **5** | MTI | T | TAI | TI |
| **6** | **6** | QE | Q | Q | Q |
| **7** | **7** | TSA | TS | STF | ST |
| **8** | **8** | P | P | P | PA |
| **9** | **9** | LAPRS | L | EDAPS | SGLADFPT |
| **10** | **10** | SP | SF | STFL | STFAL |
| **11** | **11** | L | L | LSV | LVSP |
| **12** | **12** | SA | SPA | ATESV | SAPH |
| **13** | **13** | VLA | V | VALQT | AVLR |
| **14** | **14** | STR | TIA | SAPT | ST |
| **15** | **15** | PQR | P | LPIR | VPLQR |
| **16** | **16** | EDGR | G | GR | GL |
| **17** | **17** | E | ED | QE | DEQGN |
| **18** | **18** | PTLKEAS | PSA | RSGKT | RPGTAS |
| **19** | **19** | AV | AV | VA | VASI |
| **20** | **20** | STF | S | ETDV | T |
| **21** | **21** | I | IF | MIVLT | IL |
| **22** | **22** | STY | SF | KSRETNQL | TSN |
| **23** | **23** | CY | C | C | C |

**Table 2 - Light chain variable domain FR2 residues**

| **Light Chain FR2 Position** | **Kabat light chain numbering system** | **Canine VK** | **Feline VK** | **Equine VK** | **Human VK** |
|---|---|---|---|---|---|
| **1** | **35** | W | W | W | W |
| **2** | **36** | FYILS | YF | YFH | YFL |
| **3** | **37** | RQLIM | LFR | QRS | QHLR |
| **4** | **38** | QH | Q | QHKVCRSY | QHEVGR |
| **5** | **39** | KR | K | KRV | KRISQT |
| **6** | **40** | PSA | P | PSAIL | PALSQ |
| **7** | **41** | GD | G | G | GE |
| **8** | **42** | QH | QR | QE | KQNRHT |
| **9** | **43** | SATP | S | ARSTVP | APSTGV |
| **10** | **44** | P | P | PL | P |
| **11** | **45** | QKER | R | KERIL | KRNQES |
| **12** | **46** | RLPGASDTI | RL | RQLAHGWEK | LFRSVM |
| **13** | **47** | LR | L | LVIFM | LV |
| **14** | **48** | ILT | IM | IFMVT | ILVMF |
| **15** | **49** | YFNSEVH | YHA | YSVFDEQRTWACGHL | YFSH |

**Table 3 - Light chain variable domain FR3 residues**

| **Light chain FR3 position** | **Kabat light chain numbering position** | **Canine VK** | **Feline VK** | **Equine VK** | **Human VK** |
|---|---|---|---|---|---|
| **1** | **57** | GA | GR | GDF | G |
| **2** | **58** | VA | V | VFA | VI |
| **3** | **59** | PS | P | PLS | PST |
| **4** | **60** | DSE | D | DAESGL | SDALEPW |
| **5** | **61** | RG | R | R | RM |
| **6** | **62** | FLVI | FI | FLY | FI |
| **7** | **63** | SIA | ST | SCFGNRT | SGTV |
| **8** | **64** | GA | G | GA | GAP |
| **9** | **65** | SR | S | SGRDEKTW | SGI |
| **10** | **66** | G | G | GRAV | GERV |
| **11** | **67** | S | S | SFYTA | SAP |
| **12** | **68** | GV | G | GET | GAS |
| **13** | **69** | TA | TAS | TASW | TAP |
| **14** | **70** | DE | D | DE | DEVS |
| **15** | **71** | FC | F | FY | FYH |
| **16** | **72** | TSR | TIA | TSAVY | TSIAN |
| **17** | **73** | LF | L | LFIP | LFHS |
| **18** | **74** | RTKE | RTK | TISAV | TKIAE |
| **19** | **75** | I | I | IV | IMSV |
| **20** | **76** | STG | SAGT | SNDTG | SNTAD |
| **21** | **77** | RGST | RG | SPTEDNR | SRGDNIPT |
| **22** | **78** | VLA | VM | LVFP | LVM |
| **23** | **79** | EVG | EQ | QER | QEKHLR |
| **24** | **80** | APD | AVPT | AEST | PAS |
| **25** | **81** | DEGINA | DE | ETAGD | ED |
| **26** | **82** | DG | D | DN | DN |
| **27** | **82A** | AVTGS | VIHL | VAEGLS | FVSI |
| **28** | **82B** | GA | G | AG | AG |
| **29** | **82C** | VIL | V | IVTDSMNLEFGY | TVSI |
| **30** | **83** | Y | Y | YC | Y |
| **31** | **84** | YHFC | YF | YFHSTVW | YF |
| **32** | **85** | C | C | C | C |

**Table 4 - Light chain variable domain FR4 residues**

| **Light chain FR4 position** | **Kabat light chain FR4 position** | **Canine VK** | **Feline VK** | **Equine VK** | **Human VK** |
|---|---|---|---|---|---|
| **1** | **95** | FLS | FS | FIL | FL |
| **2** | **96** | GS | G | G | GA |
| **3** | **97** | AQPTK | QP | QAL | QGP |
| **4** | **98** | GE | G | G | G |
| **5** | **99** | TPA | T | TS | TA |
| **6** | **100** | KQSNH | KHQEST | KNRM | KRT |
| **7** | **101** | VLW | L | LVM | VLI |
| **8** | **102** | DERVG | ED | EADK | EDP |
| **9** | **103** | LIM | IVML | ILVFM | ITVFM |
| **10** | **104** | KR | KRDT | KERTAGIQV | KRTEN |

**Table 5 - Heavy chain variable domain FR1 residues**

| **Heavy chain FR1 position** | **Kabat heavy chain numbering position** | **Canine VH** | **Feline VH** | **Equine VH** | **Human VH** |
|---|---|---|---|---|---|
| **1** | **1** | EDG | QDEH | Q | QEHL |
| **2** | **2** | VGLEIM | VE | V | VLGIM |
| **3** | **3** | QHRAVEKLPS | LQR | Q | QRHIKY |
| **4** | **4** | LVP | LV | L | LV |
| **5** | **5** | VALEM | VM | KQ | VQLEK |
| **6** | **6** | EQA | QED | E | EQDV |
| **7** | **7** | SFLT | S | S | SWP |
| **8** | **8** | GA | G | G | GAE |
| **9** | **9** | GEA | GAR | P | GAPT |
| **10** | **10** | DAGNETW | EDN | GD | GEDARV |
| **11** | **11** | LQRVW | LVR | LQ | LVFPSW |
| **12** | **12** | VAIMK | VRSK | VM | VKLIMRAGT |
| **13** | **13** | KRNQ | KTQENR | KMNR | KQERNT |
| **14** | **14** | PFT | PT | PIS | PALR |
| **15** | **15** | GAETS | GE | SAG | GSER |
| **16** | **16** | GEA | GATE | QE | GESRAQTVD |
| **17** | **17** | STP | SA | TA | STA |
| **18** | **18** | LRV | LV | L | LVRE |
| **19** | **19** | RKTGV | RKES | ST | RKSTHI |
| **20** | **20** | LIV | ILP | L | LVIR |
| **21** | **21** | SY | FTS | TVIS | STFN |
| **22** | **22** | C | C | C | C |
| **23** | **23** | VLAIEK | VKAMQ | TASF | AKTEVSIRQDG |
| **24** | **24** | ATVGIS | ATDV | VI | AVGITS |
| **25** | **25** | SPGT | S | ST | SYCF |
| **26** | **26** | GDRT | GA | GA | GVAR |
| **27** | **27** | FLIDSTV | FYL | LFAGIMNQS | GDAVY |

**Table 6 - Heavy chain variable domain FR2 residues**

| **Heavy chain FR2 position** | **Kabat heavy chain numbering system** | **Canine VH** | **Feline VH** | **Equine VH** | **Human VH** |
|---|---|---|---|---|---|
| **1** | **36** | WC | W | W | W |
| **2** | **37** | VIAFL | VLWFIA | VL | VILFA |
| **3** | **38** | RQ | RCH | R | RFP |
| **4** | **39** | QLHRE | Q | Q | QHRL |
| **5** | **40** | ASTGPVDC | APVTS | APSV | APSVMLHDGT |
| **6** | **41** | PL | P | P | PSAR |
| **7** | **42** | GERL | GAES | G | GSEQRW |
| **8** | **43** | KREGAMQ | KQTE | KRW | KQRNT |
| **9** | **44** | GERDTV | G | GR | GRK |
| **10** | **45** | LTPFM | LFP | LPW | LHIP |
| **11** | **46** | QEHDLPRK | QE | E | EQVADK |
| **12** | **47** | WLCSYFM | WCL | FYWEHRSV | W |
| **13** | **48** | VLIFM | VMI | VI | VMIL |
| **14** | **49** | ATSGLV | AGTS | GASD | GSA |

**Table 7 - Heavy chain variable domain FR3 residues**

| **Heavy chain FR3 position** | **Kabat heavy chain numbering position** | **Canine VH** | **Feline VH** | **Equine VH** | **Human VH** |
|---|---|---|---|---|---|
| **1** | **66** | RQ | RQK | R | RQHG |
| **2** | **67** | FVL | FL | AVGCIT | FVILMAT |
| **3** | **68** | TAIS | TIA | SRIDMNT | TISAVH |
| **4** | **69** | IVLMT | ILVM | IV | IMVFALT |
| **5** | **70** | SAFT | ST | TSIL | STLNV |
| **6** | **71** | RKA | RAITKG V | KRES | RAVLTISPEKW |
| **7** | **72** | DEN | D | DEN | DN |
| **8** | **73** | NDTSIGL | TNDAS | TSAEIPY | NTDEKAMIS |
| **9** | **74** | AGVSDP T | ASDTG | SEGKT | SAVYGT |
| **10** | **75** | KRENQG MT | KTRGQ E | KELNQR | KTRIEQANS |
| **11** | **76** | NDSKHR | NDK | SNGKR | NSKTDAG |
| **12** | **77** | TMIAS | TIA | QERH | TQSIHEFR |
| **13** | **78** | LVMAIQ F | LAVG | VILSAF | LAFVMSIT |
| **14** | **79** | YFSHT | YFASV WD | YLSTVFR | YSFHDTVW |
| **15** | **80** | LIM | LM | LV | LM |
| **16** | **81** | QHEDRA | QELRDHV | TIQA | QEKNRHDST |
| **17** | **82** | ML | MLT | LMV | MLWIV |
| **18** | **82A** | NDSTHKPRE | NSDTGRH | NTDKRS | NSTRHD |
| **19** | **82B** | SGDRNT | SNIRT | STADEGKM | SRNDGTAIL |
| **20** | **82C** | LV | L | LVM | LV |
| **21** | **83** | RTGKSI | KRTGQ | TS | RTKQ |
| **22** | **84** | AVDTSGP | STPAIV | SGDER | ASITPVGDLN |
| **23** | **85** | EDAV | EATDGS | EDG | EADSGK |
| **24** | **86** | D | D | D | DN |
| **25** | **87** | TASM | T | TA | TSA |
| **26** | **88** | AGV | AGS | AS | AGS |
| **27** | **89** | VMILFTKQY | TVMIA | VD | VIMLT |
| **28** | **90** | YH | YH | Y | Y |
| **29** | **91** | YFHAGT | YHFC | YFWAI | YFH |
| **30** | **92** | C | CR | C | C |
| **31** | **93** | AVTGMRSCLPK | AITSVG M | ATVGEIS | AVTGSLM |
| **32** | **94** | KRSNGATPDQVEIM | RKSTIVPNG | GRAEHIKS | RKGTSNVAHIY |

**Table 8 - Heavy chain variable domain FR4 residues**

| **Heavy chain** FR4 **position** | **Kabat heavy chain numbering position** | **Canine VH** | **Feline VH** | **Equine VH** | **Human VH** |
|---|---|---|---|---|---|
| **1** | **103** | WL | WRCL | W | WCISY |
| **2** | **104** | GAS | GRA | G | GLS |
| **3** | **105** | QPHRD | QPHVR | QP | QKRHLAEP |
| **4** | **106** | G | GD | G | GPR |
| **5** | **107** | TASIN | AT VIS | I | TISAEP |
| **6** | **108** | LSQPR | LIQMST | L | LTMVPQACN |
| **7** | **109** | VLIP | VI | V | VICFGLW |
| **8** | **110** | TFIASLPY | TAIR | T | TISADLV |
| **9** | **111** | VA | VIG | V | VGILP |
| **10** | **112** | SACPT | STP | S | SFTW |
| **11** | **113** | SLAP | SQPA | - | SPFGT |

### Example 2 - Production of anti-human NGF de-immunised antibody

The PETising technique can also be used to convert antibodies for human use (termed hereinbefore as "re-humanising") having alternative human amino acid heavy and light chain sequences to those of Pavone et al. using the method described above and the pool of human derived framework region position specific amino acid residues shown in Tables 1-8.

A comparison of the re-humanised framework sequences of the light chain (Figure 8A) and heavy chain (Figure 8B) are shown in addition to the resultant VH and VL sequences (Figure 9A). It is apparent that far fewer changes need be made to humanize αD11 MAb using the method disclosed herein (3 amino acid changes to the framework regions) than those used in the standard CDR grafting method by Pavone and colleagues (43 amino acid changes).

The protein sequences of the variable heavy chain (VH) and light chain (VL) variants of re-humanised αD11 antibodies (New-Hu αD11, Figure 9A) were designed with N-terminal signal sequences from rat αD11 and C-terminal human IgG constant domains from IgG4 heavy chain and human kappa light chain respectively (Figure 9B). Synthetic genes encoding these were prepared by oligonucleotide-based gene synthesis and each was subcloned into the mammalian expression vector pcDNA3.1+. Co-transfection into CHO cells and purification from cell supernatants using Protein A chromatography yielded purified antibodies. The antibodies were tested for binding to NGF by ELISA and compared to binding by the humanised variant of αD11 described by Pavone et al. (WO 06/131951, designed by CDR grafting). As can be seen from the ELISA results shown in Fig 9C, the New Hu αD11 variant of the present patent has binding to NGF that is indistinguishable to that described by Pavone et al. The antibodies described in Figure 9C were tested for inhibition of NGF by the method described in Figure 7C. Both humanised antibodies showed equivalent bioactivity.

### Example 3 - Production of canine antibodies having binding specificity to canine TNF

By way of further example, the PETising method of this patent was used with a human antibody D2E7 that binds tumour necrosis factor as the starting point in order to make a canine variant thereof.

Tables 9-16, shown below, illustrate the alignment of the framework regions of the light and heavy chain variable domains of the human monoclonal antibody D2E7 with the pool of the amino acid residues defined for each position of the canine immunoglobulin framework sequences which (as shown in Tables 1-8). Certain residues (marked * in Table 9) previously thought to be foreign at that Kabat sequence position are now considered natural to canines, hence the changes shown at the positions marked * would now no longer be made, or an alternative, more conserved, residue may be chosen (marked ** in Table 10). Based on this additional information, three light chain positions would be unchanged, including Ser9, Ala13 and Gly16 (marked * in Table 9). Light chain His42 would be an alternative choice of residue at that position (marked ** in Table 10). Modifications of the caninised D2E7 framework residues at these positions, and antibodies comprising such modifications, are intended to be within the scope of this disclosure. Such antibodies would comprise a light chain variable domain having the sequence shown in SEQ ID NO:15 with the exception that a serine residue is provided at position 9, an alanine residue is provided at position 13 and a glycine residue is provided at position 16 in place of the residues shown at these positions in SEQ ID NO:15. Optionally a histidine residue may be provided at position 42 of SEQ ID NO:15 in place of glutamine. A modified sequence showing these changes is provided as SEQ ID NO:71. The disclosure extends to antibodies comprising a light chain variable domain comprising SEQ ID NO:71, and antigen binding fragments derived from same.

**Table 9 - Caninised light chain FR1 sequence of human derived D2E7 monoclonal antibody**

| Light chain FR1 position | Kabat light chain numbering position | Human MAb D2E7 VL | Canine VK amino acid pool sequences | **Canine PETised D2E7 VL** |
|---|---|---|---|---|
| 1 | 1 | D | DEA | **D** |
| 2 | 2 | I | ILV | **I** |
| 3 | 3 | Q | V | **V** |
| 4 | 4 | M | ML | **M** |
| 5 | 5 | T | MTI | **T** |
| 6 | 6 | Q | QE | **Q** |
| 7 | 7 | S | TSA | **S** |
| 8 | 8 | P | P | **P** |
| 9 | 9 | S | LAPRS | **A*** |
| 10 | 10 | S | SP | **S** |
| 11 | 11 | L | L | **L** |
| 12 | 12 | S | SA | **S** |
| 13 | 13 | A | VLA | **L*** |
| 14 | 14 | S | STR | **S** |
| 15 | 15 | V | PQR | **Q** |
| 16 | 16 | G | EDGR | **E*** |
| 17 | 17 | D | E | **E** |
| 18 | 18 | R | PTLKEAS | **K** |
| 19 | 19 | V | AV | **V** |
| 20 | 20 | T | STF | **T** |
| 21 | 21 | I | I | **I** |
| 22 | 22 | T | STY | **T** |
| 23 | 23 | C | CY | **C** |

**Table 10 - Caninised light chain FR2 sequence of human derived D2E7 monoclonal antibody**

| Light Chain FR2 Position | Kabat light chain numbering system | Human MAb D2E7 VL | Canine VK amino acid pool sequences | **Canine PETised D2E7 VL** |
|---|---|---|---|---|
| 1 | 35 | W | W | **W** |
| 2 | 36 | Y | FYILS | **Y** |
| 3 | 37 | Q | RQLIM | **Q** |
| 4 | 38 | Q | QH | **Q** |
| 5 | 39 | K | KR | **K** |
| 6 | 40 | P | PSA | **P** |
| 7 | 41 | G | GD | **G** |
| 8 | 42 | K | QH** | **Q** |
| 9 | 43 | A | SATP | **A** |
| 10 | 44 | P | P | **P** |
| 11 | 45 | K | QKER | **K** |
| 12 | 46 | L | RLPGASDTI | **L** |
| 13 | 47 | L | LR | **L** |
| 14 | 48 | I | ILT | **I** |
| 15 | 49 | Y | YFNSEVHW | **Y** |

**Table 11 - Caninised light chain FR3 sequence of human derived D2E7 monoclonal antibody**

| Light chain FR3 position | Kabat light chain numbering position | Human MAb D2E7 VL | Canine VK amino acid pool sequences | **Canine PETised D2E7 VL** |
|---|---|---|---|---|
| 1 | 57 | G | GA | **G** |
| 2 | 58 | V | VA | **V** |
| 3 | 59 | P | PS | **P** |
| 4 | 60 | S | DSE | **S** |
| 5 | 61 | R | RG | **R** |
| 6 | 62 | F | FLVI | **F** |
| 7 | 63 | S | SIA | **S** |
| 8 | 64 | G | GA | **G** |
| 9 | 65 | S | SR | **S** |
| 10 | 66 | G | G | **G** |
| 11 | 67 | S | S | **S** |
| 12 | 68 | G | GV | **G** |
| 13 | 69 | T | TA | **T** |
| 14 | 70 | D | DE | **D** |
| 15 | 71 | F | FC | **F** |
| 16 | 72 | T | TSR | **T** |
| 17 | 73 | L | LF | **L** |
| 18 | 74 | T | RTKE | **T** |
| 19 | 75 | I | I | **I** |
| 20 | 76 | S | STG | **S** |
| 21 | 77 | S | RGST | **S** |
| 22 | 78 | L | VLA | **L** |
| 23 | 79 | Q | EVG | **E** |
| 24 | 80 | P | APD | **P** |
| 25 | 81 | E | DEGINA | **E** |
| 26 | 82 | D | DG | **D** |
| 27 | 82A | V | AVTGS | **V** |
| 28 | 82B | A | GA | **A** |
| 29 | 82C | T | VIL | **V** |
| 30 | 83 | Y | Y | **Y** |
| 31 | 84 | Y | YHFC | **Y** |
| 32 | 85 | C | C | **C** |

**Table 12 - Caninised light chain FR4 sequence of human derived D2E7 monoclonal antibody**

| Light chain FR4 position | Kabat light chain FR4 position | Human MAb D2E7 VL | Canine VK amino acid pool sequences | **Canine PETised D2E7 VL** |
|---|---|---|---|---|
| 1 | 95 | F | FLS | **F** |
| 2 | 96 | G | GS | **G** |
| 3 | 97 | Q | AQPTK | **Q** |
| 4 | 98 | G | GE | **G** |
| 5 | 99 | T | TPA | **T** |
| 6 | 100 | K | KQSNH | **K** |
| 7 | 101 | V | VLW | **V** |
| 8 | 102 | E | DERVG | **E** |
| 9 | 103 | I | L1M | **I** |
| 10 | 104 | K | KR | **K** |

**Table 13 - Caninised heavy chain FR1 sequence of human derived D2E7 monoclonal antibody**

| Heavy chain FR1 position | Kabat heavy chain numbering position | Human MAb D2E7 VH | Canine VH amino acid pool sequences | **Canine PETised D2E7 VH** |
|---|---|---|---|---|
| 1 | 1 | E | EDG | **E** |
| 2 | 2 | V | VGLEIM | **V** |
| 3 | 3 | Q | QHRAVEKLPS | **Q** |
| 4 | 4 | L | LVP | **L** |
| 5 | 5 | V | VALEM | **V** |
| 6 | 6 | E | EQA | **E** |
| 7 | 7 | S | SFLT | **S** |
| 8 | 8 | G | GA | **G** |
| 9 | 9 | G | GEA | **G** |
| 10 | 10 | G | DAGNETW | **G** |
| 11 | 11 | L | LQRVW | **L** |
| 12 | 12 | V | VAIMK | **V** |
| 13 | 13 | Q | KRNQ | **Q** |
| 14 | 14 | P | PFT | **P** |
| 15 | 15 | G | GAETS | **G** |
| 16 | 16 | R | GEA | **G** |
| 17 | 17 | S | STP | **S** |
| 18 | 18 | L | LRV | **L** |
| 19 | 19 | R | RKTGV | **R** |
| 20 | 20 | L | LIV | **L** |
| 21 | 21 | S | SY | **S** |
| 22 | 22 | C | C | **C** |
| 23 | 23 | A | VLAIEK | **A** |
| 24 | 24 | A | ATVGIS | **A** |
| 25 | 25 | S | SPGT | **S** |
| 26 | 26 | G | GDRT | **G** |
| 27 | 27 | F | FLIDSTV | **F** |

**Table 14 - Caninised heavy chain FR2 sequence of human derived D2E7 monoclonal antibody**

| Heavy chain FR2 position | Kabat heavy chain numbering system | Human MAb D2E7 VH | Canine VH amino acid pool sequences | **Canine PETised D2E7 VH** |
|---|---|---|---|---|
| 1 | 36 | W | WC | **W** |
| 2 | 37 | V | VIAFL | **V** |
| 3 | 38 | R | RQ | **R** |
| 4 | 39 | Q | QLHRE | **Q** |
| 5 | 40 | A | ASTGPVDC | **A** |
| 6 | 41 | P | PL | **P** |
| 7 | 42 | G | GERL | **G** |
| 8 | 43 | K | KREGAMQ | **K** |
| 9 | 44 | G | GERDTV | **G** |
| 10 | 45 | L | LTPFM | **L** |
| 11 | 46 | E | QEHDLPRK | **E** |
| 12 | 47 | W | WLCSYFM | **W** |
| 13 | 48 | V | VLIFM | **V** |
| 14 | 49 | S | ATSGLV | **S** |

**Table 15 - Caninised heavy chain FR3 sequence of human derived D2E7 monoclonal antibody**

| Heavy chain FR3 position | Kabat heavy chain numbering position | Human D2E7 MAb VH sequence | Canine VH amino acid pool sequences | **Canine PETised D2E7 VH** |
|---|---|---|---|---|
| 1 | 66 | R | RQ | **R** |
| 2 | 67 | F | FVL | **F** |
| 3 | 68 | T | TAIS | **T** |
| 4 | 69 | I | IVLMT | **I** |
| 5 | 70 | S | SAFT | **S** |
| 6 | 71 | R | RKA | **R** |
| 7 | 72 | D | DEN | **D** |
| 8 | 73 | N | NDTSIGL | **N** |
| 9 | 74 | A | AGVSDPT | **A** |
| 10 | 75 | K | KRENQGMT | **K** |
| 11 | 76 | N | NDSKHR | **N** |
| 12 | 77 | S | TMIAS | **S** |
| 13 | 78 | L | LVMAIQF | **L** |
| 14 | 79 | Y | YFSHT | **Y** |
| 15 | 80 | L | LIM | **L** |
| 16 | 81 | Q | QHEDRA | **Q** |
| 17 | 82 | M | ML | **M** |
| 18 | 82A | N | NDSTHKPRE | **N** |
| 19 | 82B | S | SGDRNT | **S** |
| 20 | 82C | L | LV | **L** |
| 21 | 83 | R | RTGKSI | **R** |
| 22 | 84 | A | AVDTSGP | **A** |
| 23 | 85 | E | EDAV | **E** |
| 24 | 86 | D | D | **D** |
| 25 | 87 | T | TASM | **T** |
| 26 | 88 | A | AGV | **A** |
| 27 | 89 | V | VMILFTKQY | **V** |
| 28 | 90 | Y | YH | **Y** |
| 29 | 91 | Y | YFHAGT | **Y** |
| 30 | 92 | C | C | **C** |
| 31 | 93 | A | AVTGMRSCLPK | **A** |
| 32 | 94 | K | KRSNGATPDQVEIM | **K** |

**Table 16 - Caninised heavy chain FR4 sequence of human derived D2E7 monoclonal antibody**

| Heavy chain FR4 position | Kabat heavy chain numbering position | Human D2E7 MAb VH sequence | Canine VH amino acid pool sequences | **Canine PETised D2E7 VH** |
|---|---|---|---|---|
| 1 | 103 | W | WL | **W** |
| 2 | 104 | G | GAS | **G** |
| 3 | 105 | Q | QPHRD | **Q** |
| 4 | 106 | G | G | **G** |
| 5 | 107 | T | TASIN | **T** |
| 6 | 108 | L | LSQPR | **L** |
| 7 | 109 | V | VLIP | **V** |
| 8 | 110 | T | TFIASLPY | **T** |
| 9 | 111 | V | VA | **V** |
| 10 | 112 | S | SACPT | **S** |
| 11 | 113 | S | SLAP | **S** |

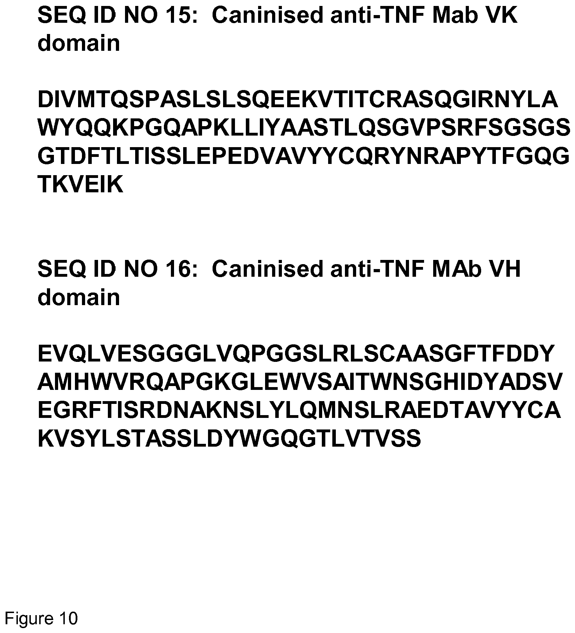

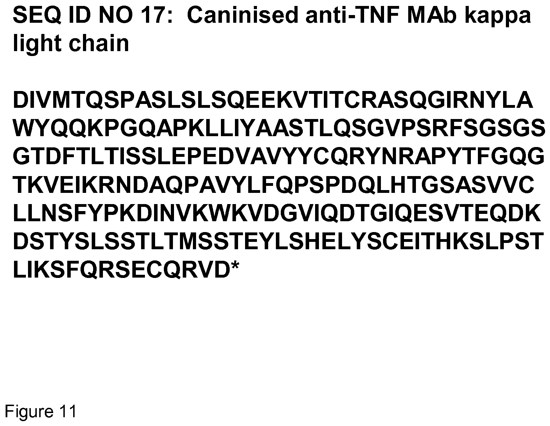

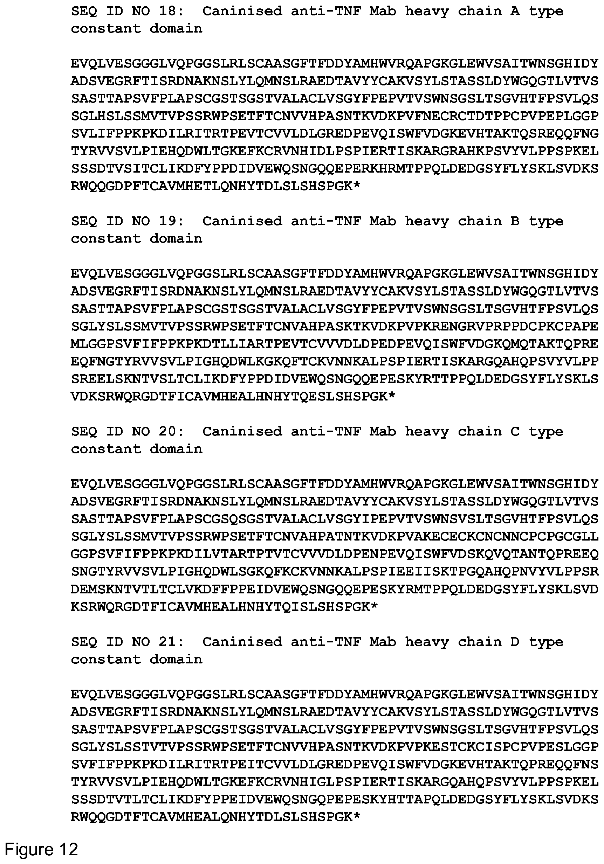

Figures 10, 11 and 12 illustrate variable domain (Figure 10) and whole antibody (Figure 11 (light chain) and Figure 12 (heavy chain)) sequences encoding canine PETised Mab variants of D2E7 in SEQ ID NO:15 to SEQ ID NO:21. The sequences were built as DNAs using oligonucleotide synthesis and subcloned to expression vectors and transfected to CHO cells as above. In particular, the sequences of SEQ ID NO:17 (light chain) and SEQ ID NO:18-21 (heavy chain isotypes A,B,C and D) were designed and built as DNAs using oligonucleotide synthesis and subcloned to pcDNA3.1+ expression vectors and transfected in various combinations into CHO cells.

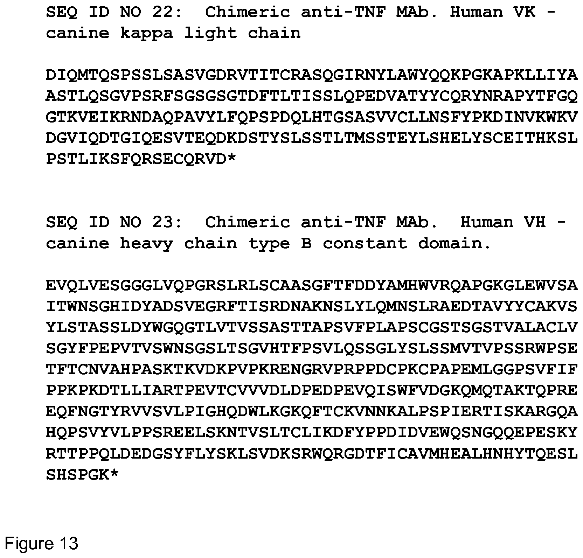

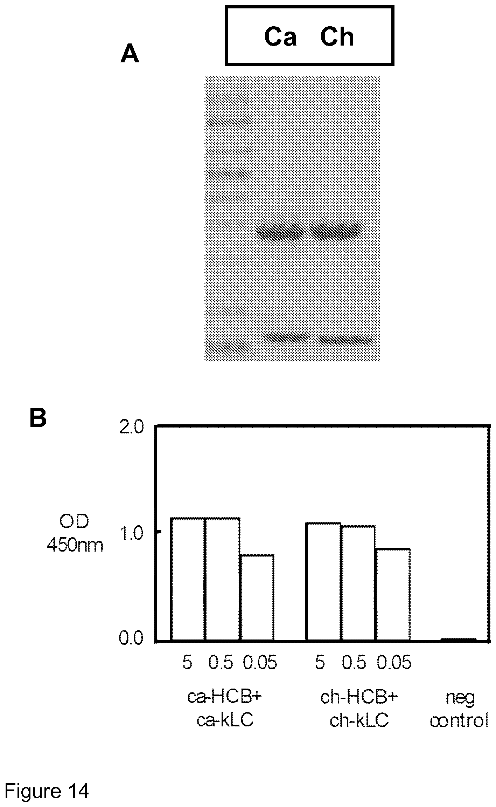

cDNAs encoding caninised anti-TNF monoclonal antibodies having the amino acid sequence of SEQ ID NO:17 (light chain) and SEQ ID NO:19 (heavy chain, isotype B) and a chimeric anti-TNF monoclonal antibody having a light chain with the amino acid of SEQ ID NO:22 and a heavy chain of SEQ ID NO:23 (Figure 13) were subcloned into pcDNA3.1+ (Invitrogen/ Life technologies) with amino-terminal secretory signal sequences (not shown). CHO cells were co-transfected with combinations of either caninised heavy and light chain sequences (ca-HCB + ca-kLC) or chimeric heavy and light chains (ch-HCB + ch-kLC). The resultant supernatants were purified on Protein A, analysed by SDS-PAGE (Figure 14A) and tested for binding to canine TNF alpha (coated at 5ug/ml; R&D systems) at the indicated concentrations of antibody (ug/ml) by ELISA and detected using anti-canine polyclonal antibody-horseradish peroxidase conjugate (Sigma A9042) (Figure 14B). The negative control was the detection polyclonal antibody on coated antigen alone.

Purified antibodies were tested for their ability to inhibit canine TNF activity using 293-HEK cells transfected with pTRH1 to produce a TNF sensitive NF-kB-EGFP reporter cell line that responds to human TNF by fluorescence (Vince et al, Cell 131, 682, 2007). It was first demonstrated that canine TNF activates GFP expression in these cells (50% maximal stimulation at approximately 1 ng/ml) and then the canine antibodies shown in Figure 14 were tested for their ability to inhibit 1 ng/mL canine TNF.

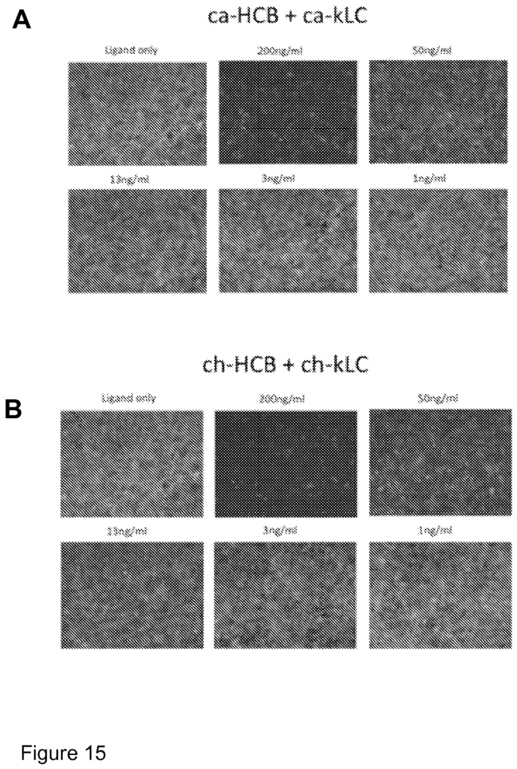

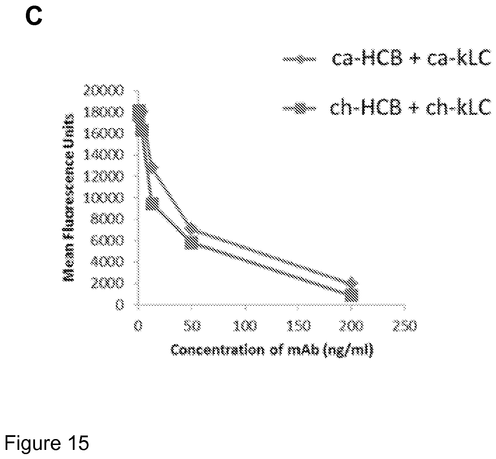

As shown in Figure 15 (A-C), both the caninised and the chimeric antibodies were potent inhibitors of canine TNF in this assay.

Together these results showed that the caninised antibodies of the disclosure and the human-canine chimeric antibodybind canine TNF and are equipotent by both ELISA and inhibition assay, demonstrating that the caninisation process has produced a fully active canine version of the original D2E7 antibody.

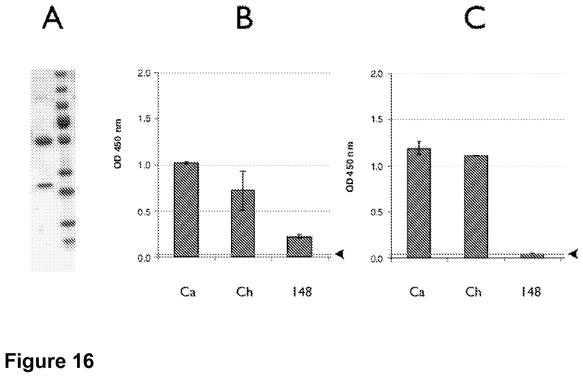

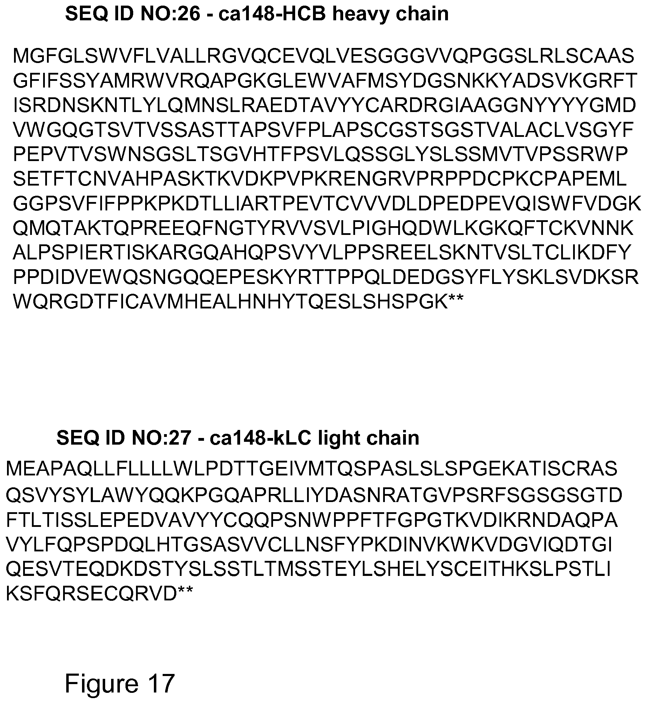

Figure 16 illustrates a comparison of the caninised and chimeric D2E7 monoclonal antibodies (MAbs) with a further caninised antibody based on anti-human TNF MAb clone 148. The caninised anti-huTNF MAb 148 (SEQ ID NO:26 and SEQ ID NO:27, Figure 17) was expressed in CHO cells and purified using Protein A chromatography (Panel A, left lane). The caninised 148 MAb was tested for binding to human TNF (Panel B) and canine TNF (Panel C) in comparison to caninised (Ca) and chimeric (Ch) D2E7 based MAbs from Figures 14 and 15 (background negative control binding is shown by the arrows). It can be seen from panels B and C that the caninised MAb 148 binds to human TNF, but not canine TNF. Accordingly, the caninised and chimeric MAbs based on D2E7 and the subject of this disclosure show unexpectedly strong binding to canine TNF equivalent to that to human TNF whereas caninised MAb 148 shows binding to human TNF alone. Therefore, caninised D2E7 based MAbs are surprisingly useful for the treatment of canine diseases mediated by canine TNF.

Taken together, the canine, equine, feline and new human versions of thePETised rat anti-NGF MAb αD11 and the canine PETised versions of the human anti-TNF MAb demonstrate that the PETising conversion of variable domain frameworks by the method described in this patent is robust, reproducible and applicable to multi-species conversion of multiple MAbs.

### Example 4 - Effect of anti-canine NGF monoclonal antibodies in reducing inflammatory pain in vivo

### Antibody therapy

Anti-canine NGF monoclonal antibodies derived from expression vectors expressing SEQ ID NO:7 and SEQ ID NO:70 (canine HCA type heavy chain) were expressed in CHO cells and purified by a combination of ion exchange chromatography, hydrophobic interaction chromatography and size exclusion chromatography and buffer exchanged into phosphate buffered saline.

### Canine model of inflammation

All experiments were carried out with prior approval of the Institutional Ethics Committee (CRL, Ireland). Beagle dogs were injected (= day -1) with kaolin into the footpad of one hind leg in order to generate a self-resolving inflammation beginning approximately 24 hours later and which causes the dogs to become temporarily lame. In this model, once the initial inflammation response to kaolin recedes, the dogs become steadily less lame over the period of approximately 1-2 weeks and then make a full recovery.

Groups of 3 dogs were injected intravenously with either anti-canine NGF monoclonal antibodies at 200 µg/kg body weight or phosphate buffered saline as vehicle control (= day 0). The dogs were assessed for lameness over 7 days by a visual scoring method (score 0, no lameness (full weight bearing); score 1, slight lameness (not full weight bearing but walking well); score 2, moderate lameness (slightly weight bearing and not walking well), score 3, severe lameness (not weight bearing)). Observers were blinded to which dogs received which injection.

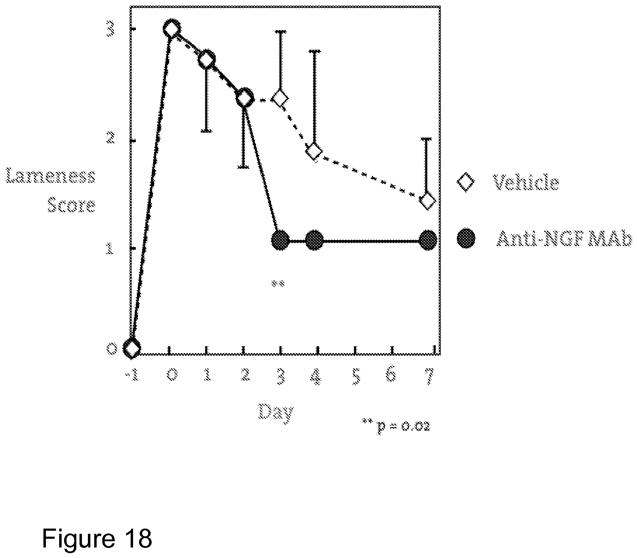

The results are shown in Figure 18. Lameness scores were reduced in the dogs receiving anti-NGF monoclonal antibodies by day 3 post-injection compared with vehicle control, indicating that the anti-NGF monoclonal antibodies had an effect in reducing the pain in the dogs over that seen with vehicle alone. The delayed activity is consistent with the plasma pharmacokinetics of anti-canine NGF monoclonal antibodies which demonstrated a slow tissue distribution (alpha) phase of approximately 30 hours and the relatively poor vascularisation of the footpad area. The results shown in Figure 18 show that the anti-canine NGF antibodies produced by the method of the present disclosure reduce inflammatory pain in dogs with a consequent reduction in lameness.

Various modifications and variations to the described instances of the disclosures will be apparent to those skilled in the art without departing from the scope of the disclosure. The disclosure has been described in connection with specific preferred instances. Indeed, various modifications of the described modes of carrying out the disclosure which are obvious to those skilled in the art are intended to be covered by the present disclosure.

## Claims

1. A method of modifying a donor immunoglobulin for use in a target species, the method comprising the steps of:
- identifying a donor immunoglobulin from a species other than the target species, wherein the donor immunoglobulin has binding specificity to a target epitope present in the target species,
- determining an amino acid sequence of framework regions of heavy and light chain variable domains of the donor immunoglobulin,
- comparing each amino acid residue of the amino acid sequence of the framework regions of the heavy and light chain variable domains of the donor immunoglobulin with an amino acid residue present at a corresponding position in an amino acid sequence of framework regions of a plurality of immunoglobulins derived from the target species to identify one or more amino acid residue within the amino acid sequence of the framework regions of the heavy and light chain variable domains of the donor immunoglobulin that is not present at the corresponding position in the amino acid sequence of the framework regions of at least one of the plurality of immunoglobulins derived from the target species, and
- substituting the one or more identified amino acid residues present in the amino acid sequence of the framework regions of the heavy and light chain variable domains of the donor immunoglobulin, but not present at the corresponding position in the amino acid sequence of the framework regions of at least one of the plurality of immunoglobulins derived from the target species, with an amino acid residue which is present at the corresponding position in the amino acid sequence of the framework regions of at least one of the plurality of immunoglobulins derived from the target species;
- wherein the modified donor immunoglobulin does not contain any amino acid at any position within the framework regions which would be foreign at that position in the target species;
wherein the target species is selected from the group consisting of a dog, a cat, a horse and a human;
and wherein substitution of an amino acid residue present in the amino acid sequence of the framework regions of the heavy and light chain variable domains of the donor immunoglobulin is undertaken using the principle of conservative substitution.

2. The method as claimed in claim 1, further comprising the step of replacing at least one constant domain of the heavy and light chain of the donor immunoglobulin with a constant domain of a heavy and/or light chain derived from an immunoglobulin derived from the target species.

3. A humanised neutralising antibody or an antigen binding fragment thereof which is capable of specifically binding to human nerve growth factor (NGF) wherein the antibody or antigen binding fragment comprises a light chain variable region comprising the amino acid sequence of SEQ ID NO:13 and a heavy chain variable region comprising the amino acid sequence of SEQ ID NO:14, wherein the antibody or the antigen binding fragment thereof does not contain any amino acid at any position within the framework regions which would be foreign at that position in a target species.

4. The humanised neutralising antibody or antigen binding fragment thereof as claimed in claim 3 wherein the light chain comprises the amino acid sequence of SEQ ID NO:25, or an amino acid sequence which has an identity of at least 85% thereto.

5. The humanised neutralising antibody or antigen binding fragment thereof as claimed in claim 3 or claim 4 wherein the heavy chain comprises the amino acid sequence of SEQ ID NO:24, or an amino acid sequence which has a sequence identity of at least 85% thereto.

6. A pharmaceutical composition comprising the humanised neutralising antibody or antigen binding fragment thereof as claimed in any one of claims 3 to 5 and at least one pharmaceutically acceptable diluent or carrier.

7. The humanised neutralising antibody or antigen binding fragment as claimed in any one of claims 3 to 5, or the pharmaceutical composition as claimed in claim 6, for use in the treatment or prevention of pain in a human.

8. The human neutralising antibody or antigen binding fragment as claimed in any one of claims 3 to 5, or the pharmaceutical composition as claimed in claim 6, for use in the treatment of arthritis in a human.

## Patentansprüche

1. Verfahren zur Modifizierung eines Donor-Immunglobulins zur Verwendung in einer Zielspezies, wobei das Verfahren folgende Schritte umfasst:
- Identifizierung eines Donor-Immunglobulins aus einer von der Zielspezies unterschiedlichen Spezies, wobei das Donor-Immunglobulin eine Bindungsspezifität für ein Zielepitop hat, welches in der Zielspezies anwesend ist,
- Bestimmung einer Aminosäuresequenz der Gerüstregionen der variablen Domänen der schweren und leichten Kette des Donor-Immunglobulins,
- Vergleich jedes Aminosäurerests der Aminosäuresequenz der Gerüstregionen der variablen Domänen der schweren und leichten Kette des Donor-Immunglobulins mit einem Aminosäurerest, welcher an einer entsprechenden Position in einer Aminosäuresequenz der Gerüstregionen einer Vielzahl von Immunglobulinen, welche aus der Zielspezies abgeleitet sind, um einen oder mehrere Aminosäurereste innerhalb der Aminosäuresequenz der Gerüstregionen der variablen Domänen der schweren und leichten Kette des Donor-Immunglobulins zu identifizieren, welche(r) nicht an der entsprechenden Position in der Aminosäuresequenz der Gerüstregionen von wenigstens einer der Vielzahl von Immunglobulinen, welche aus der Zielspezies abgeleitet sind, anwesend ist (sind),
- Substituierung des einen oder der mehreren identifizierten Aminosäurereste, welche(r) in der Aminosäuresequenz der Gerüstregionen der variablen Domänen der schweren und leichten Kette des Donor-Immunglobulins anwesend ist (sind), aber welche(r) nicht an der entsprechenden Position in der Aminosäuresequenz der Gerüstregionen der wenigstens einen der Vielzahl von Immunglobulinen, welche aus den Zielspezies stammen, anwesend ist (sind), mit einem Aminosäurerest, welcher an der entsprechenden Position in der Aminosäuresequenz der Gerüstregionen der wenigstens einen der Vielzahl von Immunglobulinen, welche aus den Zielspezies abgeleitet sind, anwesend ist;
- wobei das modifizierte Donor-Immunglobulin keine Aminosäure an einer beliebigen Position innerhalb der Gerüstregionen enthält, welche an dieser Position fremd in den Zielspezies sein würde;
wobei die Zielspezies ausgewählt ist aus der Gruppe, bestehend aus einem Hund, einer Katze, einem Pferd und einem Menschen;
und wobei die Substituierung eines Aminosäurerestes, welcher in der Aminosäuresequenz der Gerüstregionen der variablen Domänen der schweren und leichten Kette des Donor-Immunglobulins anwesend ist, unternommen wird, unter Verwendung des Prinzips der konservativen Substitution.

2. Verfahren, wie in Anspruch 1 beansprucht, zusätzlich umfassend den Schritt des Ersetzens wenigstens einer konstanten Domäne der schweren und leichten Kette des Donor-Immunglobulins mit einer konstanten Domäne einer schweren und/oder leichten Kette, abgeleitet aus einem Immunglobulin, welches aus der Zielspezies stammt.

3. Humanisierter neutralisierender Antikörper oder ein Antigen-bindendes Fragment davon, welcher(s) in der Lage ist, spezifisch an den humanen Nervenwachstumsfaktor (NGF) zu binden, wobei der Antikörper oder das Antigen-bindende Fragment eine variable Region der leichten Kette, umfassend die Aminosäuresequenz von SEQ ID NO:13, und eine variable Region der schweren Kette, umfassend die Aminosäuresequenz von SEQ ID NO:14, umfasst, wobei der Antikörper oder das Antigen-bindende Fragment davon keine Aminosäure an einer unbeliebigen Position enthält innerhalb der Gerüstregionen, welche fremd an dieser Position in einer Zielspezies sein würde.

4. Humanisierter neutralisierender Antikörper oder Antigen-bindendes Fragment davon, wie in Anspruch 3 beansprucht, wobei die leichte Kette die Aminosäuresequenz von SEQ ID NO:25, oder eine Aminosäuresequenz, welche eine Identität von mindestens 85% dazu aufweist, umfasst.

5. Humanisierter neutralisierender Antikörper oder Antigen-bindendes Fragment davon, wie in Anspruch 3 oder 4 beansprucht, wobei die schwere Kette die Aminosäuresequenz von SEQ ID NO:24, oder eine Aminosäuresequenz, welche eine Sequenzidentität von mindestens 85% dazu hat, umfasst.

6. Pharmazeutische Zusammensetzung, umfassend den humanisierten neutralisierenden Antikörper oder ein Antigen-bindendes Fragment davon, wie in einem der Ansprüche 3 bis 5 beansprucht, und mindestens ein pharmazeutisch verträgliches Verdünnungsmittel oder einen pharmazeutisch verträglichen Trägerstoff.

7. Humanisierter neutralisierender Antikörper oder Antigen-bindendes Fragment, wie in einem der Ansprüche 3 bis 5 beansprucht, oder pharmazeutische Zusammensetzung, wie in Anspruch 6 beansprucht, zur Verwendung in der Behandlung oder Prävention von Schmerz bei einem Menschen.

8. Menschlicher neutralisierender Antikörper oder Antigen-bindendes Fragment, wie in einem der Ansprüche 3 bis 5 beansprucht, oder pharmazeutische Zusammensetzung, wie in Anspruch 6 beansprucht, zur Verwendung in der Behandlung von Arthritis bei einem Menschen.

## Revendications

1. Procédé de modification d'une immunoglobuline donneuse pour une utilisation dans une espèce cible, le procédé comprenant les étapes suivantes :
- l'identification d'une immunoglobuline donneuse d'une espèce autre que l'espèce cible, dans lequel l'immunoglobuline donneuse présente une spécificité de liaison envers un épitope cible présent dans l'espèce cible,
- la détermination d'une séquence d'acides aminés de régions charpentes des domaines variables des chaînes lourdes et légères de l'immunoglobuline donneuse,
- la comparaison de chaque résidu d'acide aminé de la séquence d'acides aminés des régions charpentes des domaines variables des chaînes lourdes et légères de l'immunoglobuline donneuse à un résidu d'acide aminé présent à une position correspondante dans une séquence d'acides aminés de régions charpentes d'une pluralité d'immunoglobulines dérivées de l'espèce cible pour identifier un ou plusieurs résidus d'acides aminés au sein de la séquence d'acides aminés des régions charpentes des domaines variables des chaînes lourdes et légères de l'immunoglobuline donneuse qui ne sont pas présents à la position correspondante dans la séquence d'acides aminés des régions charpentes d'au moins l'une de la pluralité d'immunoglobulines dérivées de l'espèce cible, et
- la substitution de l'un ou plusieurs résidus d'acides aminés identifiés présents dans la séquence d'acides aminés des régions charpentes des domaines variables des chaînes lourdes et légères de l'immunoglobuline donneuse, mais non présents à la position correspondante dans la séquence d'acides aminés des régions charpentes d'au moins l'une de la pluralité d'immunoglobulines dérivées de l'espèce cible, par un résidu d'acide aminé qui est présent à la position correspondante dans la séquence d'acides aminés des régions charpentes d'au moins l'une de la pluralité d'immunoglobulines dérivées de l'espèce cible ;
- dans lequel l'immunoglobuline donneuse modifiée ne contient aucun acide aminé à aucune position au sein des régions charpentes qui serait étranger au niveau de cette position dans l'espèce cible ;
dans lequel l'espèce cible est choisie dans le groupe constitué d'un chien, d'un chat, d'un cheval et d'un être humain ;
et dans lequel la substitution d'un résidu d'acide aminé présent dans la séquence d'acides aminés des régions charpentes des domaines variables des chaînes lourdes et légères de l'immunoglobuline donneuse est entreprise en utilisant le principe de substitution conservative.

2. Procédé selon la revendication 1, comprenant en outre l'étape de remplacement d'au moins un domaine constant de la chaîne lourde et légère de l'immunoglobuline donneuse par un domaine constant d'une chaîne lourde et/ou légère dérivé d'une immunoglobuline dérivée de l'espèce cible.

3. Anticorps neutralisant humanisé ou l'un de ses fragments de liaison de l'antigène qui est capable de se lier spécifiquement au facteur de croissance des nerfs (NGF) humain où l'anticorps ou le fragment de liaison de l'antigène comprend une région variable de chaîne légère comprenant la séquence d'acides aminés de SEQ ID NO : 13 et une région variable de chaîne lourde comprenant la séquence d'acides aminés de SEQ ID NO : 14, où l'anticorps ou son fragment de liaison de l'antigène ne contient aucun acide aminé à aucune position au sein des régions charpentes qui serait étranger à cette position dans une espèce cible.

4. Anticorps neutralisant humanisé ou l'un de ses fragments de liaison de l'antigène selon la revendication 3 dans lequel la chaîne légère comprend la séquence d'acides aminés de SEQ ID NO : 25, ou une séquence d'acides aminés qui présente une identité d'au moins 85 % avec celle-ci.

5. Anticorps neutralisant humanisé ou l'un de ses fragments de liaison de l'antigène selon la revendication 3 ou la revendication 4 dans lequel la chaîne lourde comprend la séquence d'acides aminés de SEQ ID NO : 24, ou une séquence d'acides aminés qui présente une identité de séquence d'au moins 85 % avec celle-ci.

6. Composition pharmaceutique comprenant l'anticorps neutralisant humanisé ou l'un de ses fragments de liaison de l'antigène selon l'une quelconque des revendications 3 à 5 et au moins un diluant ou support pharmaceutiquement acceptable.

7. Anticorps neutralisant humanisé ou l'un de ses fragments de liaison de l'antigène selon l'une quelconque des revendications 3 à 5, ou composition pharmaceutique selon la revendication 6, pour une utilisation dans le traitement ou la prévention de la douleur chez un être humain.

8. Anticorps neutralisant humanisé ou l'un de ses fragments de liaison de l'antigène selon l'une quelconque des revendications 3 à 5, ou composition pharmaceutique selon la revendication 6, pour une utilisation dans le traitement de l'arthrite chez un être humain.


### SEQUENCE LISTING

<110> NVIP Pty Ltd Gearing, David
<120> Modified antibodies and method for the production of same
<130> P120110.WO.01
<150> US 61/531439
   <151> 2011-09-06
<160> 71
<170> PatentIn version 3.5
<210> 1
   <211> 107
   <212> PRT
   <213> Artificial Sequence
<220>
   <223> light chain variable domain of a canine version of alpha D11 anti-NGF MAb - canine VK
<400> 1
   Asp Ile Val Met Thr Gln Ser Pro Ala Ser Leu Ser Leu Ser Gln Glu
   1               5                   10                  15
   Glu Lys Val Thr Ile Thr Cys Arg Ala Ser Glu Asp Ile Tyr Asn Ala
               20                  25                  30
   Leu Ala Trp Tyr Gln Gln Lys Pro Gly Gln Ala Pro Lys Leu Leu Ile
           35                  40                  45
   Tyr Asn Thr Asp Thr Leu His Thr Gly Val Pro Ser Arg Phe Ser Gly
       50                  55                  60
   Ser Gly Ser Gly Thr Glu Phe Ser Leu Thr Ile Ser Ser Leu Glu Pro
   65                  70                  75                  80
   Glu Asp Val Ala Val Tyr Tyr Cys Gln His Tyr Phe His Tyr Pro Arg
                   85                  90                  95
   Thr Phe Gly Ala Gly Thr Lys Val Glu Leu Lys
               100                 105
<210> 2
   <211> 122
   <212> PRT
   <213> Artificial Sequence
<220>
   <223> heavy chain variable domain of a canine version of alpha D11 anti-NGF MAb - canine VH
<400> 2
   Glu Val Gln Leu Val Glu Ser Gly Gly Asp Leu Val Asn Pro Gly Gly
   1               5                   10                  15
   Thr Leu Thr Leu Ser Cys Val Val Ser Gly Phe Ser Leu Thr Asn Asn
               20                  25                  30
   Asn Val Asn Trp Val Arg Gln Ala Leu Gly Arg Gly Leu Glu Trp Val
           35                  40                  45
   Gly Gly Val Trp Ala Gly Gly Ala Thr Asp Tyr Asn Ser Ala Leu Lys
       50                  55                  60
   Ser Arg Leu Thr Ile Thr Arg Asp Thr Ser Lys Ser Thr Val Phe Leu
   65                  70                  75                  80
   Gln Met His Ser Leu Arg Ser Glu Asp Thr Ala Thr Tyr Tyr Cys Ala
                   85                  90                  95
   Arg Asp Gly Gly Tyr Ser Ser Ser Thr Leu Tyr Ala Met Asp Ala Trp
               100                 105                 110
   Gly Gln Gly Thr Leu Val Thr Val Ser Ser
           115                 120
<210> 3
   <211> 107
   <212> PRT
   <213> Artificial Sequence
<220>
   <223> light chain variable domain of a feline version of alpha D11 anti-NGF MAb - feline VK
<400> 3
   Asp Ile Val Met Thr Gln Thr Pro Leu Ser Leu Ser Val Thr Pro Gly
   1               5                   10                  15
   Glu Pro Ala Ser Ile Ser Cys Arg Ala Ser Glu Asp Ile Tyr Asn Ala
               20                  25                  30
   Leu Ala Trp Tyr Leu Gln Lys Pro Gly Gln Ser Pro Arg Arg Leu Ile
           35                  40                  45
   Tyr Asn Thr Asp Thr Leu His Thr Gly Val Pro Asp Arg Phe Ser Gly
       50                  55                  60
   Ser Gly Ser Gly Thr Asp Phe Thr Leu Arg Ile Ser Arg Val Glu Ala
   65 70 75 80
   Asp Asp Val Gly Val Tyr Phe Cys Gln His Tyr Phe His Tyr Pro Arg
   85 90 95
   Thr Phe Gly Pro Gly Thr Lys Leu Glu Ile Lys
   100 105
<210> 4
   <211> 122
   <212> PRT
   <213> Artificial Sequence
<220>
   <223> heavy chain variable domain of a feline version of alpha D11 anti-NGF MAb - feline VH
<400> 4
   Gln Val Gln Leu Val Glu Ser Gly Gly Asp Leu Val Gln Pro Gly Gly
   1 5 10 15
   Ser Leu Arg Leu Thr Cys Ala Ala Ser Gly Phe Ser Leu Thr Asn Asn
   20 25 30
   Asn Val Asn Trp Val Arg Gln Ala Pro Gly Lys Gly Leu Glu Trp Met
   35 40 45
   Gly Gly Val Trp Ala Gly Gly Ala Thr Asp Tyr Asn Ser Ala Leu Lys
   50 55 60
   Gly Arg Phe Thr Ile Ser Arg Asp Asn Ala Lys Asn Thr Leu Tyr Leu
   65 70 75 80
   Gln Met Asn Ser Leu Lys Thr Glu Asp Thr Ala Thr Tyr Tyr Cys Ala
   85 90 95
   Arg Asp Gly Gly Tyr Ser Ser Ser Thr Leu Tyr Ala Met Asp Ala Trp
   100 105 110
   Gly Gln Gly Thr Leu Val Thr Val Ser Ser
   115 120
<210> 5
   <211> 107
   <212> PRT
   <213> Artificial Sequence
<220>
   <223> light chain variable domain of an equine version of alpha D11 anti-NGF MAb - equine VK
<400> 5
   Asp Ile Val Met Thr Gln Ser Pro Ala Ser Leu Ser Ala Ser Leu Gly
   1               5                   10                  15
   Glu Thr Val Thr Ile Glu Cys Arg Ala Ser Glu Asp Ile Tyr Asn Ala
               20                  25                  30
   Leu Ala Trp Tyr Gln Gln Lys Pro Gly Gln Ser Pro Lys Leu Leu Ile
           35                  40                  45
   Tyr Asn Thr Asp Thr Leu His Thr Gly Val Pro Ser Arg Phe Ser Gly
       50                  55                  60
   Ser Gly Ser Gly Thr Asp Tyr Ser Leu Thr Ile Asn Ser Leu Gln Ser
   65                  70                  75                  80
   Glu Asp Val Ala Ser Tyr Phe Cys Gln His Tyr Phe His Tyr Pro Arg
                   85                  90                  95
   Thr Phe Gly Gln Gly Thr Lys Leu Glu Leu Lys
               100                 105
<210> 6
   <211> 122
   <212> PRT
   <213> Artificial Sequence
<220>
   <223> heavy chain variable domain of an equine version of alpha D11 anti-NGF MAb - equine VH
<400> 6
   Gln Val Gln Leu Lys Glu Ser Gly Pro Gly Leu Val Asn Pro Ser Gln
   1               5                   10                  15
   Thr Leu Ser Leu Thr Cys Thr Val Ser Gly Phe Ser Leu Thr Asn Asn
               20                  25                  30
   Asn Val Asn Trp Val Arg Gln Ala Pro Gly Lys Gly Leu Glu Trp Val
           35                  40                  45
   Gly Gly Val Trp Ala Gly Gly Ala Thr Asp Tyr Asn Ser Ala Leu Lys
       50                  55                  60
   Ser Arg Ala Thr Ile Thr Arg Asp Thr Ser Lys Ser Gln Val Phe Leu
   65                  70                  75                  80
   Gln Met Asn Ser Leu Thr Ser Glu Asp Thr Ala Val Tyr Tyr Cys Ala
                   85                  90                  95
   Arg Asp Gly Gly Tyr Ser Ser Ser Thr Leu Tyr Ala Met Asp Ala Trp
               100                 105                 110
   Gly Gln Gly Ile Leu Val Thr Val Ser Ser
           115                 120
<210> 7
   <211> 217
   <212> PRT
   <213> Artificial Sequence
<220>
   <223> complete light chain of a caninised version of alpha D11 anti-NGF MAb - canine VK and canine kappa light chain constant
<400> 7
   Asp Ile Val Met Thr Gln Ser Pro Ala Ser Leu Ser Leu Ser Gln Glu
   1               5                   10                  15
   Glu Lys Val Thr Ile Thr Cys Arg Ala Ser Glu Asp Ile Tyr Asn Ala
               20                  25                  30
   Leu Ala Trp Tyr Gln Gln Lys Pro Gly Gln Ala Pro Lys Leu Leu Ile
           35                  40                  45
   Tyr Asn Thr Asp Thr Leu His Thr Gly Val Pro Ser Arg Phe Ser Gly
       50                  55                  60
   Ser Gly Ser Gly Thr Glu Phe Ser Leu Thr Ile Ser Ser Leu Glu Pro
   65                  70                  75                  80
   Glu Asp Val Ala Val Tyr Tyr Cys Gln His Tyr Phe His Tyr Pro Arg
                   85                  90                  95
   Thr Phe Gly Ala Gly Thr Lys Val Glu Leu Lys Arg Asn Asp Ala Gln
               100                 105                 110
   Pro Ala Val Tyr Leu Phe Gln Pro Ser Pro Asp Gln Leu His Thr Gly
           115                 120                 125
   Ser Ala Ser Val Val Cys Leu Leu Asn Ser Phe Tyr Pro Lys Asp Ile
       130                 135                 140
   Asn Val Lys Trp Lys Val Asp Gly Val Ile Gln Asp Thr Gly Ile Gln
   145                 150                 155                 160
   Glu Ser Val Thr Glu Gln Asp Lys Asp Ser Thr Tyr Ser Leu Ser Ser
                   165                 170                 175
   Thr Leu Thr Met Ser Ser Thr Glu Tyr Leu Ser His Glu Leu Tyr Ser
               180                 185                 190
   Cys Glu Ile Thr His Lys Ser Leu Pro Ser Thr Leu Ile Lys Ser Phe
           195                 200                 205
   Gln Arg Ser Glu Cys Gln Arg Val Asp
       210                 215
<210> 8
   <211> 457
   <212> PRT
   <213> Artificial Sequence
<220>
   <223> complete heavy chain of a canine version of alpha D11 anti-NGF MAb - canine VH and canine heavy chain B constant
<400> 8
   Glu Val Gln Leu Val Glu Ser Gly Gly Asp Leu Val Asn Pro Gly Gly
   1               5                   10                  15
   Thr Leu Thr Leu Ser Cys Val Val Ser Gly Phe Ser Leu Thr Asn Asn
               20                  25                  30
   Asn Val Asn Trp Val Arg Gln Ala Leu Gly Arg Gly Leu Glu Trp Val
           35                  40                  45
   Gly Gly Val Trp Ala Gly Gly Ala Thr Asp Tyr Asn Ser Ala Leu Lys
       50                  55                  60
   Ser Arg Leu Thr Ile Thr Arg Asp Thr Ser Lys Ser Thr Val Phe Leu
   65                  70                  75                  80
   Gln Met His Ser Leu Arg Ser Glu Asp Thr Ala Thr Tyr Tyr Cys Ala
                   85                  90                  95
   Arg Asp Gly Gly Tyr Ser Ser Ser Thr Leu Tyr Ala Met Asp Ala Trp
               100                 105                 110
   Gly Gln Gly Thr Leu Val Thr Val Ser Ser Ala Ser Thr Thr Ala Pro
           115                 120                 125
   Ser Val Phe Pro Leu Ala Pro Ser Cys Gly Ser Thr Ser Gly Ser Thr
       130                 135                 140
   Val Ala Leu Ala Cys Leu Val Ser Gly Tyr Phe Pro Glu Pro Val Thr
   145                 150                 155                 160
   Val Ser Trp Asn Ser Gly Ser Leu Thr Ser Gly Val His Thr Phe Pro
                   165                 170                 175
   Ser Val Leu Gln Ser Ser Gly Leu Tyr Ser Leu Ser Ser Met Val Thr
               180                 185                 190
   Val Pro Ser Ser Arg Trp Pro Ser Glu Thr Phe Thr Cys Asn Val Ala
           195                 200                 205
   His Pro Ala Ser Lys Thr Lys Val Asp Lys Pro Val Pro Lys Arg Glu
       210                 215                 220
   Asn Gly Arg Val Pro Arg Pro Pro Asp Cys Pro Lys Cys Pro Ala Pro
   225                 230                 235                 240
   Glu Met Leu Gly Gly Pro Ser Val Phe Ile Phe Pro Pro Lys Pro Lys
                   245                 250                 255
   Asp Thr Leu Leu Ile Ala Arg Thr Pro Glu Val Thr Cys Val Val Val
               260                 265                 270
   Asp Leu Asp Pro Glu Asp Pro Glu Val Gln Ile Ser Trp Phe Val Asp
           275                 280                 285
   Gly Lys Gln Met Gln Thr Ala Lys Thr Gln Pro Arg Glu Glu Gln Phe
       290                 295                 300
   Asn Gly Thr Tyr Arg Val Val Ser Val Leu Pro Ile Gly His Gln Asp
   305                 310                 315                 320
   Trp Leu Lys Gly Lys Gln Phe Thr Cys Lys Val Asn Asn Lys Ala Leu
                   325                 330                 335
   Pro Ser Pro Ile Glu Arg Thr Ile Ser Lys Ala Arg Gly Gln Ala His
               340                 345                 350
   Gln Pro Ser Val Tyr Val Leu Pro Pro Ser Arg Glu Glu Leu Ser Lys
           355                 360                 365
   Asn Thr Val Ser Leu Thr Cys Leu Ile Lys Asp Phe Tyr Pro Pro Asp
       370                 375                 380
   Ile Asp Val Glu Trp Gln Ser Asn Gly Gln Gln Glu Pro Glu Ser Lys
   385                 390                 395                 400
   Tyr Arg Thr Thr Pro Pro Gln Leu Asp Glu Asp Gly Ser Tyr Phe Leu
   405 410 415
   Tyr Ser Lys Leu Ser Val Asp Lys Ser Arg Trp Gln Arg Gly Asp Thr
   420 425 430
   Phe Ile Cys Ala Val Met His Glu Ala Leu His Asn His Tyr Thr Gln
   435 440 445
   Glu Ser Leu Ser His Ser Pro Gly Lys
   450 455
<210> 9
   <211> 217
   <212> PRT
   <213> Artificial Sequence
<220>
   <223> complete light chain of a feline version of alpha D11 anti-NGF MAb - feline VK and feline kappa light chain constant
<400> 9
   Asp Ile Val Met Thr Gln Thr Pro Leu Ser Leu Ser Val Thr Pro Gly
   1               5                   10                  15
   Glu Pro Ala Ser Ile Ser Cys Arg Ala Ser Glu Asp Ile Tyr Asn Ala
               20                  25                  30
   Leu Ala Trp Tyr Leu Gln Lys Pro Gly Gln Ser Pro Arg Arg Leu Ile
           35                  40                  45
   Tyr Asn Thr Asp Thr Leu His Thr Gly Val Pro Asp Arg Phe Ser Gly
       50                  55                  60
   Ser Gly Ser Gly Thr Asp Phe Thr Leu Arg Ile Ser Arg Val Glu Ala
   65                  70                  75                  80
   Asp Asp Val Gly Val Tyr Phe Cys Gln His Tyr Phe His Tyr Pro Arg
                   85                  90                  95
   Thr Phe Gly Pro Gly Thr Lys Leu Glu Ile Lys Arg Ser Asp Ala Gln
               100                 105                 110
   Pro Ser Val Phe Leu Phe Gln Pro Ser Leu Asp Glu Leu His Thr Gly
           115                 120                 125
   Ser Ala Ser Ile Val Cys Ile Leu Asn Asp Phe Tyr Pro Lys Glu Val
       130                 135                 140
   Asn Val Lys Trp Lys Val Asp Gly Val Val Gln Thr Lys Ala Ser Lys
   145                 150                 155                 160
   Glu Ser Thr Thr Glu Gln Asn Ser Lys Asp Ser Thr Tyr Ser Leu Ser
                   165                 170                 175
   Ser Thr Leu Thr Met Ser Arg Thr Glu Tyr Gln Ser His Glu Lys Phe
               180                 185                 190
   Ser Cys Glu Val Thr His Lys Ser Leu Ala Ser Thr Leu Val Lys Ser
           195                 200                 205
   Phe Asn Arg Ser Glu Cys Gln Arg Glu
       210                 215
<210> 10
   <211> 457
   <212> PRT
   <213> Artificial Sequence
<220>
   <223> complete heavy chain of a feline version of alpha D11 anti-NGF MAb - feline VH and feline heavy chain constant
<400> 10
   Gln Val Gln Leu Val Glu Ser Gly Gly Asp Leu Val Gln Pro Gly Gly
   1               5                   10                  15
   Ser Leu Arg Leu Thr Cys Ala Ala Ser Gly Phe Ser Leu Thr Asn Asn
               20                  25                  30
   Asn Val Asn Trp Val Arg Gln Ala Pro Gly Lys Gly Leu Glu Trp Met
           35                  40                  45
   Gly Gly Val Trp Ala Gly Gly Ala Thr Asp Tyr Asn Ser Ala Leu Lys
       50                  55                  60
   Gly Arg Phe Thr Ile Ser Arg Asp Asn Ala Lys Asn Thr Leu Tyr Leu
   65                  70                  75                  80
   Gln Met Asn Ser Leu Lys Thr Glu Asp Thr Ala Thr Tyr Tyr Cys Ala
                   85                  90                  95
   Arg Asp Gly Gly Tyr Ser Ser Ser Thr Leu Tyr Ala Met Asp Ala Trp
               100                 105                 110
   Gly Gln Gly Thr Leu Val Thr Val Ser Ser Ala Ser Thr Thr Ala Pro
           115                 120                 125
   Ser Val Phe Pro Leu Ala Pro Ser Cys Gly Thr Thr Ser Gly Ala Thr
   130 135 140
   Val Ala Leu Ala Cys Leu Val Leu Gly Tyr Phe Pro Glu Pro Val Thr
   145 150 155 160
   Val Ser Trp Asn Ser Gly Ala Leu Thr Ser Gly Val His Thr Phe Pro
   165 170 175
   Ala Val Leu Gln Ala Ser Gly Leu Tyr Ser Leu Ser Ser Met Val Thr
   180 185 190
   Val Pro Ser Ser Arg Trp Leu Ser Asp Thr Phe Thr Cys Asn Val Ala
   195 200 205
   His Pro Pro Ser Asn Thr Lys Val Asp Lys Thr Val Arg Lys Thr Asp
   210 215 220
   His Pro Pro Gly Pro Lys Pro Cys Asp Cys Pro Lys Cys Pro Pro Pro
   225 230 235 240
   Glu Met Leu Gly Gly Pro Ser Ile Phe Ile Phe Pro Pro Lys Pro Lys
   245 250 255
   Asp Thr Leu Ser Ile Ser Arg Thr Pro Glu Val Thr Cys Leu Val Val
   260 265 270
   Asp Leu Gly Pro Asp Asp Ser Asp Val Gln Ile Thr Trp Phe Val Asp
   275 280 285
   Asn Thr Gln Val Tyr Thr Ala Lys Thr Ser Pro Arg Glu Glu Gln Phe
   290 295 300
   Asn Ser Thr Tyr Arg Val Val Ser Val Leu Pro Ile Leu His Gln Asp
   305 310 315 320
   Trp Leu Lys Gly Lys Glu Phe Lys Cys Lys Val Asn Ser Lys Ser Leu
   325 330 335
   Pro Ser Pro Ile Glu Arg Thr Ile Ser Lys Ala Lys Gly Gln Pro His
   340 345 350
   Glu Pro Gln Val Tyr Val Leu Pro Pro Ala Gln Glu Glu Leu Ser Arg
   355 360 365
   Asn Lys Val Ser Val Thr Cys Leu Ile Lys Ser Phe His Pro Pro Asp
   370 375 380
   Ile Ala Val Glu Trp Glu Ile Thr Gly Gln Pro Glu Pro Glu Asn Asn
   385 390 395 400
   Tyr Arg Thr Thr Pro Pro Gln Leu Asp Ser Asp Gly Thr Tyr Phe Val
   405 410 415
   Tyr Ser Lys Leu Ser Val Asp Arg Ser His Trp Gln Arg Gly Asn Thr
   420 425 430
   Tyr Thr Cys Ser Val Ser His Glu Ala Leu His Ser His His Thr Gln
   435 440 445
   Lys Ser Leu Thr Gln Ser Pro Gly Lys
   450 455
<210> 11
   <211> 214
   <212> PRT
   <213> Artificial Sequence
<220>
   <223> complete light chain of an equine version of alpha D11 anti-NGF MAb - equine VK and equine kappa light chain constant
<400> 11
   Asp Ile Val Met Thr Gln Ser Pro Ala Ser Leu Ser Ala Ser Leu Gly
   1 5 10 15
   Glu Thr Val Thr Ile Glu Cys Arg Ala Ser Glu Asp Ile Tyr Asn Ala
   20 25 30
   Leu Ala Trp Tyr Gln Gln Lys Pro Gly Gln Ser Pro Lys Leu Leu Ile
   35 40 45
   Tyr Asn Thr Asp Thr Leu His Thr Gly Val Pro Ser Arg Phe Ser Gly
   50 55 60
   Ser Gly Ser Gly Thr Asp Tyr Ser Leu Thr Ile Asn Ser Leu Gln Ser
   65 70 75 80
   Glu Asp Val Ala Ser Tyr Phe Cys Gln His Tyr Phe His Tyr Pro Arg
   85 90 95
   Thr Phe Gly Gln Gly Thr Lys Leu Glu Leu Lys Arg Asp Asp Ala Lys
   100 105 110
   Pro Ser Ala Phe Ile Phe Pro Pro Ser Ser Glu Glu Leu Ser Ser Gly
   115 120 125
   Ser Ala Ser Val Val Cys Leu Val Tyr Gly Phe Tyr Pro Ser Gly Ala
   130 135 140
   Thr Ile Asn Trp Lys Val Asp Gly Leu Ala Lys Thr Ser Ser Phe His
   145 150 155 160
   Ser Ser Leu Thr Glu Gln Asp Ser Lys Asp Asn Thr Tyr Ser Leu Ser
   165 170 175
   Ser Thr Leu Thr Leu Pro Lys Ala Asp Tyr Glu Ala His Asn Val Tyr
   180 185 190
   Ala Cys Glu Val Ser His Lys Thr Leu Ser Ser Pro Leu Val Lys Ser
   195 200 205
   Phe Lys Arg Gln Asp Cys
   210
<210> 12
   <211> 464
   <212> PRT
   <213> Artificial Sequence
<220>
   <223> complete heavy chain of an equine version of alpha D11 anti-NGF MAb - equine VH and equine heavy chain 2 constant
<400> 12
   Gln Val Gln Leu Lys Glu Ser Gly Pro Gly Leu Val Asn Pro Ser Gln
   1 5 10 15
   Thr Leu Ser Leu Thr Cys Thr Val Ser Gly Phe Ser Leu Thr Asn Asn
   20 25 30
   Asn Val Asn Trp Val Arg Gln Ala Pro Gly Lys Gly Leu Glu Trp Val
   35 40 45
   Gly Gly Val Trp Ala Gly Gly Ala Thr Asp Tyr Asn Ser Ala Leu Lys
   50 55 60
   Ser Arg Ala Thr Ile Thr Arg Asp Thr Ser Lys Ser Gln Val Phe Leu
   65 70 75 80
   Gln Met Asn Ser Leu Thr Ser Glu Asp Thr Ala Val Tyr Tyr Cys Ala
   85 90 95
   Arg Asp Gly Gly Tyr Ser Ser Ser Thr Leu Tyr Ala Met Asp Ala Trp
   100 105 110
   Gly Gln Gly Ile Leu Val Thr Val Ser Ser Ala Ser Thr Thr Ala Pro
           115                 120                 125
   Lys Tyr Phe Gln Leu Thr Pro Ser Cys Gly Ile Thr Ser Asp Ala Thr
       130                 135                 140
   Val Ala Leu Gly Cys Leu Val Ser Asp Tyr Tyr Pro Glu Pro Val Thr
   145                 150                 155                 160
   Val Ser Trp Asn Ser Gly Ala Leu Thr Ser Gly Val His Thr Phe Pro
                   165                 170                 175
   Ser Val Leu Gln Ser Ser Gly Leu Tyr Ala Leu Ser Ser Met Val Thr
               180                 185                 190
   Val Pro Ala Ser Thr Trp Thr Ser Glu Thr Tyr Ile Cys Asn Val Ala
           195                 200                 205
   His Pro Ala Ser Ser Thr Lys Val Asp Lys Arg Ile Pro Pro Cys Val
       210                 215                 220
   Leu Ser Ala Glu Gly Val Ile Pro Ile Pro Ser Val Pro Lys Pro Gln
   225                 230                 235                 240
   Cys Pro Pro Tyr Thr His Ser Lys Phe Leu Gly Gly Pro Ser Val Phe
                   245                 250                 255
   Ile Phe Pro Pro Asn Pro Lys Asp Ala Leu Met Ile Ser Arg Thr Pro
               260                 265                 270
   Val Val Thr Cys Val Val Val Asn Leu Ser Asp Gln Tyr Pro Asp Val
           275                 280                 285
   Gln Phe Ser Trp Tyr Val Asp Asn Thr Glu Val His Ser Ala Ile Thr
       290                 295                 300
   Lys Gln Arg Glu Ala Gln Phe Asn Ser Thr Tyr Arg Val Val Ser Val
   305                 310                 315                 320
   Leu Pro Ile Gln His Gln Asp Trp Leu Ser Gly Lys Glu Phe Lys Cys
                   325                 330                 335
   Ser Val Thr Asn Val Gly Val Pro Gln Pro Ile Ser Arg Ala Ile Ser
               340                 345                 350
   Arg Gly Lys Gly Pro Ser Arg Val Pro Gln Val Tyr Val Leu Pro Pro
           355                 360                 365
   His Pro Asp Glu Leu Ala Lys Ser Lys Val Ser Val Thr Cys Leu Val
   370 375 380
   Lys Asp Phe Tyr Pro Pro Asp Ile Ser Val Glu Trp Gln Ser Asn Arg
   385 390 395 400
   Trp Pro Glu Leu Glu Gly Lys Tyr Ser Thr Thr Pro Ala Gln Leu Asp
   405 410 415
   Gly Asp Gly Ser Tyr Phe Leu Tyr Ser Lys Leu Ser Leu Glu Thr Ser
   420 425 430
   Arg Trp Gln Gln Val Glu Ser Phe Thr Cys Ala Val Met His Glu Ala
   435 440 445
   Leu His Asn His Phe Thr Lys Thr Asp Ile Ser Glu Ser Leu Gly Lys
   450 455 460
<210> 13
   <211> 107
   <212> PRT
   <213> Artificial Sequence
<220>
   <223> new Hu alpha D11 VL
<400> 13
   Asp Ile Gln Met Thr Gln Ser Pro Ala Ser Leu Ser Ala Ser Leu Gly
   1 5 10 15
   Glu Thr Val Thr Ile Asn Cys Arg Ala Ser Glu Asp Ile Tyr Asn Ala
   20 25 30
   Leu Ala Trp Tyr Gln Gln Lys Pro Gly Lys Ser Pro Gln Leu Leu Ile
   35 40 45
   Tyr Asn Thr Asp Thr Leu His Thr Gly Val Pro Ser Arg Phe Ser Gly
   50 55 60
   Ser Gly Ser Gly Thr Glu Tyr Ser Leu Lys Ile Asn Ser Leu Gln Ser
   65 70 75 80
   Glu Asp Val Ala Ser Tyr Phe Cys Gln His Tyr Phe His Tyr Pro Arg
   85 90 95
   Thr Phe Gly Gly Gly Thr Lys Leu Glu Ile Lys
   100 105
<210> 14
   <211> 122
   <212> PRT
   <213> Artificial Sequence
<220>
   <223> new Hu alpha D11 VH
<400> 14
   Gln Val Gln Leu Lys Glu Ser Gly Pro Gly Leu Val Gln Pro Ser Gln
   1               5                   10                  15
   Thr Leu Ser Leu Thr Cys Thr Val Ser Gly Phe Ser Leu Thr Asn Asn
               20                  25                  30
   Asn Val Asn Trp Val Arg Gln Ala Ser Gly Arg Gly Leu Glu Trp Met
           35                  40                  45
   Gly Gly Val Trp Ala Gly Gly Ala Thr Asp Tyr Asn Ser Ala Leu Lys
       50                  55                  60
   Ser Arg Leu Thr Ile Thr Arg Asp Thr Ser Lys Ser Gln Val Phe Leu
   65                  70                  75                  80
   Lys Met His Ser Leu Gln Ser Glu Asp Thr Ala Thr Tyr Tyr Cys Ala
                   85                  90                  95
   Arg Asp Gly Gly Tyr Ser Ser Ser Thr Leu Tyr Ala Met Asp Ala Trp
               100                 105                 110
   Gly Gln Gly Thr Thr Val Thr Val Ser Ser
           115                 120
<210> 15
   <211> 107
   <212> PRT
   <213> Artificial Sequence
<220>
   <223> light chain variable domain of a canine version of human D2E7 anti-TNF MAb - canine VK
<400> 15
   Asp Ile Val Met Thr Gln Ser Pro Ala Ser Leu Ser Leu Ser Gln Glu
   1               5                   10                  15
   Glu Lys Val Thr Ile Thr Cys Arg Ala Ser Gln Gly Ile Arg Asn Tyr
               20                  25                  30
   Leu Ala Trp Tyr Gln Gln Lys Pro Gly Gln Ala Pro Lys Leu Leu Ile
           35                  40                  45
   Tyr Ala Ala Ser Thr Leu Gln Ser Gly Val Pro Ser Arg Phe Ser Gly
       50                  55                  60
   Ser Gly Ser Gly Thr Asp Phe Thr Leu Thr Ile Ser Ser Leu Glu Pro
   65                  70                  75                  80
   Glu Asp Val Ala Val Tyr Tyr Cys Gln Arg Tyr Asn Arg Ala Pro Tyr
                   85                  90                  95
   Thr Phe Gly Gln Gly Thr Lys Val Glu Ile Lys
               100                 105
<210> 16
   <211> 121
   <212> PRT
   <213> Artificial Sequence
<220>
   <223> heavy chain variable domain of a canine version of human D2E7 anti-TNF MAb - canine VH
<400> 16
   Glu Val Gln Leu Val Glu Ser Gly Gly Gly Leu Val Gln Pro Gly Gly
   1 5 10 15
   Ser Leu Arg Leu Ser Cys Ala Ala Ser Gly Phe Thr Phe Asp Asp Tyr
   20 25 30
   Ala Met His Trp Val Arg Gln Ala Pro Gly Lys Gly Leu Glu Trp Val
   35 40 45
   Ser Ala Ile Thr Trp Asn Ser Gly His Ile Asp Tyr Ala Asp Ser Val
   50 55 60
   Glu Gly Arg Phe Thr Ile Ser Arg Asp Asn Ala Lys Asn Ser Leu Tyr
   65 70 75 80
   Leu Gln Met Asn Ser Leu Arg Ala Glu Asp Thr Ala Val Tyr Tyr Cys
   85 90 95
   Ala Lys Val Ser Tyr Leu Ser Thr Ala Ser Ser Leu Asp Tyr Trp Gly
   100 105 110
   Gln Gly Thr Leu Val Thr Val Ser Ser
   115 120
<210> 17
   <211> 217
   <212> PRT
   <213> Artificial Sequence
<220>
   <223> complete light chain of a canine version of human D2E7 anti-TNF MAb - canine VK and canine kappa light chain constant
<400> 17
   Asp Ile Val Met Thr Gln Ser Pro Ala Ser Leu Ser Leu Ser Gln Glu
   1               5                   10                  15
   Glu Lys Val Thr Ile Thr Cys Arg Ala Ser Gln Gly Ile Arg Asn Tyr
               20                  25                  30
   Leu Ala Trp Tyr Gln Gln Lys Pro Gly Gln Ala Pro Lys Leu Leu Ile
           35                  40                  45
   Tyr Ala Ala Ser Thr Leu Gln Ser Gly Val Pro Ser Arg Phe Ser Gly
       50                  55                  60
   Ser Gly Ser Gly Thr Asp Phe Thr Leu Thr Ile Ser Ser Leu Glu Pro
   65                  70                  75                  80
   Glu Asp Val Ala Val Tyr Tyr Cys Gln Arg Tyr Asn Arg Ala Pro Tyr
                   85                  90                  95
   Thr Phe Gly Gln Gly Thr Lys Val Glu Ile Lys Arg Asn Asp Ala Gln
               100                 105                 110
   Pro Ala Val Tyr Leu Phe Gln Pro Ser Pro Asp Gln Leu His Thr Gly
           115                 120                 125
   Ser Ala Ser Val Val Cys Leu Leu Asn Ser Phe Tyr Pro Lys Asp Ile
       130                 135                 140
   Asn Val Lys Trp Lys Val Asp Gly Val Ile Gln Asp Thr Gly Ile Gln
   145                 150                 155                 160
   Glu Ser Val Thr Glu Gln Asp Lys Asp Ser Thr Tyr Ser Leu Ser Ser
                   165                 170                 175
   Thr Leu Thr Met Ser Ser Thr Glu Tyr Leu Ser His Glu Leu Tyr Ser
               180                 185                 190
   Cys Glu Ile Thr His Lys Ser Leu Pro Ser Thr Leu Ile Lys Ser Phe
           195                 200                 205
   Gln Arg Ser Glu Cys Gln Arg Val Asp
       210                 215
<210> 18
   <211> 452
   <212> PRT
   <213> Artificial Sequence
<220>
   <223> complete heavy chain of a canine version of human D2E7 anti-TNF MAb - canine VH and canine heavy chain A constant
<400> 18
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Asp Asp Tyr
               20                  25                  30
   Ala Met His Trp Val Arg Gln Ala Pro Gly Lys Gly Leu Glu Trp Val
           35                  40                  45
   Ser Ala Ile Thr Trp Asn Ser Gly His Ile Asp Tyr Ala Asp Ser Val
       50                  55                  60
   Glu Gly Arg Phe Thr Ile Ser Arg Asp Asn Ala Lys Asn Ser Leu Tyr
   65                  70                  75                  80
   Leu Gln Met Asn Ser Leu Arg Ala Glu Asp Thr Ala Val Tyr Tyr Cys
                   85                  90                  95
   Ala Lys Val Ser Tyr Leu Ser Thr Ala Ser Ser Leu Asp Tyr Trp Gly
               100                 105                 110
   Gln Gly Thr Leu Val Thr Val Ser Ser Ala Ser Thr Thr Ala Pro Ser
           115                 120                 125
   Val Phe Pro Leu Ala Pro Ser Cys Gly Ser Thr Ser Gly Ser Thr Val
       130                 135                 140
   Ala Leu Ala Cys Leu Val Ser Gly Tyr Phe Pro Glu Pro Val Thr Val
   145                 150                 155                 160
   Ser Trp Asn Ser Gly Ser Leu Thr Ser Gly Val His Thr Phe Pro Ser
                   165                 170                 175
   Val Leu Gln Ser Ser Gly Leu His Ser Leu Ser Ser Met Val Thr Val
               180                 185                 190
   Pro Ser Ser Arg Trp Pro Ser Glu Thr Phe Thr Cys Asn Val Val His
           195                 200                 205
   Pro Ala Ser Asn Thr Lys Val Asp Lys Pro Val Phe Asn Glu Cys Arg
       210                 215                 220
   Cys Thr Asp Thr Pro Pro Cys Pro Val Pro Glu Pro Leu Gly Gly Pro
   225                 230                 235                 240
   Ser Val Leu Ile Phe Pro Pro Lys Pro Lys Asp Ile Leu Arg Ile Thr
                   245                 250                 255
   Arg Thr Pro Glu Val Thr Cys Val Val Leu Asp Leu Gly Arg Glu Asp
               260                 265                 270
   Pro Glu Val Gln Ile Ser Trp Phe Val Asp Gly Lys Glu Val His Thr
           275                 280                 285
   Ala Lys Thr Gln Ser Arg Glu Gln Gln Phe Asn Gly Thr Tyr Arg Val
       290                 295                 300
   Val Ser Val Leu Pro Ile Glu His Gln Asp Trp Leu Thr Gly Lys Glu
   305                 310                 315                 320
   Phe Lys Cys Arg Val Asn His Ile Asp Leu Pro Ser Pro Ile Glu Arg
                   325                 330                 335
   Thr Ile Ser Lys Ala Arg Gly Arg Ala His Lys Pro Ser Val Tyr Val
               340                 345                 350
   Leu Pro Pro Ser Pro Lys Glu Leu Ser Ser Ser Asp Thr Val Ser Ile
           355                 360                 365
   Thr Cys Leu Ile Lys Asp Phe Tyr Pro Pro Asp Ile Asp Val Glu Trp
       370                 375                 380
   Gln Ser Asn Gly Gln Gln Glu Pro Glu Arg Lys His Arg Met Thr Pro
   385                 390                 395                 400
   Pro Gln Leu Asp Glu Asp Gly Ser Tyr Phe Leu Tyr Ser Lys Leu Ser
                   405                 410                 415
   Val Asp Lys Ser Arg Trp Gln Gln Gly Asp Pro Phe Thr Cys Ala Val
               420                 425                 430
   Met His Glu Thr Leu Gln Asn His Tyr Thr Asp Leu Ser Leu Ser His
           435                 440                 445
   Ser Pro Gly Lys
   450
<210> 19
   <211> 456
   <212> PRT
   <213> Artificial Sequence
<220>
   <223> complete heavy chain of a canine version of human D2E7 anti-TNF MAb - canine VH and canine heavy chain B constant
<400> 19
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Asp Asp Tyr
               20                  25                  30
   Ala Met His Trp Val Arg Gln Ala Pro Gly Lys Gly Leu Glu Trp Val
           35                  40                  45
   Ser Ala Ile Thr Trp Asn Ser Gly His Ile Asp Tyr Ala Asp Ser Val
       50                  55                  60
   Glu Gly Arg Phe Thr Ile Ser Arg Asp Asn Ala Lys Asn Ser Leu Tyr
   65                  70                  75                  80
   Leu Gln Met Asn Ser Leu Arg Ala Glu Asp Thr Ala Val Tyr Tyr Cys
                   85                  90                  95
   Ala Lys Val Ser Tyr Leu Ser Thr Ala Ser Ser Leu Asp Tyr Trp Gly
               100                 105                 110
   Gln Gly Thr Leu Val Thr Val Ser Ser Ala Ser Thr Thr Ala Pro Ser
           115                 120                 125
   Val Phe Pro Leu Ala Pro Ser Cys Gly Ser Thr Ser Gly Ser Thr Val
       130                 135                 140
   Ala Leu Ala Cys Leu Val Ser Gly Tyr Phe Pro Glu Pro Val Thr Val
   145                 150                 155                 160
   Ser Trp Asn Ser Gly Ser Leu Thr Ser Gly Val His Thr Phe Pro Ser
                   165                 170                 175
   Val Leu Gln Ser Ser Gly Leu Tyr Ser Leu Ser Ser Met Val Thr Val
               180                 185                 190
   Pro Ser Ser Arg Trp Pro Ser Glu Thr Phe Thr Cys Asn Val Ala His
               195                 200                 205
   Pro Ala Ser Lys Thr Lys Val Asp Lys Pro Val Pro Lys Arg Glu Asn
           210                 215                 220
   Gly Arg Val Pro Arg Pro Pro Asp Cys Pro Lys Cys Pro Ala Pro Glu
   225                 230                 235                 240
   Met Leu Gly Gly Pro Ser Val Phe Ile Phe Pro Pro Lys Pro Lys Asp
                   245                 250                 255
   Thr Leu Leu Ile Ala Arg Thr Pro Glu Val Thr Cys Val Val Val Asp
               260                 265                 270
   Leu Asp Pro Glu Asp Pro Glu Val Gln Ile Ser Trp Phe Val Asp Gly
           275                 280                 285
   Lys Gln Met Gln Thr Ala Lys Thr Gln Pro Arg Glu Glu Gln Phe Asn
       290                 295                 300
   Gly Thr Tyr Arg Val Val Ser Val Leu Pro Ile Gly His Gln Asp Trp
   305                 310                 315                 320
   Leu Lys Gly Lys Gln Phe Thr Cys Lys Val Asn Asn Lys Ala Leu Pro
                   325                 330                 335
   Ser Pro Ile Glu Arg Thr Ile Ser Lys Ala Arg Gly Gln Ala His Gln
               340                 345                 350
   Pro Ser Val Tyr Val Leu Pro Pro Ser Arg Glu Glu Leu Ser Lys Asn
           355                 360                 365
   Thr Val Ser Leu Thr Cys Leu Ile Lys Asp Phe Tyr Pro Pro Asp Ile
       370                 375                 380
   Asp Val Glu Trp Gln Ser Asn Gly Gln Gln Glu Pro Glu Ser Lys Tyr
   385                 390                 395                 400
   Arg Thr Thr Pro Pro Gln Leu Asp Glu Asp Gly Ser Tyr Phe Leu Tyr
                   405                 410                 415
   Ser Lys Leu Ser Val Asp Lys Ser Arg Trp Gln Arg Gly Asp Thr Phe
               420                 425                 430
   Ile Cys Ala Val Met His Glu Ala Leu His Asn His Tyr Thr Gln Glu
           435                 440                 445
   Ser Leu Ser His Ser Pro Gly Lys
   450 455
<210> 20
   <211> 454
   <212> PRT
   <213> Artificial Sequence
<220>
   <223> complete heavy chain of a canine version of human D2E7 anti-TNF MAb - canine VH and canine heavy chain C constant
<400> 20
   Glu Val Gln Leu Val Glu Ser Gly Gly Gly Leu Val Gln Pro Gly Gly
   1 5 10 15
   Ser Leu Arg Leu Ser Cys Ala Ala Ser Gly Phe Thr Phe Asp Asp Tyr
   20 25 30
   Ala Met His Trp Val Arg Gln Ala Pro Gly Lys Gly Leu Glu Trp Val
   35 40 45
   Ser Ala Ile Thr Trp Asn Ser Gly His Ile Asp Tyr Ala Asp Ser Val
   50 55 60
   Glu Gly Arg Phe Thr Ile Ser Arg Asp Asn Ala Lys Asn Ser Leu Tyr
   65 70 75 80
   Leu Gln Met Asn Ser Leu Arg Ala Glu Asp Thr Ala Val Tyr Tyr Cys
   85 90 95
   Ala Lys Val Ser Tyr Leu Ser Thr Ala Ser Ser Leu Asp Tyr Trp Gly
   100 105 110
   Gln Gly Thr Leu Val Thr Val Ser Ser Ala Ser Thr Thr Ala Pro Ser
   115 120 125
   Val Phe Pro Leu Ala Pro Ser Cys Gly Ser Gln Ser Gly Ser Thr Val
   130 135 140
   Ala Leu Ala Cys Leu Val Ser Gly Tyr Ile Pro Glu Pro Val Thr Val
   145 150 155 160
   Ser Trp Asn Ser Val Ser Leu Thr Ser Gly Val His Thr Phe Pro Ser
   165 170 175
   Val Leu Gln Ser Ser Gly Leu Tyr Ser Leu Ser Ser Met Val Thr Val
   180 185 190
   Pro Ser Ser Arg Trp Pro Ser Glu Thr Phe Thr Cys Asn Val Ala His
           195                 200                 205
   Pro Ala Thr Asn Thr Lys Val Asp Lys Pro Val Ala Lys Glu Cys Glu
       210                 215                 220
   Cys Lys Cys Asn Cys Asn Asn Cys Pro Cys Pro Gly Cys Gly Leu Leu
   225                 230                 235                 240
   Gly Gly Pro Ser Val Phe Ile Phe Pro Pro Lys Pro Lys Asp Ile Leu
                   245                 250                 255
   Val Thr Ala Arg Thr Pro Thr Val Thr Cys Val Val Val Asp Leu Asp
               260                 265                 270
   Pro Glu Asn Pro Glu Val Gln Ile Ser Trp Phe Val Asp Ser Lys Gln
           275                 280                 285
   Val Gln Thr Ala Asn Thr Gln Pro Arg Glu Glu Gln Ser Asn Gly Thr
       290                 295                 300
   Tyr Arg Val Val Ser Val Leu Pro Ile Gly His Gln Asp Trp Leu Ser
   305                 310                 315                 320
   Gly Lys Gln Phe Lys Cys Lys Val Asn Asn Lys Ala Leu Pro Ser Pro
                   325                 330                 335
   Ile Glu Glu Ile Ile Ser Lys Thr Pro Gly Gln Ala His Gln Pro Asn
               340                 345                 350
   Val Tyr Val Leu Pro Pro Ser Arg Asp Glu Met Ser Lys Asn Thr Val
           355                 360                 365
   Thr Leu Thr Cys Leu Val Lys Asp Phe Phe Pro Pro Glu Ile Asp Val
       370                 375                 380
   Glu Trp Gln Ser Asn Gly Gln Gln Glu Pro Glu Ser Lys Tyr Arg Met
   385                 390                 395                 400
   Thr Pro Pro Gln Leu Asp Glu Asp Gly Ser Tyr Phe Leu Tyr Ser Lys
                   405                 410                 415
   Leu Ser Val Asp Lys Ser Arg Trp Gln Arg Gly Asp Thr Phe Ile Cys
               420                 425                 430
   Ala Val Met His Glu Ala Leu His Asn His Tyr Thr Gln Ile Ser Leu
           435                 440                 445
   Ser His Ser Pro Gly Lys
   450
<210> 21
   <211> 452
   <212> PRT
   <213> Artificial Sequence
<220>
   <223> complete heavy chain of a canine version of human D2E7 anti-TNF MAb - canine VH and canine heavy chain D constant
<400> 21
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Asp Asp Tyr
               20                  25                  30
   Ala Met His Trp Val Arg Gln Ala Pro Gly Lys Gly Leu Glu Trp Val
           35                  40                  45
   Ser Ala Ile Thr Trp Asn Ser Gly His Ile Asp Tyr Ala Asp Ser Val
       50                  55                  60
   Glu Gly Arg Phe Thr Ile Ser Arg Asp Asn Ala Lys Asn Ser Leu Tyr
   65                  70                  75                  80
   Leu Gln Met Asn Ser Leu Arg Ala Glu Asp Thr Ala Val Tyr Tyr Cys
                   85                  90                  95
   Ala Lys Val Ser Tyr Leu Ser Thr Ala Ser Ser Leu Asp Tyr Trp Gly
               100                 105                 110
   Gln Gly Thr Leu Val Thr Val Ser Ser Ala Ser Thr Thr Ala Pro Ser
           115                 120                 125
   Val Phe Pro Leu Ala Pro Ser Cys Gly Ser Thr Ser Gly Ser Thr Val
       130                 135                 140
   Ala Leu Ala Cys Leu Val Ser Gly Tyr Phe Pro Glu Pro Val Thr Val
   145                 150                 155                 160
   Ser Trp Asn Ser Gly Ser Leu Thr Ser Gly Val His Thr Phe Pro Ser
                   165                 170                 175
   Val Leu Gln Ser Ser Gly Leu Tyr Ser Leu Ser Ser Thr Val Thr Val
               180                 185                 190
   Pro Ser Ser Arg Trp Pro Ser Glu Thr Phe Thr Cys Asn Val Val His
           195                 200                 205
   Pro Ala Ser Asn Thr Lys Val Asp Lys Pro Val Pro Lys Glu Ser Thr
       210                 215                 220
   Cys Lys Cys Ile Ser Pro Cys Pro Val Pro Glu Ser Leu Gly Gly Pro
   225                 230                 235                 240
   Ser Val Phe Ile Phe Pro Pro Lys Pro Lys Asp Ile Leu Arg Ile Thr
                   245                 250                 255
   Arg Thr Pro Glu Ile Thr Cys Val Val Leu Asp Leu Gly Arg Glu Asp
               260                 265                 270
   Pro Glu Val Gln Ile Ser Trp Phe Val Asp Gly Lys Glu Val His Thr
           275                 280                 285
   Ala Lys Thr Gln Pro Arg Glu Gln Gln Phe Asn Ser Thr Tyr Arg Val
       290                 295                 300
   Val Ser Val Leu Pro Ile Glu His Gln Asp Trp Leu Thr Gly Lys Glu
   305                 310                 315                 320
   Phe Lys Cys Arg Val Asn His Ile Gly Leu Pro Ser Pro Ile Glu Arg
                   325                 330                 335
   Thr Ile Ser Lys Ala Arg Gly Gln Ala His Gln Pro Ser Val Tyr Val
               340                 345                 350
   Leu Pro Pro Ser Pro Lys Glu Leu Ser Ser Ser Asp Thr Val Thr Leu
           355                 360                 365
   Thr Cys Leu Ile Lys Asp Phe Tyr Pro Pro Glu Ile Asp Val Glu Trp
       370                 375                 380
   Gln Ser Asn Gly Gln Pro Glu Pro Glu Ser Lys Tyr His Thr Thr Ala
   385                 390                 395                 400
   Pro Gln Leu Asp Glu Asp Gly Ser Tyr Phe Leu Tyr Ser Lys Leu Ser
                   405                 410                 415
   Val Asp Lys Ser Arg Trp Gln Gln Gly Asp Thr Phe Thr Cys Ala Val
               420                 425                 430
   Met His Glu Ala Leu Gln Asn His Tyr Thr Asp Leu Ser Leu Ser His
           435                 440                 445
   Ser Pro Gly Lys
   450
<210> 22
   <211> 217
   <212> PRT
   <213> Artificial Sequence
<220>
   <223> chimeric anti-TNF MAb light chain - human VK and canine kappa light chain
<400> 22
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Gly Ile Arg Asn Tyr
               20                  25                  30
   Leu Ala Trp Tyr Gln Gln Lys Pro Gly Lys Ala Pro Lys Leu Leu Ile
           35                  40                  45
   Tyr Ala Ala Ser Thr Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Val Ala Thr Tyr Tyr Cys Gln Arg Tyr Asn Arg Ala Pro Tyr
                   85                  90                  95
   Thr Phe Gly Gln Gly Thr Lys Val Glu Ile Lys Arg Asn Asp Ala Gln
               100                 105                 110
   Pro Ala Val Tyr Leu Phe Gln Pro Ser Pro Asp Gln Leu His Thr Gly
           115                 120                 125
   Ser Ala Ser Val Val Cys Leu Leu Asn Ser Phe Tyr Pro Lys Asp Ile
       130                 135                 140
   Asn Val Lys Trp Lys Val Asp Gly Val Ile Gln Asp Thr Gly Ile Gln
   145                 150                 155                 160
   Glu Ser Val Thr Glu Gln Asp Lys Asp Ser Thr Tyr Ser Leu Ser Ser
                   165                 170                 175
   Thr Leu Thr Met Ser Ser Thr Glu Tyr Leu Ser His Glu Leu Tyr Ser
               180                 185                 190
   Cys Glu Ile Thr His Lys Ser Leu Pro Ser Thr Leu Ile Lys Ser Phe
   195 200 205
   Gln Arg Ser Glu Cys Gln Arg Val Asp
   210 215
<210> 23
   <211> 456
   <212> PRT
   <213> Artificial Sequence
<220>
   <223> chimeric anti-TNF MAb heavy chain - human VH and canine heavy chain type B constant domain
<400> 23
   Glu Val Gln Leu Val Glu Ser Gly Gly Gly Leu Val Gln Pro Gly Arg
   1 5 10 15
   Ser Leu Arg Leu Ser Cys Ala Ala Ser Gly Phe Thr Phe Asp Asp Tyr
   20 25 30
   Ala Met His Trp Val Arg Gln Ala Pro Gly Lys Gly Leu Glu Trp Val
   35 40 45
   Ser Ala Ile Thr Trp Asn Ser Gly His Ile Asp Tyr Ala Asp Ser Val
   50 55 60
   Glu Gly Arg Phe Thr Ile Ser Arg Asp Asn Ala Lys Asn Ser Leu Tyr
   65 70 75 80
   Leu Gln Met Asn Ser Leu Arg Ala Glu Asp Thr Ala Val Tyr Tyr Cys
   85 90 95
   Ala Lys Val Ser Tyr Leu Ser Thr Ala Ser Ser Leu Asp Tyr Trp Gly
   100 105 110
   Gln Gly Thr Leu Val Thr Val Ser Ser Ala Ser Thr Thr Ala Pro Ser
   115 120 125
   Val Phe Pro Leu Ala Pro Ser Cys Gly Ser Thr Ser Gly Ser Thr Val
   130 135 140
   Ala Leu Ala Cys Leu Val Ser Gly Tyr Phe Pro Glu Pro Val Thr Val
   145 150 155 160
   Ser Trp Asn Ser Gly Ser Leu Thr Ser Gly Val His Thr Phe Pro Ser
   165 170 175
   Val Leu Gln Ser Ser Gly Leu Tyr Ser Leu Ser Ser Met Val Thr Val
   180 185 190
   Pro Ser Ser Arg Trp Pro Ser Glu Thr Phe Thr Cys Asn Val Ala His
   195 200 205
   Pro Ala Ser Lys Thr Lys Val Asp Lys Pro Val Pro Lys Arg Glu Asn
   210 215 220
   Gly Arg Val Pro Arg Pro Pro Asp Cys Pro Lys Cys Pro Ala Pro Glu
   225 230 235 240
   Met Leu Gly Gly Pro Ser Val Phe Ile Phe Pro Pro Lys Pro Lys Asp
   245 250 255
   Thr Leu Leu Ile Ala Arg Thr Pro Glu Val Thr Cys Val Val Val Asp
   260 265 270
   Leu Asp Pro Glu Asp Pro Glu Val Gln Ile Ser Trp Phe Val Asp Gly
   275 280 285
   Lys Gln Met Gln Thr Ala Lys Thr Gln Pro Arg Glu Glu Gln Phe Asn
   290 295 300
   Gly Thr Tyr Arg Val Val Ser Val Leu Pro Ile Gly His Gln Asp Trp
   305 310 315 320
   Leu Lys Gly Lys Gln Phe Thr Cys Lys Val Asn Asn Lys Ala Leu Pro
   325 330 335
   Ser Pro Ile Glu Arg Thr Ile Ser Lys Ala Arg Gly Gln Ala His Gln
   340 345 350
   Pro Ser Val Tyr Val Leu Pro Pro Ser Arg Glu Glu Leu Ser Lys Asn
   355 360 365
   Thr Val Ser Leu Thr Cys Leu Ile Lys Asp Phe Tyr Pro Pro Asp Ile
   370 375 380
   Asp Val Glu Trp Gln Ser Asn Gly Gln Gln Glu Pro Glu Ser Lys Tyr
   385 390 395 400
   Arg Thr Thr Pro Pro Gln Leu Asp Glu Asp Gly Ser Tyr Phe Leu Tyr
   405 410 415
   Ser Lys Leu Ser Val Asp Lys Ser Arg Trp Gln Arg Gly Asp Thr Phe
   420 425 430
   Ile Cys Ala Val Met His Glu Ala Leu His Asn His Tyr Thr Gln Glu
   435 440 445
   Ser Leu Ser His Ser Pro Gly Lys
   450 455
<210> 24
   <211> 468
   <212> PRT
   **<**213> Artificial Sequence
<220>
   <223> new Hu alpha D11 complete HC-IgG4
<400> 24
   Met Ala Val Leu Val Leu Leu Leu Cys Leu Val Thr Phe Pro Thr Cys
   1               5                   10                  15
   Val Leu Ser Gln Val Gln Leu Lys Glu Ser Gly Pro Gly Leu Val Gln
               20                  25                  30
   Pro Ser Gln Thr Leu Ser Leu Thr Cys Thr Val Ser Gly Phe Ser Leu
           35                  40                  45
   Thr Asn Asn Asn Val Asn Trp Val Arg Gln Ala Ser Gly Arg Gly Leu
       50                  55                  60
   Glu Trp Met Gly Gly Val Trp Ala Gly Gly Ala Thr Asp Tyr Asn Ser
   65                  70                  75                  80
   Ala Leu Lys Ser Arg Leu Thr Ile Thr Arg Asp Thr Ser Lys Ser Gln
                   85                  90                  95
   Val Phe Leu Lys Met His Ser Leu Gln Ser Glu Asp Thr Ala Thr Tyr
               100                 105                 110
   Tyr Cys Ala Arg Asp Gly Gly Tyr Ser Ser Ser Thr Leu Tyr Ala Met
           115                 120                 125
   Asp Ala Trp Gly Gln Gly Thr Thr Val Thr Val Ser Ser Ala Ser Thr
       130                 135                 140
   Lys Gly Pro Ser Val Phe Pro Leu Ala Pro Cys Ser Arg Ser Thr Ser
   145                 150                 155                 160
   Glu Ser Thr Ala Ala Leu Gly Cys Leu Val Lys Asp Tyr Phe Pro Glu
                   165                 170                 175
   Pro Val Thr Val Ser Trp Asn Ser Gly Ala Leu Thr Ser Gly Val His
               180                 185                 190
   Thr Phe Pro Ala Val Leu Gln Ser Ser Gly Leu Tyr Ser Leu Ser Ser
           195                 200                 205
   Val Val Thr Val Pro Ser Ser Ser Leu Gly Thr Lys Thr Tyr Thr Cys
       210                 215                 220
   Asn Val Asp His Lys Pro Ser Asn Thr Lys Val Asp Lys Arg Val Glu
   225                 230                 235                 240
   Ser Lys Tyr Gly Pro Pro Cys Pro Pro Cys Pro Ala Pro Glu Phe Leu
                   245                 250                 255
   Gly Gly Pro Ser Val Phe Leu Phe Pro Pro Lys Pro Lys Asp Thr Leu
               260                 265                 270
   Met Ile Ser Arg Thr Pro Glu Val Thr Cys Val Val Val Asp Val Ser
           275                 280                 285
   Gln Glu Asp Pro Glu Val Gln Phe Asn Trp Tyr Val Asp Gly Val Glu
       290                 295                 300
   Val His Asn Ala Lys Thr Lys Pro Arg Glu Glu Gln Phe Asn Ser Thr
   305                 310                 315                 320
   Tyr Arg Val Val Ser Val Leu Thr Val Leu His Gln Asp Trp Leu Asn
                   325                 330                 335
   Gly Lys Glu Tyr Lys Cys Lys Val Ser Asn Lys Gly Leu Pro Ser Ser
               340                 345                 350
   Ile Glu Lys Thr Ile Ser Lys Ala Lys Gly Gln Pro Arg Glu Pro Gln
           355                 360                 365
   Val Tyr Thr Leu Pro Pro Ser Gln Glu Glu Met Thr Lys Asn Gln Val
       370                 375                 380
   Ser Leu Thr Cys Leu Val Lys Gly Phe Tyr Pro Ser Asp Ile Ala Val
   385                 390                 395                 400
   Glu Trp Glu Ser Asn Gly Gln Pro Glu Asn Asn Tyr Lys Thr Thr Pro
                   405                 410                 415
   Pro Val Leu Asp Ser Asp Gly Ser Phe Phe Leu Tyr Ser Arg Leu Thr
   420 425 430
   Val Asp Lys Ser Arg Trp Gln Glu Gly Asn Val Phe Ser Cys Ser Val
   435 440 445
   Met His Glu Ala Leu His Asn His Tyr Thr Gln Lys Ser Leu Ser Leu
   450 455 460
   Ser Leu Gly Lys
   465
<210> 25
   <211> 234
   <212> PRT
   <213> Artificial Sequence
<220>
   <223> new Hu alpha D11 complete kappa LC
<400> 25
   Met Gly Val Pro Thr Gln Leu Leu Gly Leu Leu Leu Leu Trp Ile Thr
   1               5                   10                  15
   Asp Ala Ile Cys Asp Ile Gln Met Thr Gln Ser Pro Ala Ser Leu Ser
               20                  25                  30
   Ala Ser Leu Gly Glu Thr Val Thr Ile Asn Cys Arg Ala Ser Glu Asp
           35                  40                  45
   Ile Tyr Asn Ala Leu Ala Trp Tyr Gln Gln Lys Pro Gly Lys Ser Pro
       50                  55                  60
   Gln Leu Leu Ile Tyr Asn Thr Asp Thr Leu His Thr Gly Val Pro Ser
   65                  70                  75                  80
   Arg Phe Ser Gly Ser Gly Ser Gly Thr Glu Tyr Ser Leu Lys Ile Asn
                   85                  90                  95
   Ser Leu Gln Ser Glu Asp Val Ala Ser Tyr Phe Cys Gln His Tyr Phe
               100                 105                 110
   His Tyr Pro Arg Thr Phe Gly Gly Gly Thr Lys Leu Glu Ile Lys Arg
           115                 120                 125
   Thr Val Ala Ala Pro Ser Val Phe Ile Phe Pro Pro Ser Asp Glu Gln
       130                 135                 140
   Leu Lys Ser Gly Thr Ala Ser Val Val Cys Leu Leu Asn Asn Phe Tyr
   145                 150                 155                 160
   Pro Arg Glu Ala Lys Val Gln Trp Lys Val Asp Asn Ala Leu Gln Ser
                   165                 170                 175
   Gly Asn Ser Gln Glu Ser Val Thr Glu Gln Asp Ser Lys Asp Ser Thr
               180                 185                 190
   Tyr Ser Leu Ser Ser Thr Leu Thr Leu Ser Lys Ala Asp Tyr Glu Lys
           195                 200                 205
   His Lys Val Tyr Ala Cys Glu Val Thr His Gln Gly Leu Ser Ser Pro
       210                 215                 220
   Val Thr Lys Ser Phe Asn Arg Gly Glu Cys
   225                 230
<210> 26
   <211> 480
   <212> PRT
   <213> Artificial Sequence
<220>
   <223> ca148-HCB heavy chain
<400> 26
   Met Gly Phe Gly Leu Ser Trp Val Phe Leu Val Ala Leu Leu Arg Gly
   1               5                   10                  15
   Val Gln Cys Glu Val Gln Leu Val Glu Ser Gly Gly Gly Val Val Gln
               20                  25                  30
   Pro Gly Gly Ser Leu Arg Leu Ser Cys Ala Ala Ser Gly Phe Ile Phe
           35                  40                  45
   Ser Ser Tyr Ala Met Arg Trp Val Arg Gln Ala Pro Gly Lys Gly Leu
       50                  55                  60
   Glu Trp Val Ala Phe Met Ser Tyr Asp Gly Ser Asn Lys Lys Tyr Ala
   65                  70                  75                  80
   Asp Ser Val Lys Gly Arg Phe Thr Ile Ser Arg Asp Asn Ser Lys Asn
                   85                  90                  95
   Thr Leu Tyr Leu Gln Met Asn Ser Leu Arg Ala Glu Asp Thr Ala Val
               100                 105                 110
   Tyr Tyr Cys Ala Arg Asp Arg Gly Ile Ala Ala Gly Gly Asn Tyr Tyr
           115                 120                 125
   Tyr Tyr Gly Met Asp Val Trp Gly Gln Gly Thr Ser Val Thr Val Ser
       130                 135                 140
   Ser Ala Ser Thr Thr Ala Pro Ser Val Phe Pro Leu Ala Pro Ser Cys
   145                 150                 155                 160
   Gly Ser Thr Ser Gly Ser Thr Val Ala Leu Ala Cys Leu Val Ser Gly
                   165                 170                 175
   Tyr Phe Pro Glu Pro Val Thr Val Ser Trp Asn Ser Gly Ser Leu Thr
               180                 185                 190
   Ser Gly Val His Thr Phe Pro Ser Val Leu Gln Ser Ser Gly Leu Tyr
           195                 200                 205
   Ser Leu Ser Ser Met Val Thr Val Pro Ser Ser Arg Trp Pro Ser Glu
       210                 215                 220
   Thr Phe Thr Cys Asn Val Ala His Pro Ala Ser Lys Thr Lys Val Asp
   225                 230                 235                 240
   Lys Pro Val Pro Lys Arg Glu Asn Gly Arg Val Pro Arg Pro Pro Asp
                   245                 250                 255
   Cys Pro Lys Cys Pro Ala Pro Glu Met Leu Gly Gly Pro Ser Val Phe
               260                 265                 270
   Ile Phe Pro Pro Lys Pro Lys Asp Thr Leu Leu Ile Ala Arg Thr Pro
           275                 280                 285
   Glu Val Thr Cys Val Val Val Asp Leu Asp Pro Glu Asp Pro Glu Val
       290                 295                 300
   Gln Ile Ser Trp Phe Val Asp Gly Lys Gln Met Gln Thr Ala Lys Thr
   305                 310                 315                 320
   Gln Pro Arg Glu Glu Gln Phe Asn Gly Thr Tyr Arg Val Val Ser Val
                   325                 330                 335
   Leu Pro Ile Gly His Gln Asp Trp Leu Lys Gly Lys Gln Phe Thr Cys
               340                 345                 350
   Lys Val Asn Asn Lys Ala Leu Pro Ser Pro Ile Glu Arg Thr Ile Ser
           355                 360                 365
   Lys Ala Arg Gly Gln Ala His Gln Pro Ser Val Tyr Val Leu Pro Pro
       370                 375                 380
   Ser Arg Glu Glu Leu Ser Lys Asn Thr Val Ser Leu Thr Cys Leu Ile
   385                 390                 395                 400
   Lys Asp Phe Tyr Pro Pro Asp Ile Asp Val Glu Trp Gln Ser Asn Gly
                   405                 410                 415
   Gln Gln Glu Pro Glu Ser Lys Tyr Arg Thr Thr Pro Pro Gln Leu Asp
               420                 425                 430
   Glu Asp Gly Ser Tyr Phe Leu Tyr Ser Lys Leu Ser Val Asp Lys Ser
           435                 440                 445
   Arg Trp Gln Arg Gly Asp Thr Phe Ile Cys Ala Val Met His Glu Ala
       450                 455                 460
   Leu His Asn His Tyr Thr Gln Glu Ser Leu Ser His Ser Pro Gly Lys
   465                 470                 475                 480
<210> 27
   <211> 238
   <212> PRT
   <213> Artificial Sequence
<220>
   <223> ca148-kLC light chain
<400> 27
   Met Glu Ala Pro Ala Gln Leu Leu Phe Leu Leu Leu Leu Trp Leu Pro
   1               5                   10                  15
   Asp Thr Thr Gly Glu Ile Val Met Thr Gln Ser Pro Ala Ser Leu Ser
               20                  25                  30
   Leu Ser Pro Gly Glu Lys Ala Thr Ile Ser Cys Arg Ala Ser Gln Ser
           35                  40                  45
   Val Tyr Ser Tyr Leu Ala Trp Tyr Gln Gln Lys Pro Gly Gln Ala Pro
       50                  55                  60
   Arg Leu Leu Ile Tyr Asp Ala Ser Asn Arg Ala Thr Gly Val Pro Ser
   65                  70                  75                  80
   Arg Phe Ser Gly Ser Gly Ser Gly Thr Asp Phe Thr Leu Thr Ile Ser
                   85                  90                  95
   Ser Leu Glu Pro Glu Asp Val Ala Val Tyr Tyr Cys Gln Gln Pro Ser
               100                 105                 110
   Asn Trp Pro Pro Phe Thr Phe Gly Pro Gly Thr Lys Val Asp Ile Lys
   115 120 125
   Arg Asn Asp Ala Gln Pro Ala Val Tyr Leu Phe Gln Pro Ser Pro Asp
   130 135 140
   Gln Leu His Thr Gly Ser Ala Ser Val Val Cys Leu Leu Asn Ser Phe
   145 150 155 160
   Tyr Pro Lys Asp Ile Asn Val Lys Trp Lys Val Asp Gly Val Ile Gln
   165 170 175
   Asp Thr Gly Ile Gln Glu Ser Val Thr Glu Gln Asp Lys Asp Ser Thr
   180 185 190
   Tyr Ser Leu Ser Ser Thr Leu Thr Met Ser Ser Thr Glu Tyr Leu Ser
   195 200 205
   His Glu Leu Tyr Ser Cys Glu Ile Thr His Lys Ser Leu Pro Ser Thr
   210 215 220
   Leu Ile Lys Ser Phe Gln Arg Ser Glu Cys Gln Arg Val Asp
   225 230 235
<210> 28
   <211> 23
   <212> PRT
   <213> Artificial Sequence
<220>
   <223> rat alpha D11 FR1 region of VK light chain
<400> 28
   Asp Ile Gln Met Thr Gln Ser Pro Ala Ser Leu Ser Ala Ser Leu Gly
   1 5 10 15
   Glu Thr Val Thr Ile Glu Cys
   20
<210> 29
   <211> 23
   <212> PRT
   <213> Artificial Sequence
<220>
   <223> caninised FR1 region of VK light chain of rat alpha D11 antibody
<400> 29
   Asp Ile Val Met Thr Gln Ser Pro Ala Ser Leu Ser Leu Ser Gln Glu
   1               5                   10                  15
   Glu Lys Val Thr Ile Thr Cys
               20
<210> 30
   <211> 23
   <212> PRT
   <213> Artificial Sequence
<220>
   <223> felinised FR1 region of VK light chain of rat alpha D11 antibody
<400> 30
   Asp Ile Val Met Thr Gln Thr Pro Leu Ser Leu Ser Val Thr Pro Gly
   1               5                   10                  15
   Glu Pro Ala Ser Ile Ser Cys
               20
<210> 31
   <211> 23
   <212> PRT
   <213> Artificial Sequence
<220>
   <223> equinised FR1 region of VK light chain of rat alpha D11 antibody
<400> 31
   Asp Ile Val Met Thr Gln Ser Pro Ala Ser Leu Ser Ala Ser Leu Gly
   1               5                   10                  15
   Glu Thr Val Thr Ile Glu Cys
               20
<210> 32
   <211> 15
   <212> PRT
   <213> Artificial Sequence
<220>
   <223> rat alpha D11 FR2 region of VK light chain
<400> 32
   Trp Tyr Gln Gln Lys Pro Gly Lys Ser Pro Gln Leu Leu Ile Tyr
   1               5                   10                  15
<210> 33
   <211> 15
   <212> PRT
   <213> Artificial Sequence
<220>
   <223> caninised FR2 region of VK light chain of rat alpha D11 antibody
<400> 33
   Trp Tyr Gln Gln Lys Pro Gly Gln Ala Pro Lys Leu Leu Ile Tyr
   1               5                   10                  15
<210> 34
   <211> 15
   <212> PRT
   <213> Artificial Sequence
<220>
   <223> felinised FR2 region of VK light chain of rat alpha D11 antibody
<400> 34
   Trp Tyr Leu Gln Lys Pro Gly Gln Ser Pro Arg Arg Leu Ile Tyr
   1               5                   10                  15
<210> 35
   <211> 15
   <212> PRT
   <213> Artificial Sequence
<220>
   <223> equinised FR2 region of VK light chain of rat alpha D11 antibody
<400> 35
   Trp Tyr Gln Gln Lys Pro Gly Gln Ser Pro Lys Leu Leu Ile Tyr
   1               5                   10                  15
<210> 36
   <211> 32
   <212> PRT
   <213> Artificial Sequence
<220>
   <223> rat alpha D11 FR3 region of VK light chain
<400> 36
   Gly Val Pro Ser Arg Phe Ser Gly Ser Gly Ser Gly Thr Gln Tyr Ser
   1               5                   10                  15
   Leu Lys Ile Asn Ser Leu Gln Ser Glu Asp Val Ala Ser Tyr Phe Cys
               20                  25                  30
<210> 37
   <211> 32
   <212> PRT
   <213> Artificial Sequence
<220>
   <223> caninised FR3 region of VK light chain of rat alpha D11 antibody
<400> 37
   Gly Val Pro Ser Arg Phe Ser Gly Ser Gly Ser Gly Thr Glu Phe Ser
   1               5                   10                  15
   Leu Thr Ile Ser Ser Leu Glu Pro Glu Asp Val Ala Val Tyr Tyr Cys
               20                  25                  30
<210> 38
   <211> 32
   <212> PRT
   <213> Artificial Sequence
<220>
   <223> felinised FR3 region of VK light chain of rat alpha D11 antibody
<400> 38
   Gly Val Pro Asp Arg Phe Ser Gly Ser Gly Ser Gly Thr Asp Phe Thr
   1               5                   10                  15
   Leu Arg Ile Ser Arg Val Glu Ala Asp Asp Val Gly Val Tyr Phe Cys
               20                  25                  30
<210> 39
   <211> 32
   <212> PRT
   <213> Artificial Sequence
<220>
   <223> equinised FR3 region of VK light chain of rat alpha D11 antibody
<400> 39
   Gly Val Pro Ser Arg Phe Ser Gly Ser Gly Ser Gly Thr Asp Tyr Ser
   1               5                   10                  15
   Leu Thr Ile Asn Ser Leu Gln Ser Glu Asp Val Ala Ser Tyr Phe Cys
               20                  25                  30
<210> 40
   <211> 10
   <212> PRT
   <213> Artificial Sequence
<220>
   <223> rat alpha D11 FR4 region of VK light chain
<400> 40
   Phe Gly Gly Gly Thr Lys Leu Glu Leu Lys
   1               5                   10
<210> 41
   <211> 10
   <212> PRT
   <213> Artificial Sequence
<220>
   <223> caninised FR4 region of VK light chain of rat alpha D11 antibody
<400> 41
   Phe Gly Ala Gly Thr Lys Val Glu Leu Lys
   1               5                   10
<210> 42
   <211> 10
   <212> PRT
   <213> Artificial Sequence
<220>
   <223> felinised FR4 region of VK light chain of rat alpha D11 antibody
<400> 42
   Phe Gly Pro Gly Thr Lys Leu Glu Ile Lys
   1               5                   10
<210> 43
   <211> 10
   <212> PRT
   <213> Artificial Sequence
<220>
   <223> equinised FR4 region of VK light chain of rat alpha D11 antibody
<400> 43
   Phe Gly Gln Gly Thr Lys Leu Glu Leu Lys
   1               5                   10
<210> 44
   <211> 27
   <212> PRT
   <213> Artificial Sequence
<220>
   <223> rat alpha D11 FR1 region of heavy chain
<400> 44
   Gln Val Gln Leu Lys Glu Ser Gly Pro Gly Leu Val Gln Pro Ser Gln
   1               5                   10                  15
   Thr Leu Ser Leu Thr Cys Thr Val Ser Gly Phe
               20                  25
<210> 45
   <211> 27
   <212> PRT
   <213> Artificial Sequence
<220>
   <223> caninised FR1 region of heavy chain of rat alpha D11 antibody
<400> 45
   Glu Val Gln Leu Val Glu Ser Gly Gly Asp Leu Val Asn Pro Gly Gly
   1               5                   10                  15
   Thr Leu Thr Leu Ser Cys Val Val Ser Gly Phe
               20                  25
<210> 46
   <211> 27
   <212> PRT
   <213> Artificial Sequence
<220>
   <223> felinised FR1 region of heavy chain of rat alpha D11 antibody
<400> 46
   Gln Val Gln Leu Val Glu Ser Gly Gly Asp Leu Val Gln Pro Gly Gly
   1               5                   10                  15
   Ser Leu Arg Leu Thr Cys Ala Ala Ser Gly Phe
               20                  25
<210> 47
   <211> 27
   <212> PRT
   <213> Artificial Sequence
<220>
   <223> equinised FR1 region of heavy chain of rat alpha D11 antibody
<400> 47
   Gln Val Gln Leu Lys Glu Ser Gly Pro Gly Leu Val Asn Pro Ser Gln
   1               5                   10                  15
   Thr Leu Ser Leu Thr Cys Thr Val Ser Gly Phe
               20                  25
<210> 48
   <211> 14
   <212> PRT
   <213> Artificial Sequence
<220>
   <223> rat alpha D11 FR2 region of heavy chain
<400> 48
   Trp Val Arg Gln Ala Thr Gly Arg Gly Leu Glu Trp Met Gly
   1               5                   10
<210> 49
   <211> 14
   <212> **PRT**
   <213> Artificial Sequence
<220>
   <223> caninised FR2 region of heavy chain of rat alpha D11 antibody
<400> 49
   Trp Val Arg Gln Ala Leu Gly Arg Gly Leu Glu Trp Val Gly
   1               5                   10
<210> 50
   <211> 14
   <212> PRT
   <213> Artificial Sequence
<220>
   <223> felinised FR2 region of heavy chain of rat alpha D11 antibody
<400> 50
   Trp Val Arg Gln Ala Pro Gly Lys Gly Leu Glu Trp Met Gly
   1               5                   10
<210> 51
   <211> 14
   <212> PRT
   <213> Artificial Sequence
<220>
   <223> equinised FR2 region of heavy chain of rat alpha D11 antibody
<400> 51
   Trp Val Arg Gln Ala Pro Gly Lys Gly Leu Glu Trp Val Gly
   1               5                   10
<210> 52
   <211> 32
   <212> PRT
   <213> Artificial Sequence
<220>
   <223> rat alpha D11 FR3 region of heavy chain
<400> 52
   Arg Leu Thr Ile Thr Arg Asp Thr Ser Lys Ser Gln Val Phe Leu Lys
   1               5                   10                  15
   Met His Ser Leu Gln Ser Glu Asp Thr Ala Thr Tyr Tyr Cys Ala Arg
               20                  25                  30
<210> 53
   <211> 32
   <212> PRT
   <213> Artificial Sequence
<220>
   <223> caninised FR3 region of heavy chain of rat alpha D11 antibody
<400> 53
   Arg Leu Thr Ile Thr Arg Asp Thr Ser Lys Ser Thr Val Phe Leu Gln
   1               5                   10                  15
   Met His Ser Leu Arg Ser Glu Asp Thr Ala Thr Tyr Tyr Cys Ala Arg
               20                  25                  30
<210> 54
   <211> 32
   <212> PRT
   <213> Artificial Sequence
<220>
   <223> felinised FR3 region of heavy chain of rat alpha D11 antibody
<400> 54
   Arg Phe Thr Ile Ser Arg Asp Asn Ala Lys Asn Thr Leu Tyr Leu Gln
   1               5                   10                  15
   Met Asn Ser Leu Lys Thr Glu Asp Thr Ala Thr Tyr Tyr Cys Ala Arg
               20                  25                  30
<210> 55
   <211> 32
   <212> PRT
   <213> Artificial Sequence
<220>
   <223> equinised FR3 region of heavy chain of rat alpha D11 antibody
<400> 55
   Arg Ala Thr Ile Thr Arg Asp Thr Ser Lys Ser Gln Val Phe Leu Gln
   1               5                   10                  15
   Met Asn Ser Leu Thr Ser Glu Asp Thr Ala Val Tyr Tyr Cys Ala Arg
               20                  25                  30
<210> 56
   <211> 11
   <212> PRT
   <213> Artificial Sequence
<220>
   <223> rat alpha D11 FR4 region of heavy chain
<400> 56
   Trp Gly Gln Gly Thr Thr Val Thr Val Ser Ser
   1 5 10
<210> 57
   <211> 11
   <212> PRT
   <213> Artificial Sequence
<220>
   <223> caninised FR4 region of heavy chain of rat alpha D11 antibody
<400> 57
   Trp Gly Gln Gly Thr Leu Val Thr Val Ser Ser
   1 5 10
<210> 58
   <211> 11
   <212> PRT
   <213> Artificial Sequence
<220>
   <223> felinised FR4 region of heavy chain of rat alpha D11 antibody
<400> 58
   Trp Gly Gln Gly Thr Leu Val Thr Val Ser Ser
   1 5 10
<210> 59
   <211> 11
   <212> PRT
   <213> Artificial Sequence
<220>
   <223> equinised FR4 region of heavy chain of rat alpha D11 antibody
<400> 59
   Trp Gly Gln Gly Ile Leu Val Thr Val Ser Ser
   1 5 10
<210> 60
   <211> 23
   <212> PRT
   <213> Artificial Sequence
<220>
   <223> FR1 region of light chain of a novel humanised variant of alpha D11 (new Hu)
<400> 60
   Asp Ile Gln Met Thr Gln Ser Pro Ala Ser Leu Ser Ala Ser Leu Gly
   1 5 10 15
   Glu Thr Val Thr Ile Asn Cys
   20
<210> 61
   <211> 15
   <212> **PRT**
   <213> Artificial Sequence
<220>
   <223> FR2 region of light chain of a novel humanised variant of alpha D11 (new Hu)
<400> 61
   Trp Tyr Gln Gln Lys Pro Gly Lys Ser Pro Gln Leu Leu Ile Tyr
   1               5                   10                  15
<210> 62
   <211> 32
   <212> PRT
   <213> Artificial Sequence
<220>
   <223> FR3 region of light chain of a novel humanised variant of alpha D11 (new Hu)
<400> 62
   Gly Val Pro Ser Arg Phe Ser Gly Ser Gly Ser Gly Thr Glu Tyr Ser
   1               5                   10                  15
   Leu Lys Ile Asn Ser Leu Gln Ser Glu Asp Val Ala Ser Tyr Phe Cys
               20                  25                  30
<210> 63
   <211> 10
   <212> PRT
   <213> Artificial Sequence
<220>
   <223> FR4 region of light chain of a novel humanised variant of alpha D11 (new Hu)
<400> 63
   Phe Gly Gly Gly Thr Lys Leu Glu Ile Lys
   1               5                   10
<210> 64
   <211> 22
   <212> PRT
   <213> Artificial Sequence
<220>
   <223> FR1 region of heavy chain of a novel humanised variant of alpha D11 (new Hu)
<400> 64
   Gln Val Gln Leu Lys Glu Ser Gly Pro Gly Leu Val Gln Pro Ser Gln
   1               5                   10                  15
   Thr Leu Ser Leu Thr Cys
               20
<210> 65
   <211> 14
   <212> PRT
   <213> Artificial Sequence
<220>
   <223> FR2 region of heavy chain of a novel humanised variant of alpha D11 (new Hu)
<400> 65
   Trp Val Arg Gln Ala Ser Gly Arg Gly Leu Glu Trp Met Gly
   1               5                   10
<210> 66
   <211> 32
   <212> PRT
   <213> Artificial Sequence
<220>
   <223> FR3 region of heavy chain of a novel humanised variant of alpha D11 (new Hu)
<400> 66
   Arg Leu Thr Ile Thr Arg Asp Thr Ser Lys Ser Gln Val Phe Leu Lys
   1               5                   10                  15
   Met His Ser Leu Gln Ser Glu Asp Thr Ala Thr Tyr Tyr Cys Ala Arg
               20                  25                  30
<210> 67
   <211> 11
   <212> PRT
   <213> Artificial Sequence
<220>
   <223> FR4 region of heavy chain of a novel humanised variant of alpha D11 (new Hu)
<400> 67
   Trp Gly Gln Gly Thr Thr Val Thr Val Ser Ser
   1               5                   10
<210> 68
   <211> 453
   <212> PRT
   <213> Artificial Sequence
<220>
   <223> complete heavy chain of a caninised version of alpha D11 anti-NGF MAb - VH and HCA
<400> 68
   Glu Val Gln Leu Val Glu Ser Gly Gly Asp Leu Val Asn Pro Gly Gly
   1               5                   10                  15
   Thr Leu Thr Leu Ser Cys Val Val Ser Gly Phe Ser Leu Thr Asn Asn
               20                  25                  30
   Asn Val Asn Trp Val Arg Gln Ala Leu Gly Arg Gly Leu Glu Trp Val
           35                  40                  45
   Gly Gly Val Trp Ala Gly Gly Ala Thr Asp Tyr Asn Ser Ala Leu Lys
       50                  55                  60
   Ser Arg Leu Thr Ile Thr Arg Asp Thr Ser Lys Ser Thr Val Phe Leu
   65                  70                  75                  80
   Lys Met His Ser Leu Gln Ser Glu Asp Thr Ala Thr Tyr Tyr Cys Ala
                   85                  90                  95
   Arg Asp Gly Gly Tyr Ser Ser Ser Thr Leu Tyr Ala Met Asp Ala Trp
               100                 105                 110
   Gly Gln Gly Thr Leu Val Thr Val Ser Ser Ala Ser Thr Thr Ala Pro
           115                 120                 125
   Ser Val Phe Pro Leu Ala Pro Ser Cys Gly Ser Thr Ser Gly Ser Thr
       130                 135                 140
   Val Ala Leu Ala Cys Leu Val Ser Gly Tyr Phe Pro Glu Pro Val Thr
   145                 150                 155                 160
   Val Ser Trp Asn Ser Gly Ser Leu Thr Ser Gly Val His Thr Phe Pro
                   165                 170                 175
   Ser Val Leu Gln Ser Ser Gly Leu His Ser Leu Ser Ser Met Val Thr
               180                 185                 190
   Val Pro Ser Ser Arg Trp Pro Ser Glu Thr Phe Thr Cys Asn Val Val
           195                 200                 205
   His Pro Ala Ser Asn Thr Lys Val Asp Lys Pro Val Phe Asn Glu Cys
       210                 215                 220
   Arg Cys Thr Asp Thr Pro Pro Cys Pro Val Pro Glu Pro Leu Gly Gly
   225                 230                 235                 240
   Pro Ser Val Leu Ile Phe Pro Pro Lys Pro Lys Asp Ile Leu Arg Ile
                   245                 250                 255
   Thr Arg Thr Pro Glu Val Thr Cys Val Val Leu Asp Leu Gly Arg Glu
               260                 265                 270
   Asp Pro Glu Val Gln Ile Ser Trp Phe Val Asp Gly Lys Glu Val His
           275                 280                 285
   Thr Ala Lys Thr Gln Ser Arg Glu Gln Gln Phe Asn Gly Thr Tyr Arg
       290                 295                 300
   Val Val Ser Val Leu Pro Ile Glu His Gln Asp Trp Leu Thr Gly Lys
   305                 310                 315                 320
   Glu Phe Lys Cys Arg Val Asn His Ile Asp Leu Pro Ser Pro Ile Glu
                   325                 330                 335
   Arg Thr Ile Ser Lys Ala Arg Gly Arg Ala His Lys Pro Ser Val Tyr
               340                 345                 350
   Val Leu Pro Pro Ser Pro Lys Glu Leu Ser Ser Ser Asp Thr Val Ser
           355                 360                 365
   Ile Thr Cys Leu Ile Lys Asp Phe Tyr Pro Pro Asp Ile Asp Val Glu
       370                 375                 380
   Trp Gln Ser Asn Gly Gln Gln Glu Pro Glu Arg Lys His Arg Met Thr
   385                 390                 395                 400
   Pro Pro Gln Leu Asp Glu Asp Gly Ser Tyr Phe Leu Tyr Ser Lys Leu
                   405                 410                 415
   Ser Val Asp Lys Ser Arg Trp Gln Gln Gly Asp Pro Phe Thr Cys Ala
               420                 425                 430
   Val Met His Glu Thr Leu Gln Asn His Tyr Thr Asp Leu Ser Leu Ser
           435                 440                 445
   His Ser Pro Gly Lys
       450
<210> 69
   <211> 122
   <212> PRT
   <213> Artificial Sequence
<220>
   <223> heavy chain variable domain of an alternative canine version of alpha D11 anti-NGF MAb - canine VH
<400> 69
   Glu Val Gln Leu Val Glu Ser Gly Gly Gly Leu Val Gln Pro Gly Gly
   1               5                   10                  15
   Ser Leu Arg Leu Ser Cys Val Ala Ser Gly Phe Ser Leu Thr Asn Asn
               20                  25                  30
   Asn Val Asn Trp Val Arg Gln Ala Pro Gly Lys Gly Leu Glu Trp Val
           35                  40                  45
   Gly Gly Val Trp Ala Gly Gly Ala Thr Asp Tyr Asn Ser Ala Leu Lys
       50                  55                  60
   Ser Arg Phe Thr Ile Ser Arg Asp Asn Ala Lys Asn Thr Val Phe Leu
   65                  70                  75                  80
   Gln Met His Ser Leu Arg Ser Glu Asp Thr Ala Val Tyr Tyr Cys Ala
                   85                  90                  95
   Arg Asp Gly Gly Tyr Ser Ser Ser Thr Leu Tyr Ala Met Asp Ala Trp
               100                 105                 110
   Gly Gln Gly Thr Ser Val Thr Val Ser Ser
           115                 120
<210> 70
   <211> 453
   <212> PRT
   <213> Artificial Sequence
<220>
   <223> complete heavy chain of an alternative caninised version of alpha D11 anti-NGF MAb - VH and HCA
<400> 70
   Glu Val Gln Leu Val Glu Ser Gly Gly Gly Leu Val Gln Pro Gly Gly
   1               5                   10                  15
   Ser Leu Arg Leu Ser Cys Val Ala Ser Gly Phe Ser Leu Thr Asn Asn
               20                  25                  30
   Asn Val Asn Trp Val Arg Gln Ala Pro Gly Lys Gly Leu Glu Trp Val
           35                  40                  45
   Gly Gly Val Trp Ala Gly Gly Ala Thr Asp Tyr Asn Ser Ala Leu Lys
       50                  55                  60
   Ser Arg Phe Thr Ile Ser Arg Asp Asn Ala Lys Asn Thr Val Phe Leu
   65                  70                  75                  80
   Gln Met His Ser Leu Arg Ser Glu Asp Thr Ala Val Tyr Tyr Cys Ala
                   85                  90                  95
   Arg Asp Gly Gly Tyr Ser Ser Ser Thr Leu Tyr Ala Met Asp Ala Trp
               100                 105                 110
   Gly Gln Gly Thr Ser Val Thr Val Ser Ser Ala Ser Thr Thr Ala Pro
           115                 120                 125
   Ser Val Phe Pro Leu Ala Pro Ser Cys Gly Ser Thr Ser Gly Ser Thr
       130                 135                 140
   Val Ala Leu Ala Cys Leu Val Ser Gly Tyr Phe Pro Glu Pro Val Thr
   145                 150                 155                 160
   Val Ser Trp Asn Ser Gly Ser Leu Thr Ser Gly Val His Thr Phe Pro
                   165                 170                 175
   Ser Val Leu Gln Ser Ser Gly Leu His Ser Leu Ser Ser Met Val Thr
               180                 185                 190
   Val Pro Ser Ser Arg Trp Pro Ser Glu Thr Phe Thr Cys Asn Val Val
           195                 200                 205
   His Pro Ala Ser Asn Thr Lys Val Asp Lys Pro Val Phe Asn Glu Cys
       210                 215                 220
   Arg Cys Thr Asp Thr Pro Pro Cys Pro Val Pro Glu Pro Leu Gly Gly
   225                 230                 235                 240
   Pro Ser Val Leu Ile Phe Pro Pro Lys Pro Lys Asp Ile Leu Arg Ile
                   245                 250                 255
   Thr Arg Thr Pro Glu Val Thr Cys Val Val Leu Asp Leu Gly Arg Glu
               260                 265                 270
   Asp Pro Glu Val Gln Ile Ser Trp Phe Val Asp Gly Lys Glu Val His
           275                 280                 285
   Thr Ala Lys Thr Gln Ser Arg Glu Gln Gln Phe Asn Gly Thr Tyr Arg
       290                 295                 300
   Val Val Ser Val Leu Pro Ile Glu His Gln Asp Trp Leu Thr Gly Lys
   305                 310                 315                 320
   Glu Phe Lys Cys Arg Val Asn His Ile Asp Leu Pro Ser Pro Ile Glu
                   325                 330                 335
   Arg Thr Ile Ser Lys Ala Arg Gly Arg Ala His Lys Pro Ser Val Tyr
   340 345 350
   Val Leu Pro Pro Ser Pro Lys Glu Leu Ser Ser Ser Asp Thr Val Ser
   355 360 365
   Ile Thr Cys Leu Ile Lys Asp Phe Tyr Pro Pro Asp Ile Asp Val Glu
   370 375 380
   Trp Gln Ser Asn Gly Gln Gln Glu Pro Glu Arg Lys His Arg Met Thr
   385 390 395 400
   Pro Pro Gln Leu Asp Glu Asp Gly Ser Tyr Phe Leu Tyr Ser Lys Leu
   405 410 415
   Ser Val Asp Lys Ser Arg Trp Gln Gln Gly Asp Pro Phe Thr Cys Ala
   420 425 430
   Val Met His Glu Thr Leu Gln Asn His Tyr Thr Asp Leu Ser Leu Ser
   435 440 445
   His Ser Pro Gly Lys
   450
<210> 71
   <211> 107
   <212> PRT
   <213> Artificial Sequence
<220>
   <223> updated light chain variable domain of a canine version of human D2E7 anti-TNF MAb - canine VK
<400> 71
   Asp Ile Val Met Thr Gln Ser Pro Ser Ser Leu Ser Ala Ser Gln Gly
   1 5 10 15
   Glu Lys Val Thr Ile Thr Cys Arg Ala Ser Gln Gly Ile Arg Asn Tyr
   20 25 30
   Leu Ala Trp Tyr Gln Gln Lys Pro Gly His Ala Pro Lys Leu Leu Ile
   35 40 45
   Tyr Ala Ala Ser Thr Leu Gln Ser Gly Val Pro Ser Arg Phe Ser Gly
   50 55 60
   Ser Gly Ser Gly Thr Asp Phe Thr Leu Thr Ile Ser Ser Leu Glu Pro
   65 70 75 80
   Glu Asp Val Ala Val Tyr Tyr Cys Gln Arg Tyr Asn Arg Ala Pro Tyr
                   85                  90                  95
   Thr Phe Gly Gln Gly Thr Lys Val Glu Ile Lys
               100                 105